# EUROPEAN PATENT SPECIFICATION

(11) **EP 3 988 544 B1**
(45) Date of publication and mention of the grant of the patent: **21.05.2025**
(21) Application number: 20827811.9
(22) Date of filing: 22.06.2020
(51) Int. Cl.: C07D 401/12, C07D 401/14, A61K 31/551, A61P 7/02, A61P 9/10, A61P 9/08, A61P 27/06, A61P 9/12

(54) **ISOQUINOLINONE DERIVATIVES SERVING AS ROCK PROTEIN KINASE INHIBITORS AND USE THEREOF**
ISOCHINOLINONDERIVATE ALS ROCK-PROTEINKINASEINHIBITOREN UND IHRE VERWENDUNG
DÉRIVÉS D'ISOQUINOLINONE SERVANT D'INHIBITEURS DE PROTÉINE KINASE ROCK ET LEUR UTILISATION

(30) Priority: 21.06.2019 CN 201910544202; 06.11.2019 CN 201911078066
(43) Date of publication of application: 27.04.2022
(73) Proprietor: Guangzhou Ocusun Ophthalmic Biotechnology Co., Ltd., Guangzhou, Guangdong 511400 (CN)
(72) Inventor: LIU, Yizhi, Guangzhou, Guangdong 510060 (CN); WANG, Yandong, Guangzhou, Guangdong 510060 (CN); WU, Lingyun, Shanghai 200131 (CN); YOU, Xu, Shanghai 200131 (CN); XIAO, Zheming, Shanghai 200131 (CN); CHEN, Shuhui, Shanghai 200131 (CN)
(74) Representative: Latscha Schöllhorn Partner AG
(86) International application number: PCT/CN2020/097503
(87) International publication number: WO 2020/253882

(56) References cited:
- EP-A1- 1 818 329
- EP-A1- 1 932 841
- EP-A1- 2 130 828
- WO-A1-2004/106325
- WO-A1-2015/165342
- WO-A1-2022/135421
- CN-A- 101 253 166
- CN-A- 101 622 243
- CN-A- 105 085 478
- CN-A- 105 085 525

## Description

### TECHNICAL FIELD

The present disclosure relates to a series of isoquinolone derivatives as ROCK protein kinase inhibitors and uses thereof in preparation of ROCK protein kinase inhibitor-related medicaments for treating glaucoma or ocular hypertension, and particularly relates to a compound of formula (I) or a pharmaceutically acceptable salt thereof.

### BACKGROUND

RHO associated kinase (ROCK), belonging to a serine/threonine protein kinase, is a downstream target molecule of RHO and is widely expressed in human body. ROCK is involved in regulation of myosin light chain (MLC) and is suitable for treatment of vasodilation; ROCK kinase can also act on trabecular outflow tract cells to relax trabecular cells and reduce aqueous outflow resistance. Recent studies have shown that ROCK kinase inhibitors can also promote damage repair of corneal endothelial cells and prevent fibrosis, showing great application prospects.

Isoquinoline sulfonamide compounds are an important type of ROCK kinase inhibitors. The currently launched Fasudil and K-115 (WO2006057397A1) are both isoquinoline sulfonamide compounds. Wherein, Fasudil, as a new drug with extensive pharmacological effects, is a RHO kinase inhibitor, which dilates blood vessels by increasing the activity of myosin light chain phosphatase, reduces the tension of endothelial cells, improves brain tissue microcirculation, but does not induce or aggravate cerebral steal, and at the same time can antagonize the inflammatory factor, protect the nerve against apoptosis and promote the nerve regeneration. K-115 has a wide range of approved and potential applications, including treatment of glaucoma, ocular hypertension, diabetic retinal injury complications, age-related macular degeneration, corneal injury, cataract and glaucoma recovery after surgery and so on, and at the same time may be further extended to systemic drugs.

WO2007026664A1 reported a series of compounds with ROCK kinase inhibition effects, for example, control compound 1 and control compound 2. The series of compounds have good enzyme activity, but need to be improved on membrane permeability, pharmacokinetics, druggability and other aspects. The present disclosure reports a kind of structurally modified similar compounds with the properties significantly improved.

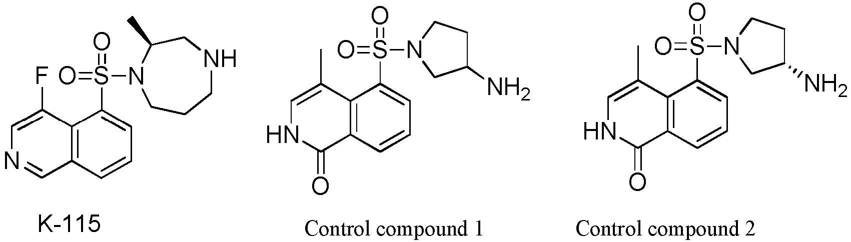

### SUMMARY

The present disclosure provides a compound of formula (I) or a pharmaceutically acceptable salt thereof, 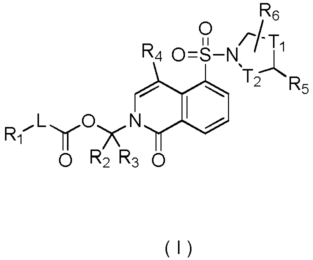 wherein,
the structure unit 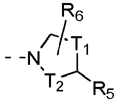 is selected from a group consisting of 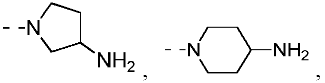 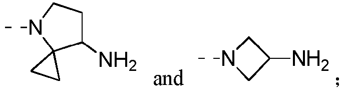
R₁ is selected from a group consisting of C₁₋₁₆ alkyl, phenyl, C₃₋₇ cycloalkyl, 3-8 membered heterocycloalkyl and 5-10 membered heteroaryl, wherein, each of C₁₋₁₆ alkyl, phenyl, C₃₋₇ cycloalkyl, 3-8 membered heterocycloalkyl and 5-10 membered heteroaryl is optionally substituted by 1, 2 or 3 Rₐ;
each of R₂ and R₃ is independently selected from a group consisting of H, F, Cl, Br, I, OH, NH₂, CN and C₁₋₃ alkyl;
R₄ is selected from a group consisting of H, F, Cl, Br, I, OH, NH₂, CN, and C₁₋₃ alkyl which is optionally substituted by 1, 2 or 3 R_{b};
L is selected from a group consisting of a single bond, -O- and -NR₁₁-;
R₁ is selected from a group consisting of H and C₁₋₃ alkyl;
Rₐ is selected from a group consisting of H, F, Cl, Br, I, OH, NH₂, CN, C₁₋₃ alkyl and C₁₋₃ alkoxy, wherein each of C₁₋₃ alkyl and C₁₋₃ alkoxy is optionally substituted by 1, 2 or 3 R;
each of R_{b} and Rₑ is independently selected from a group consisting of H, F, Cl, Br, I, OH, NH₂, CN and C₁₋₃ alkyl;
R is selected from a group consisting of F, Cl, Br, I, OH, NH₂, CN and CH₃;
wherein, each of the 3-8 membered heterocycloalkyl and 5-10 membered heteroaryl is independently include 1, 2, 3 or 4 heteroatom(s) or heteroatom group(s) which is/are independently selected from a group consisting of -NH-, -O-, -S- and N.

In some embodiments of the present disclosure, Rₐ is selected from a group consisting of F, Cl, Br, I, OH, NH₂, CN, CH₃, CF₃, CH₂F, CHF₂, CH₂CH₃ and OCH₃, other variables are as defined in the present disclosure.

In some embodiments of the present disclosure, Rₐ is selected from a group consisting of H, F, Cl, Br, I, OH, NH₂, CN, CH₃, CF₃, CH₂F, CHF₂, CH₂CH₃ and OCH₃, other variables are as defined in the present disclosure.

In some embodiments of the present disclosure, R₁ is selected from a group consisting of C₁₋₁₂ alkyl, phenyl, cyclobutyl, cyclopentyl, cyclohexyl, tetrahydrofuranyl, tetrahydropyranyl, piperidinyl, thienyl, furyl, pyrrolyl and benzofuryl, wherein, each of C₁₋₁₂ alkyl, phenyl, cyclobutyl, cyclopentyl, cyclohexyl, tetrahydrofuranyl, tetrahydropyranyl, piperidinyl, thienyl, furyl, pyrrolyl and benzofuryl is optionaly substituted by 1, 2 or 3 Rₐ, other variables are as defined in the present disclosure.

In some embodiments of the present disclosure, R₁ is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, 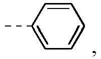 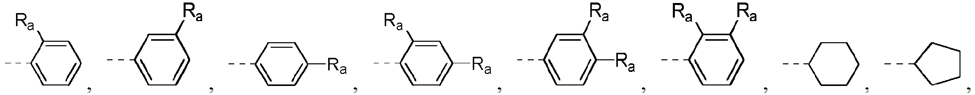 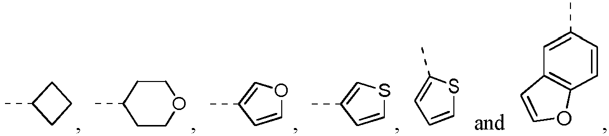 other variables are as defined in the present disclosure.

In some embodiments of the present disclosure, R₁ is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, 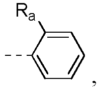 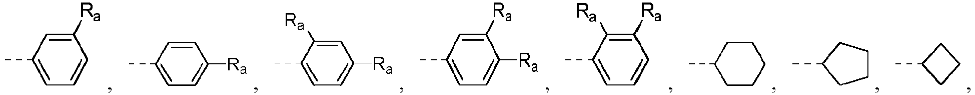 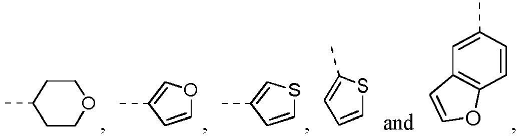 other variables are as defined in the present disclosure.

In some embodiments of the present disclosure, R₁ is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, 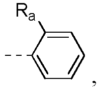 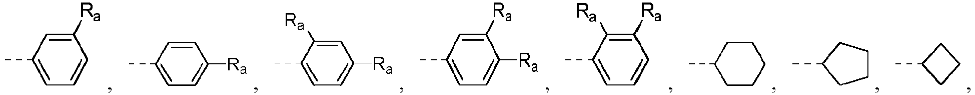 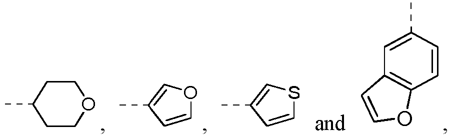 other variables are as defined in the present disclosure.

In some embodiments of the present disclosure, R₁ is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, 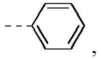 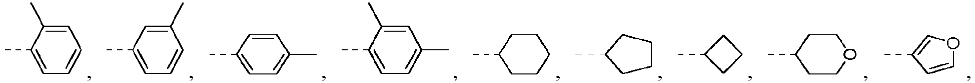 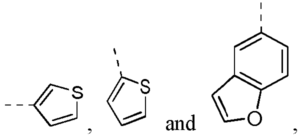 other variables are as defined in the present disclosure.

In some embodiments of the present disclosure, R₁ is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, 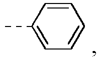 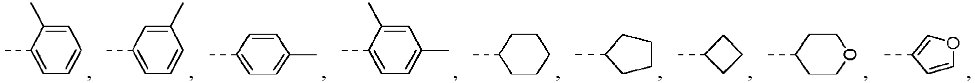 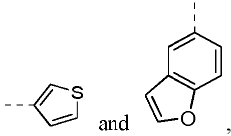 other variables are as defined in the present disclosure.

In some embodiments of the present disclosure, each of R₂ and R₃ is independently selected from a group consisting of H, F, Cl, Br, I, OH and NH₂, other variables are as defined in the present disclosure.

In some embodiments of the present disclosure, each of R₂ and R₃ is independently selected from a group consisting of H, F, Cl, Br, I, OH, NH₂, other variables are as defined in the present disclosure.

In some embodiments of the present disclosure, R₄ is selected from a group consisting of H, F, Cl, Br, I, OH, NH₂, CN, CH₃ and CH₂CH₃, other variables are as defined in the present disclosure.

In some embodiments of the present disclosure, L is selected from a group consisting of a single bond, -O-, -NH- and -N(CH₃)-, other variables are as defined in the present disclosure.

The structure unit 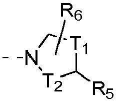 is selected from a group consisting of 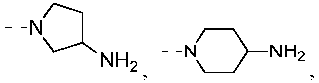 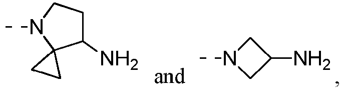 other variables are as defined in the present disclosure.

In some embodiments of the present disclosure, the structure unit 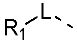 is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, OCH₃, OCH₂CH₃, O(CH₂)₂CH₃, O(CH₂)₃CH₃, O(CH₂)₄CH₃, O(CH₂)₅CH₃, O(CH₂)₆CH₃, OCH(CH₃)₂, OC(CH₃)₃, N(CH₃)₂, 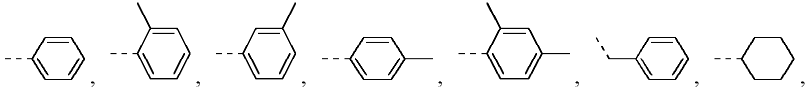 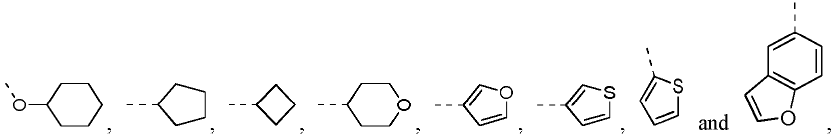 other variables are as defined in the present disclosure.

In some embodiments of the present disclosure, the structure unit 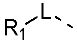 is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, OCH₃, OCH₂CH₃, O(CH₂)₂CH₃, O(CH₂)₃CH₃, O(CH₂)₄CH₃, O(CH₂)₅CH₃, O(CH₂)₆CH₃, OCH(CH₃)₂, OC(CH₃)₃, N(CH₃)₂, 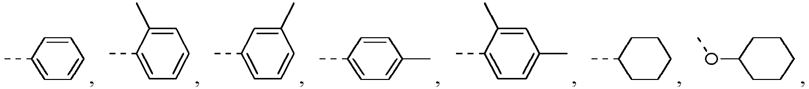 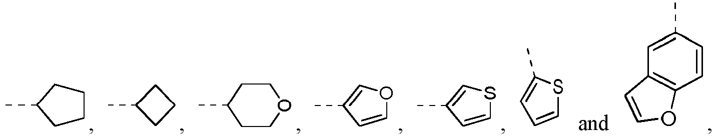 other variables are as defined in the present disclosure.

In some embodiments of the present disclosure, the structure unit 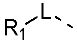 is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, OCH₃, OCH₂CH₃, O(CH₂)₂CH₃, O(CH₂)₃CH₃, O(CH₂)₄CH₃, O(CH₂)₅CH₃, O(CH₂)₆CH₃, OCH(CH₃)₂, OC(CH₃)₃, N(CH₃)₂, 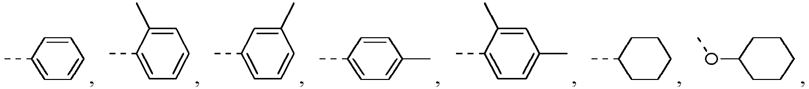 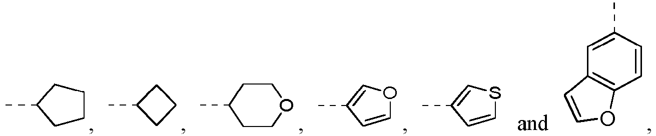 other variables are as defined in the present disclosure.

Some other embodiments of the present disclosure are derived from any combination of the above variables.

In some embodiments of the present disclosure, the compound or the pharmaceutically acceptable salt thereof is selected from a group consisting of: 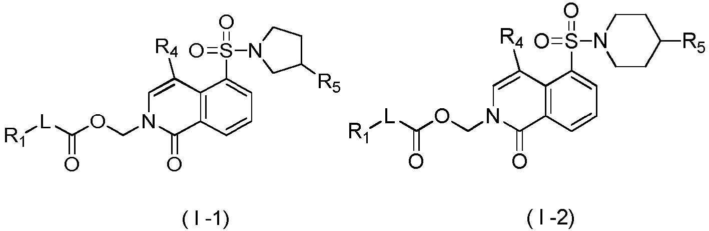 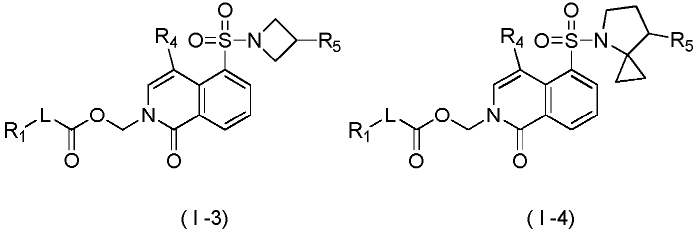 wherein,
R₁, R₄, ₅ and L are as defined in the present disclosure.

In some embodiments of the present disclosure, the compound or the pharmaceutically acceptable salt thereof is selected from a group consisting of: 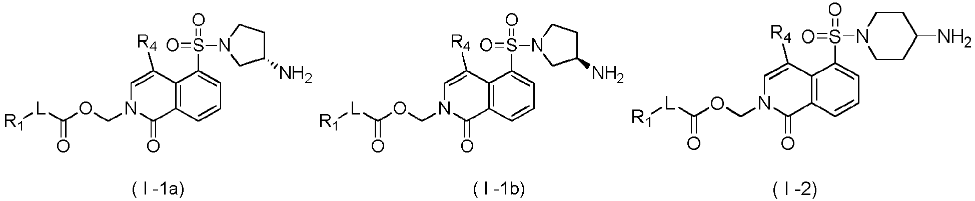 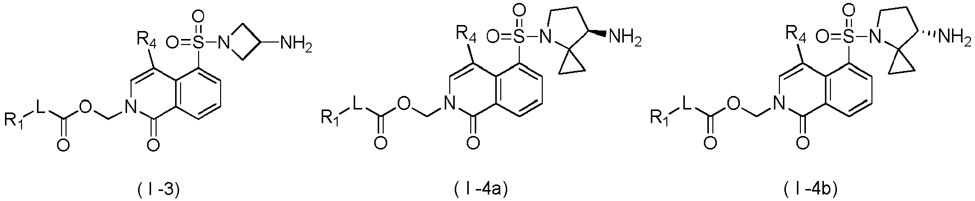 wherein,
R₁, R₄ and L are as defined in the present disclosure.

The present disclosure also provides a compound of the following formula or a pharmaceutically acceptable salt thereof, wherein the compound is selected from a group consisting of:

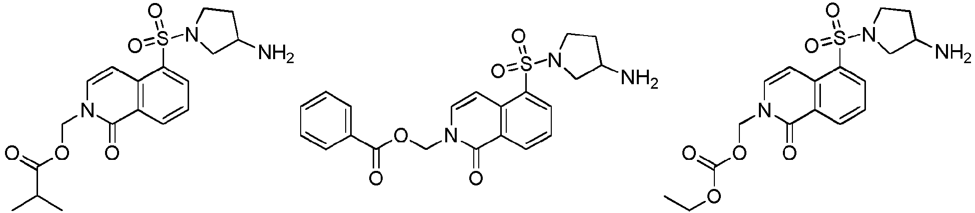

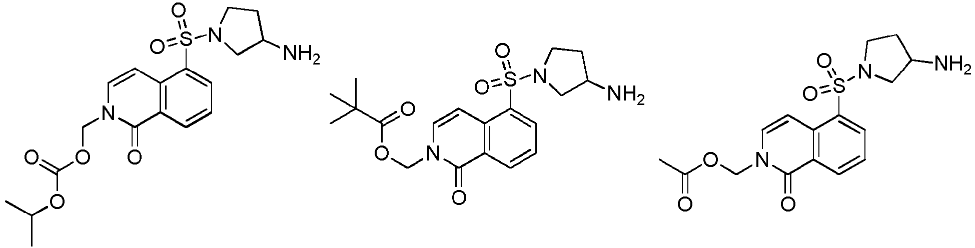

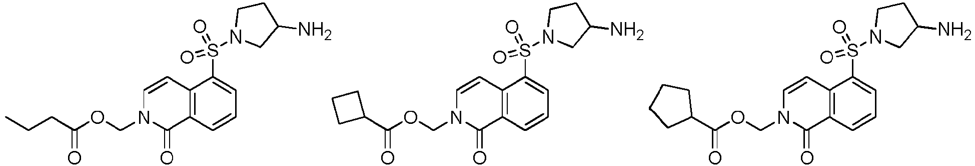

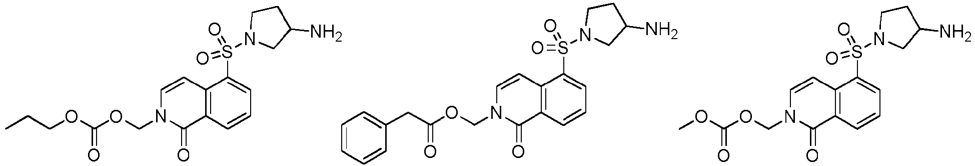

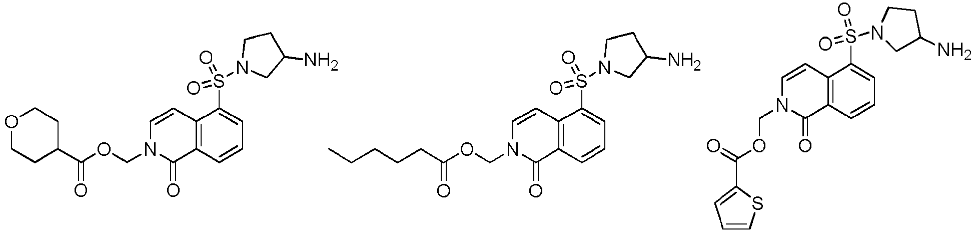

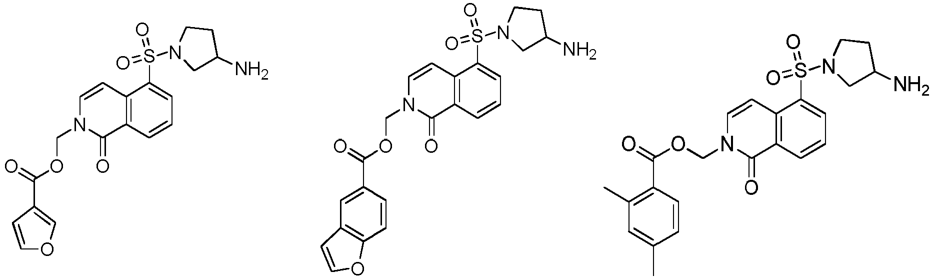

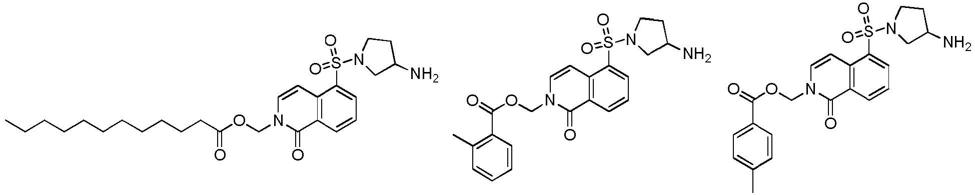

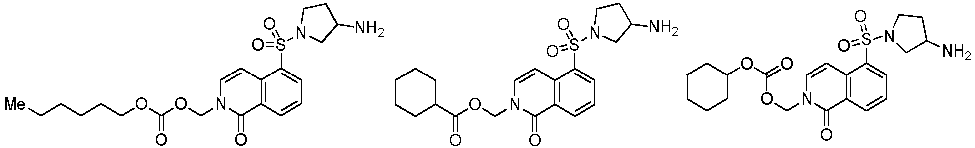

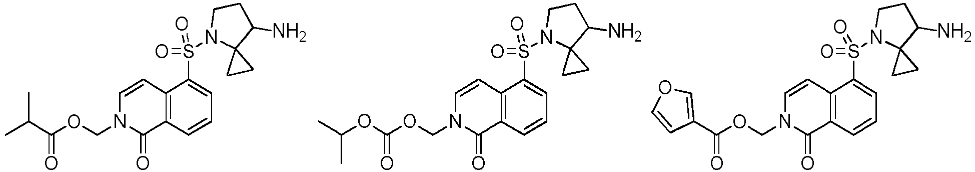

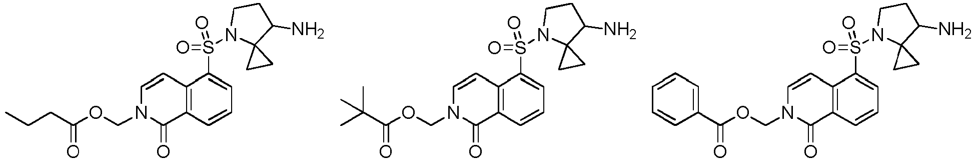

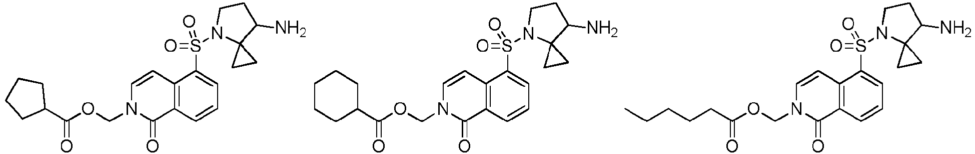

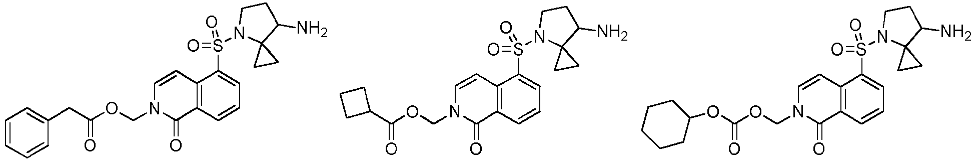

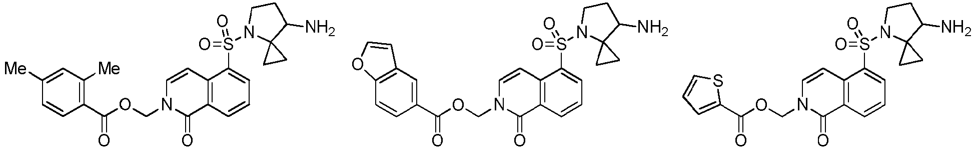

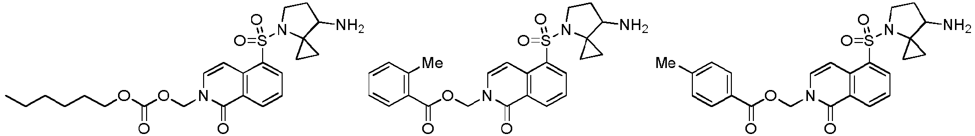

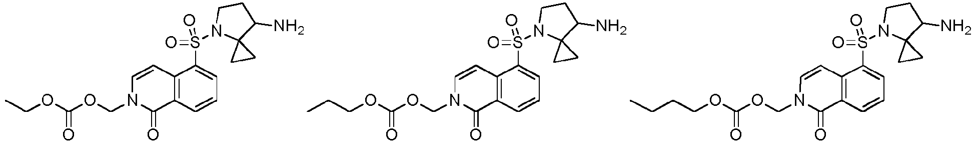

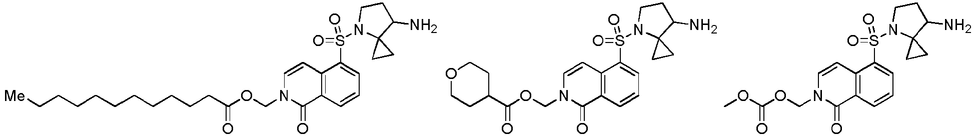

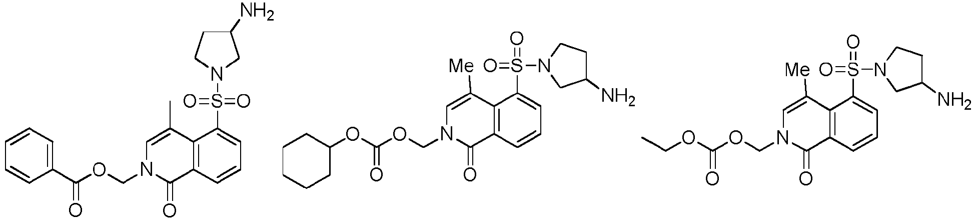

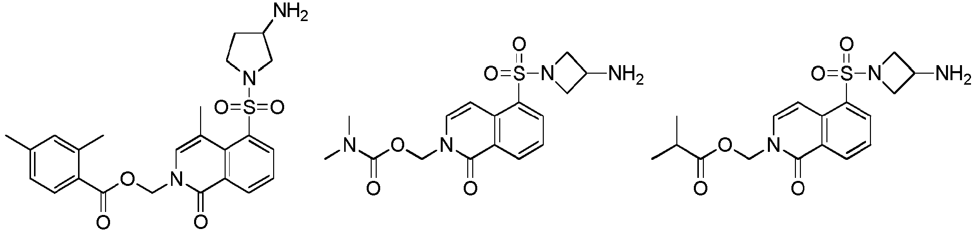

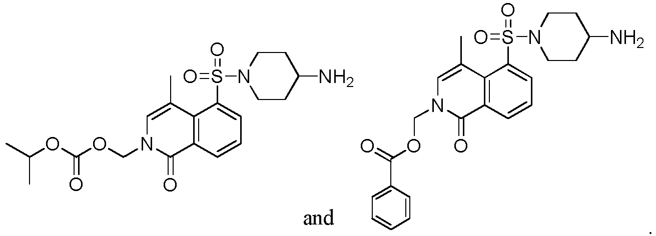

In some embodiments of the present disclosure, the compound or the pharmaceutically acceptable salt thereof is selected from a group consisting of:

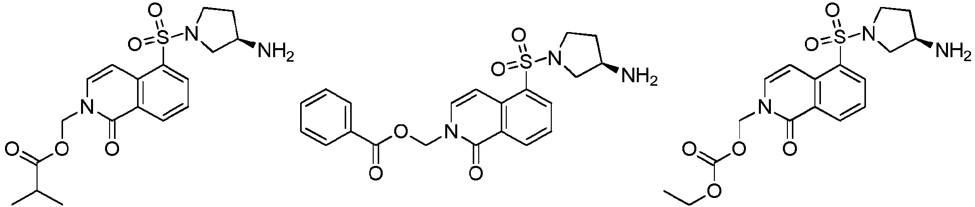

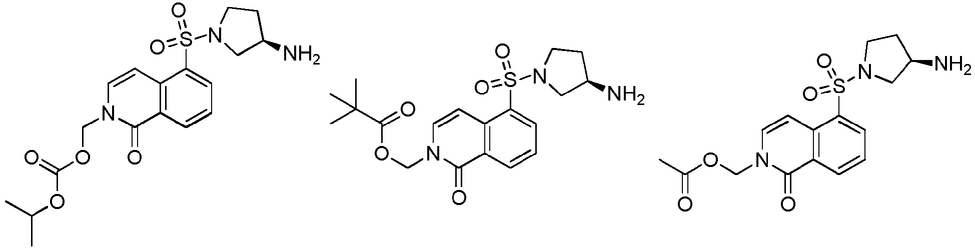

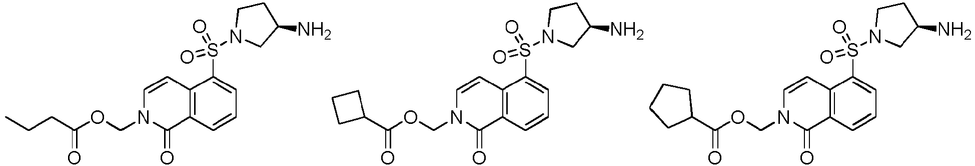

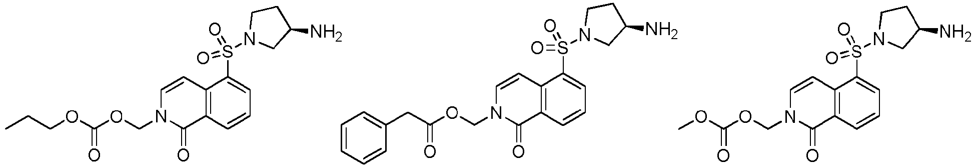

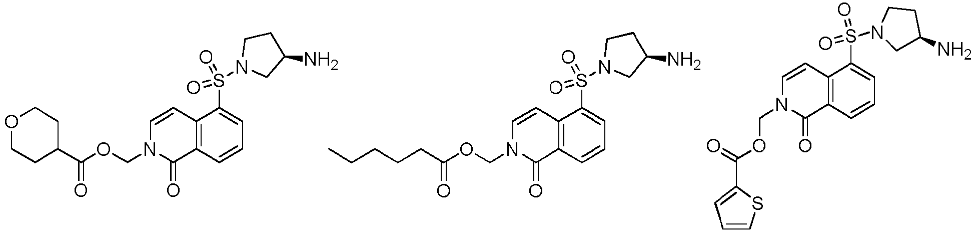

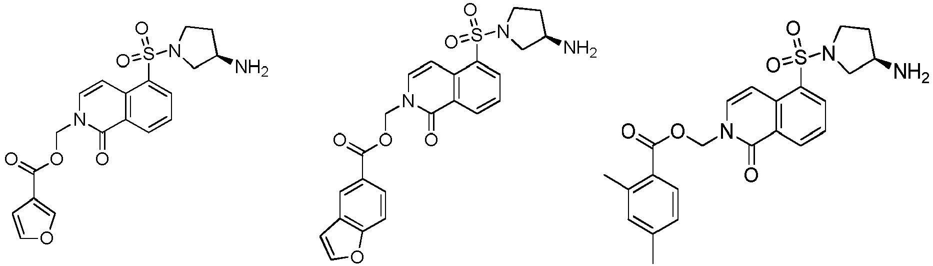

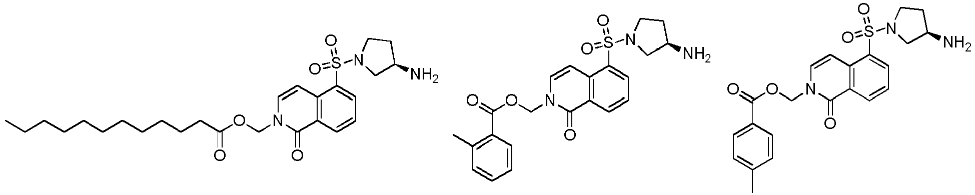

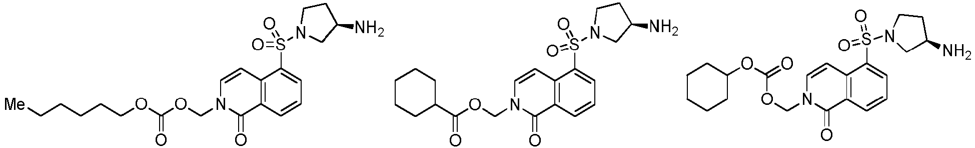

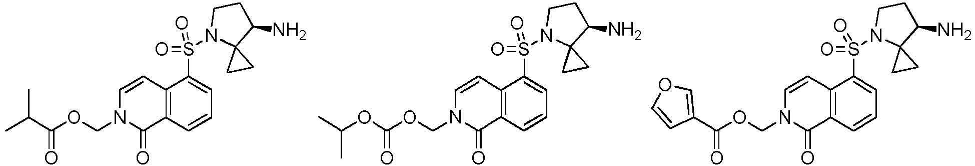

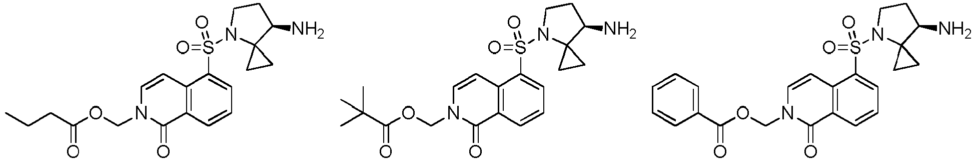

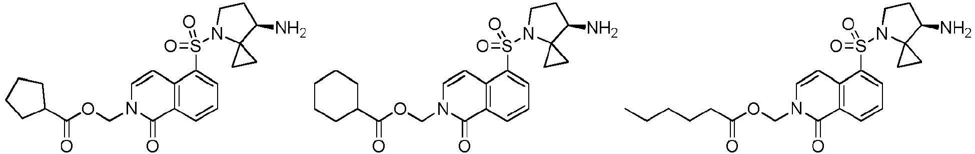

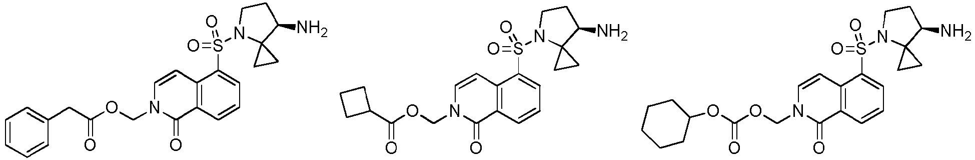

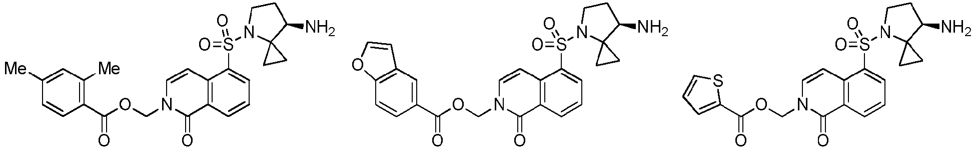

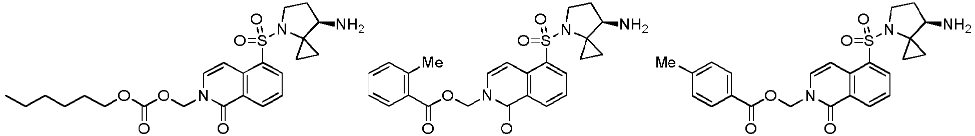

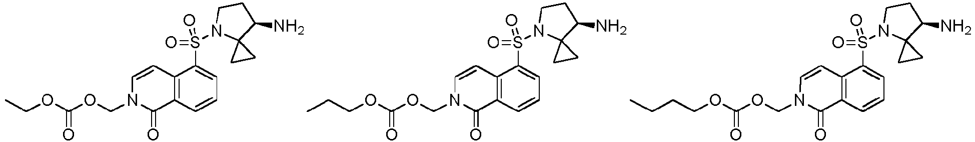

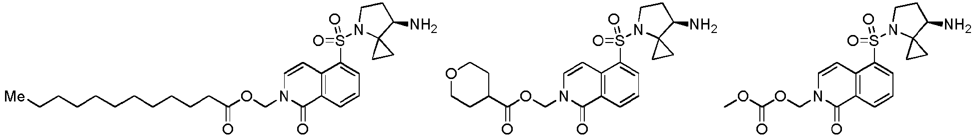

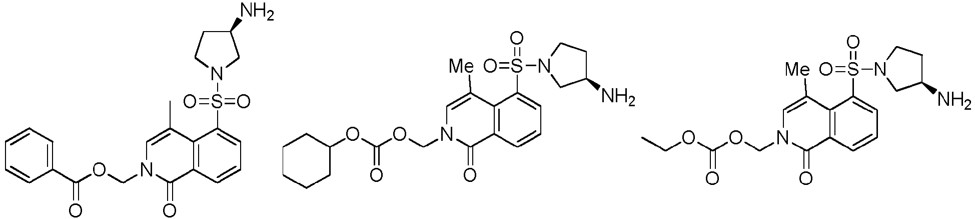

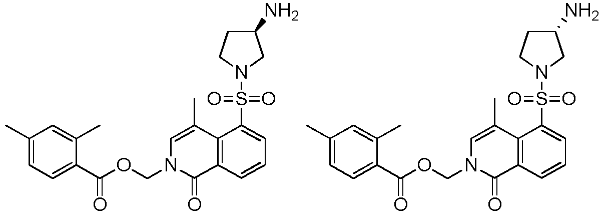

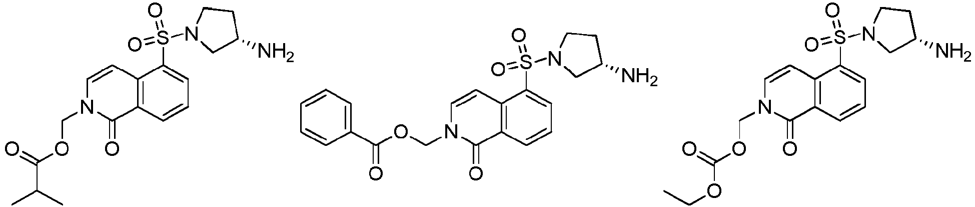

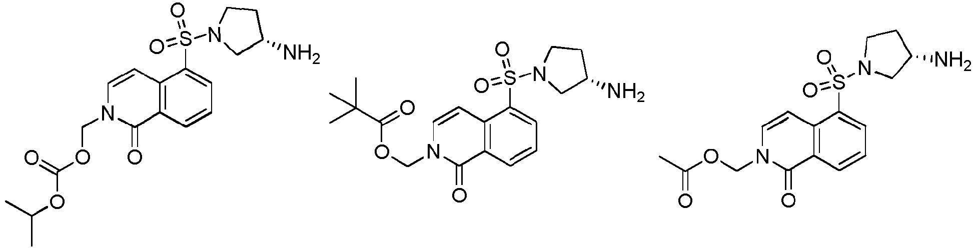

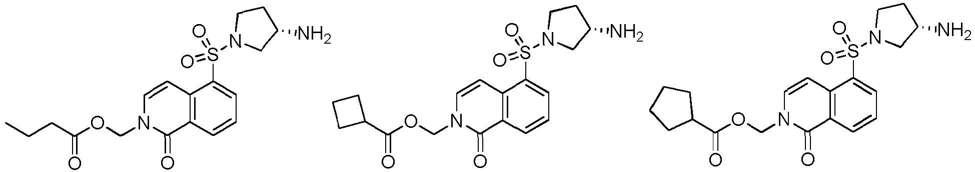

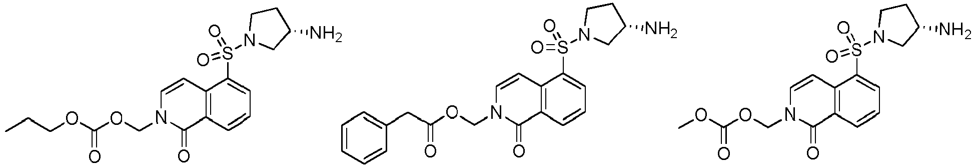

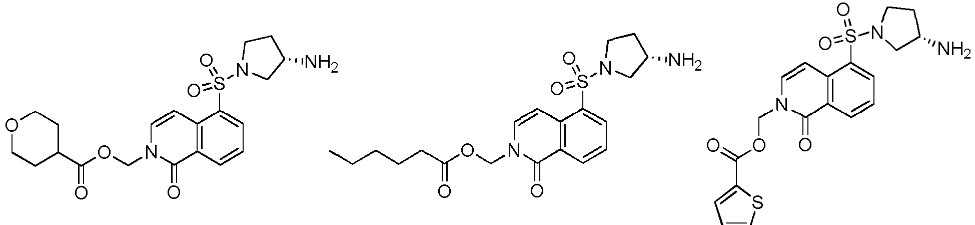

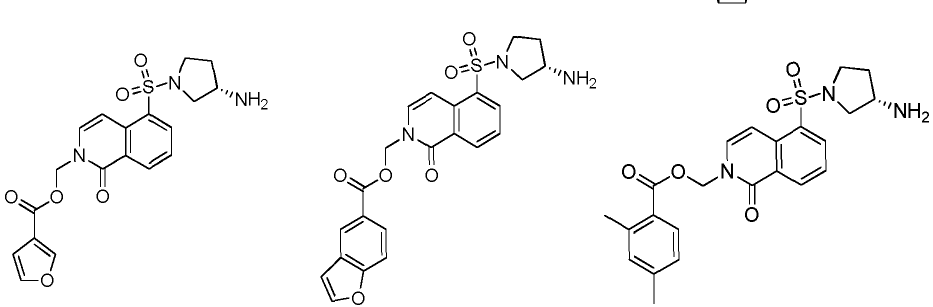

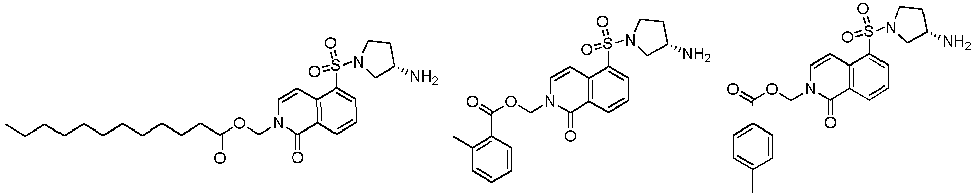

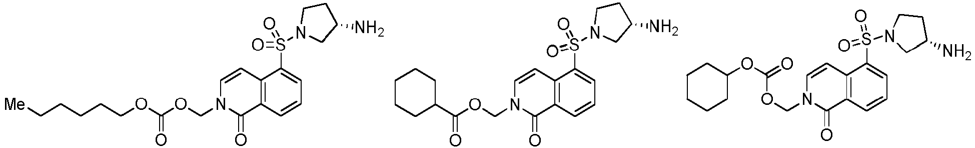

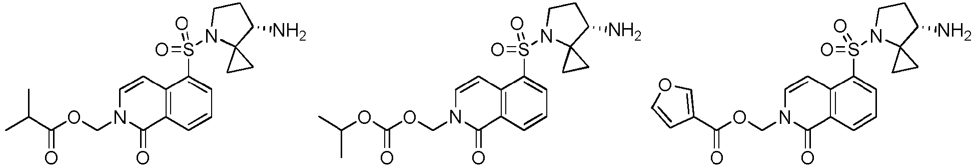

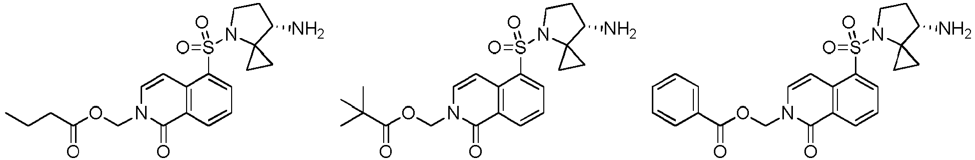

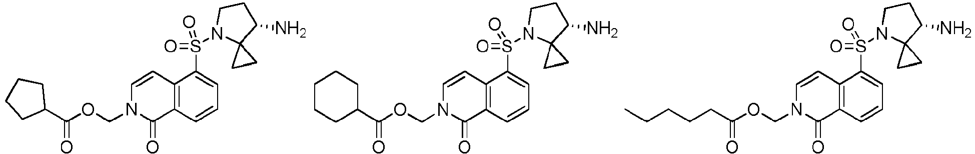

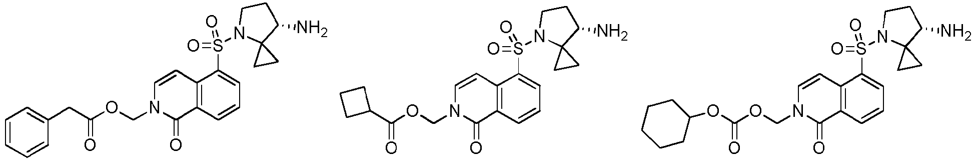

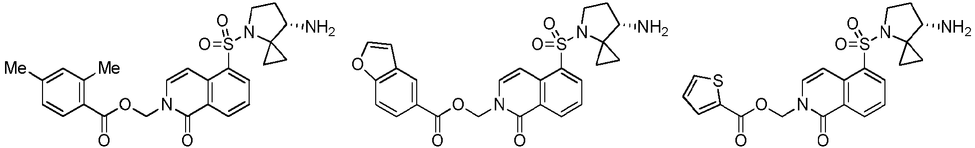

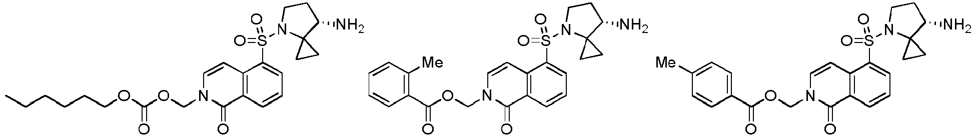

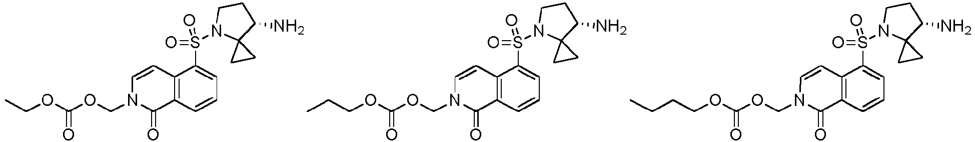

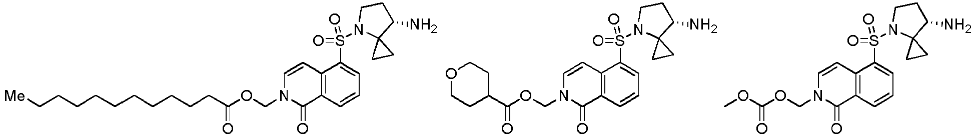

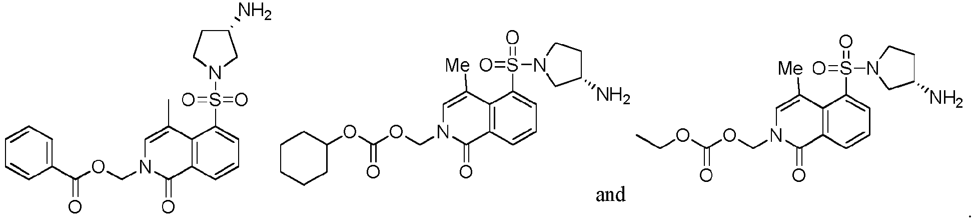

The present disclosure also provides a pharmaceutical composition, comprising a therapeutically effective amount of the compound or the pharmaceutically acceptable salt thereof and a pharmaceutically acceptable carrier.

In some embodiments of the present disclosure, providing a method of treating a disease associated with an ROCK protein kinase, comprising administrating a therapeutically effective amount of the compound or the pharmaceutically acceptable salt thereof, wherein the disease associated with an ROCK protein kinase is selected from glaucoma or ocular hypertension.

### Definitions and Explanations

Unless otherwise stated, the following terms and phrases used herein shall have the following meanings. A specific term or phrase shall not be considered as uncertain or unclear without a special definition, but shall be understood in its plain meaning. When a trade name appears herein, it is meant to refer to its corresponding commodity or its active ingredient. The term "pharmaceutically acceptable" employed herein refers to those compounds, materials, compositions, and/or dosage forms which are within the scope of sound medical judgement, suitable for use in contact with tissues of human beings and animals without excessive toxicity, irritation, allergic response, or other problem or complications, and commensurate with a reasonable benefit/risk ratio.

The term "pharmaceutically acceptable salt" refers to salts of the compounds of the present disclosure, which are prepared from the compounds with specific substituents discovered in the present disclosure and a relatively non-toxic acid or base. When the compounds of the present disclosure contain a relatively acidic functional group, a base addition salt can be obtained by contacting a neutral form of the compounds with a sufficient amount of base in a pure solution or a suitable inert solvent. The pharmaceutically acceptable base addition salts include sodium, potassium, calcium, ammonium, organic amine or magnesium salts or similar salts. When the compounds of the present disclosure contain a relatively basic functional group, an acid addition salt can be obtained by contacting a neutral form of the compounds with a sufficient amount of acid in a pure solution or a suitable inert solvent. Examples of the pharmaceutically acceptable acid addition salts include salts of inorganic acid, including, for example, hydrochloric acid, hydrobromic acid, nitric acid, carbonic acid, bicarbonate, phosphoric acid, monohydrogen phosphate, dihydrogen phosphate, sulfuric acid, hydrogen sulphate, hydroiodic acid, phosphorous acid, etc.; salts of organic acid, including, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid and other similar acids; and salts of amino acid (such as arginine) and salts of organic acids such as glucuronic acid. Certain specific compounds of the present disclosure contain basic and acidic functional groups, and therefore can be converted into any base or acid addition salts.

The pharmaceutically acceptable salts of the present disclosure can be synthesized from parent compounds which contain acidic radicals or basic groups by conventional chemical methods. Generally, such salts can be prepared by a reaction of the compounds in the form of free acid or base and a stoichiometric amount of appropriate base or acid in water or in an organic solvent, or in a mixture of the two.

In addition to the form of salt, the compounds provided by the present disclosure also exist in the form of prodrugs. The prodrugs of the compounds described herein are prone to chemical changes under physiological conditions to be converted into the compounds of the present disclosure. In addition, the prodrugs may be converted into the compounds of the present disclosure by a chemical or biochemical method in an in-vivo environment.

Some compounds of the present disclosure may exist in a non-solvated form or a solvated form, including a hydrate form. In general, the solvated form is equivalent to the non-solvated form, and both are included in the scope of the present disclosure.

The compounds of the present disclosure may exist in specific geometric or stereoisomeric forms. The present disclosure contemplates all the compounds, including cis- and trans-isomers, (-)- and (+)-enantiomers, (*R*)- and (S)-enantiomers, diastereoisomers, (D)-isomers, (L)-isomers, and racemic mixtures and other mixtures thereof, such as enriched mixtures of the enantiomers or diastereoisomers, and all of the mixtures are within the scope of the present disclosure. Additional asymmetric carbon atoms may exist in substituents such as alkyl. All these isomers and mixtures thereof are included in the scope of the present disclosure.

Unless otherwise stated, the term "enantiomer" or "optical isomer" refers to stereoisomers which are in a mutual mirrored relationship.

Unless otherwise stated, the term "cis-trans isomer" or "geometric isomer" is caused by inability of double bonds or single bonds of ring-constituting carbon atoms to rotate freely.

Unless otherwise stated, the term "diastereoisomers" refers to stereoisomers in which molecules have two or more chiral centers and the molecules are in a non-mirrored relationship.

Unless otherwise stated, "(*D*)" or "(+)" means right-handed, "(*L*)" or "(-)" means left-handed, and "(*DL*)" or "(+/-)" means racemic.

Unless otherwise stated, a wedge-shaped solid line bond ( 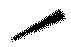 ) and a wedge-shaped dashed line bond ( 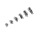 ) represent an absolute configuration of a stereocenter, a straight solid line bond ( 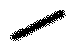 ) and a straight dashed line bond ( 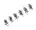 ) represent a relative configuration of a stereocenter, a wavy line ( 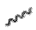 ) represents a wedge-shaped solid line bond ( 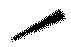 ) or a wedge-shaped dashed line bond ( 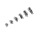 ), or a wavy line ( 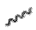 ) represents a straight solid line bond ( 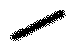 ) or a straight dashed line bond ( 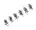 ).

The compounds of the present disclosure can exist in the specific form of tautomers. Unless otherwise stated, the term "tautomer" or "tautomeric form" refers to that at the room temperature, isomers with different functional groups are in a dynamic equilibrium and can be transformed one another quickly. If tautomers are possible (for example in a solution), a chemical equilibrium of the tautomers can be reached. For example, a proton tautomer, also referred to as a prototropic tautomer, includes an intertransformation through proton migration, for example, keto-enol isomerization and imine-enamine isomerization. A valence tautomer includes recombination of some bonding electrons for an intertransformation. A specific example of a keto-enoltautomerization is the tautomerization between two tautomers of pentane-2,4-dione and 4-hydroxypent-3-en-2-one.

Unless otherwise stated, the terms "enriched in one isomer", "enriched in isomers", "enriched in one enantiomer" or "enriched in enantiomers" refers to that the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomer excess" or "enantiomeric excess" refers to a difference between relative percentages of two isomers or two enantiomers. For example, if the content of one isomer or enantiomer is 90%, and the content of the other isomer or enantiomer is 10%, the isomer or enantiomeric excess (ee value) is 80%.

The optically active (*R*)- and (*S*)-isomers and *D* and *L* isomers can be prepared by chiral synthesis or chiral reagents or other conventional techniques. If desired, an enantiomer of a compound of the present disclosure can be prepared by asymmetric synthesis or derivatization with a chiral auxiliary, the resulting diastereomeric mixture is separated and auxiliary groups are cleaved to provide the desired pure enantiomer. Alternatively, when a molecule contains a basic functional group (such as an amino group) or an acidic functional group (such as a carboxyl group), a diastereomeric salt is formed with a suitable optically active acid or base, then diastereoisomers are resolved through a conventional method known in the art, and the pure enantiomer is recovered. In addition, separation of the enantiomer and adiastereomer is usually accomplished through chromatography which employs a chiral stationary phase and is optionally combined with chemical derivatization (for example, formation of carbamate from amines). The compounds of the present disclosure may contain atomic isotopes in an unnatural proportion on one or more of atoms constituting the compounds. For example, the compounds can be labeled with radioisotopes, such as tritium (³H), iodine-125 (¹²⁵I), or C-14 (¹⁴C). For another example, deuterated drugs can be formed by replacing hydrogen with deuterium. A bond formed by deuterium and carbon is stronger than that formed by ordinary hydrogen and carbon. Compared with undeuterated drugs, the deuterated drugs have advantages of reducing toxic and side effects, increasing drug stability, enhancing efficacy, prolonging biological half-life of drugs and the like. All changes in isotopic composition of the compounds of the present disclosure, whether radioactive or not, are included in the scope of the present disclosure.

The term "optional" or "optionally" means that the event or condition described later may but does not necessarily occur and the description includes a situation in which the event or condition occurs and a situation in which the event or condition does not occur.

The term "substituted" means that any one or more hydrogen atoms on a specific atom are replaced by substituents, including deuterium and hydrogen variants, as long as the valence of the specific atom is normal and the substituted compound is stable. When the substituent is oxygen (i.e. =O), it means that two hydrogen atoms are substituted. Oxygen substitution does not occur on aromatic groups. The term "optionally substituted" means that it can be substituted or unsubstituted. Unless otherwise stated, the type and number of substituents can be arbitrary on the basis that they can be chemically realized.

When any variable (such as R) occurs more than once in the composition or structure of a compound, its definition in each case is independent. Thus, for example, if a group is substituted by 0-2 R, the group can optionally be substituted by up to two R, and the R has independent options in each case. In addition, combinations of substituents and/or variants thereof are only allowed if such combinations result in stable compounds.

When the number of a linking group is 0, such as -(CRR)₀-, it means that the linking group is a single bond.

When one of the variables is selected from a single bond, it means that the two groups connected by the single bond are directly connected. For example, when L in A-L-Z represents a single bond, it means that the structure is actually A-Z.

When a substituent is vacant, it means that the substituent is absent. For example, when X in A-X is vacant, it means that the structure is actually A. When the listed substituents do not indicate through which atom it is connected to the substituted group, such substituents can be bonded by any atoms. For example, pyridyl can be connected to the substituted group through any one carbon atom of a pyridine ring as a substituent. When the listed linking group does not indicate its linking direction, the linking direction is arbitrary. For example, the linking group L in 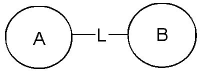 is -M-W-, on this occasion, the -M-W- can link ring A and ring B in a left-to-right reading direction to form 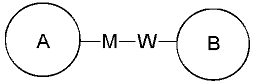 and can also link ring A and ring B in a direction opposite to the left-to-right reading direction to form 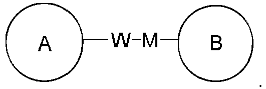 Combinations of the linking group, substituents and/or variants thereof are only allowed if such combinations result in stable compounds.

Unless otherwise stated, the term "C₁₋₁₆ alkyl" is used to indicate a linear or branched saturated hydrocarbon group composed of 1 to 16 carbon atoms. The C₁₋₁₆ alkyl includes C₁₋₁₅, C₁₋₁₄, C₁₋₁₂, C₁₋₁₀, C₁₋₉, C₁₋₈, C₁₋₆, C₁₋₅, C₁₋₄, C₁₋₃, C₁₋₂, C₂₋₆, C₂₋₄, C₁₀, C₈, C₇, C₆, C₅ alkyl, and so on, and can be monovalent (such as methyl), divalent (such as methylene) or multivalent (such as methine). Examples of the C₁₋₁₆ alkyl include, but are not limited to, methyl (Me), ethyl (Et), propyl (including n-propyl and isopropyl), butyl (including n-butyl, isobutyl, s-butyl and t-butyl), pentyl (including n-pentyl, isopentyl and neopentyl), hexyl, heptyl, octyl, and so on.

Unless otherwise stated, the term "C₁₋₁₂ alkyl" is used to indicate a linear or branched saturated hydrocarbon group composed of 1 to 12 carbon atoms. The C₁₋₁₂ alkyl includes C₁₋₁₀, C₁₋₉, C₁₋₈, C₁₋₆, C₁₋₅, C₁₋₄, C₁₋₃, C₁₋₂, C₂₋₆, C₂₋₄, C₁₀, C₈, C₇, C₆, C₅ alkyl, and so on, and can be monovalent (such as methyl), divalent (such as methylene) or multivalent (such as methine). Examples of the C₁₋₁₂ alkyl include, but are not limited to, methyl (Me), ethyl (Et), propyl (including n-propyl and isopropyl), butyl (including n-butyl, isobutyl, s-butyl and t-butyl), pentyl (including n-pentyl, isopentyl and neopentyl), hexyl, heptyl, octyl, and so on. Unless otherwise stated, the term "C₁₋₃ alkyl" is used to indicate a linear or branched saturated hydrocarbon group composed of 1 to 3 carbon atoms. The C₁₋₃ alkyl includes C₁₋₂, C₂₋₃ alkyl, and so on, and can be monovalent (such as methyl), divalent (such as methylene) or multivalent (such as methine). Examples of the C₁₋₃ alkyl include, but are not limited to, methyl (Me), ethyl (Et), propyl (including n-propyl and isopropyl), and so on.

Unless otherwise stated, the term "C₁₋₃ alkoxy" represents alkyl groups containing 1 to 3 carbon atoms linked to the rest of the molecule through an oxygen atom. The C₁₋₃ alkoxy includes C₁₋₂, C₂₋₃, C₃, C₂ alkoxy, and so on. Examples of the C₁₋₃ alkoxy include, but are not limited to, methoxy, ethoxy, propoxy (including n-propoxy and isopropoxy), and so on.

Unless otherwise stated, "C₃₋₇ cycloalkyl" represents a saturated cyclic hydrocarbon group composed of 3 to 7 carbon atoms and the saturated cyclic hydrocarbon group is a monocyclic ring system. The C₃₋₇ cycloalkyl includes C₅₋₇, C₃₋₄ and C₄₋₅ cycloalkyl, and so on, and can be monovalent, divalent or multivalent. Examples of the C₃₋₇ cycloalkyl include, but are not limited to, cyclopropyl, cyclobutyl, cyclopentyl, and so on.

Unless otherwise stated, "C₃₋₅ cycloalkyl" represents a saturated cyclic hydrocarbon group composed of 3 to 5 carbon atoms and the saturated cyclic hydrocarbon group is a monocyclic ring system. The C₃₋₅ cycloalkyl includes C₃₋₄ and C₄₋₅ cycloalkyl, and so on, and can be monovalent, divalent or multivalent. Examples of the C₃₋₅ cycloalkyl include, but are not limited to, cyclopropyl, cyclobutyl, cyclopentyl, and so on.

Unless otherwise stated, the term "3-8 membered heterocycloalkyl" by itself or in combination with other terms respectively represents a saturated cyclic group composed of 3 to 8 ring atoms, wherein 1, 2, 3 or 4 ring atoms are heteroatoms independently selected from a group consisting of O, S and N, and the rest are carbon atoms, wherein the nitrogen atom is optionally quaternized, and the nitrogen and sulfur heteroatoms can be optionally oxidized (i.e., NO and S(O)ₚ, p is 1 or 2). The saturated cyclic group includes monocyclic and bicyclic systems, wherein the bicyclic system includes spiro, fused, and bridged rings. In addition, for the "3-8 membered heterocycloalkyl", a heteroatom may occupy a linking position of the heterocycloalkyl with the rest of the molecule. The 3-8 membered heterocycloalkyl includes 3-6 membered, 3-5 membered, 4-6 membered, 5-6 membered, 4 membered, 5 membered, 6 membered heterocycloalkyl, and so on. Examples of the 3-8 membered heterocycloalkyl include, but are not limited to, azacyclobutyl, oxacyclobutyl, thiacyclobutyl, pyrrolidinyl, pyrazolidinyl, imidazolidinyl, tetrahydrothienyl (including tetrahydrothiophen-2-yl and tetrahydrothiophen-3-yl, and so on), tetrahydrofuranyl (including tetrahydrofuran-2-yl, and so on), tetrahydropyranyl, piperidinyl (including 1-piperidinyl, 2-piperidinyl, 3-piperidinyl, and so on), piperazinyl (including 1-piperazinyl, 2-piperazinyl, and so on), morpholinyl (including 3-morpholinyl, 4-morpholinyl, and so on), dioxanyl, dithiazinyl, isoxazolidinyl, isothiazolidinyl, 1,2-oxazinyl, 1,2-thiazinyl, hexahydropyridazinyl, homopiperazinyl, homopiperidinyl or dioxepan, and so on.

Unless otherwise stated, the terms "5-10 membered heteroaryl ring" and "5-10 membered heteroaryl" can be used interchangeably in the present disclosure. The term "5-10 membered heteroaryl" represents a ring group composed of 5 to 10 ring atoms and having a conjugated π-electron system, wherein 1, 2, 3 or 4 ring atoms are heteroatoms independently selected from a group consisting of O, S and N, and the rest are carbon atoms. The ring group can be monocyclic, fused bicyclic or fused tricyclic systems, wherein each ring is aromatic, wherein the nitrogen atom is optionally quaternized, the nitrogen and sulfur heteroatoms can be optionally oxidized (i.e. NO and S(O)ₚ, p is 1 or 2). The 5-10 membered heteroarylgroup can be linked to the rest of the molecule through a heteroatom or a carbon atom. The 5-10 membered heteroaryl group includes 5-8 membered, 5-7 membered, 5-6 membered, 5 membered, 6 membered heteroaryl, and so on. Examples of the 5-10 membered heteroaryl include, but are not limited to, pyrrolyl (including N-pyrrolyl, 2-pyrrolyl, 3-pyrrolyl, and so on), pyrazolyl (including 2-pyrazolyl, 3-pyrrolyl, and so on), imidazolyl (including N-imidazolyl, 2-imidazolyl, 4-imidazolyl, 5-imidazolyl, and so on), oxazolyl (including 2-oxazolyl, 4-oxazolyl, 5-oxazolyl, and so on), triazolyl (1H-1,2,3-triazolyl, 2H-1,2,3-triazolyl, 1H-1,2,4-triazolyl, 4H-1,2,4-triazolyl, and so on), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, and so on), furyl (including 2-furyl, 3-furyl, and so on), thienyl (including 2-thienyl, 3-thienyl, and so on), pyridyl (including 2-pyridyl, 3-pyridyl, 4-pyridyl, and so on), pyrazinyl, pyrimidinyl (including 2-pyrimidinyl, 4-pyrimidinyl, and so on), benzothiazolyl (including 5-benzothiazolyl, and so on), purinyl, benzimidazolyl (including 2-benzimidazolyl, and so on), benzoxazolyl, indolyl (including 5-indolyl, and so on), isoquinolinyl (including 1-isoquinolinyl, 5-isoquinolinyl, and so on), quinoxalinyl (including 2-quinoxalinyl, 5-quinoxalinyl, and so on) or quinolinyl (including 3-quinolinyl, 6-quinolinyl, and so on).

Unless otherwise stated, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂. Cₙ₋ₙ₊ₘ or Cₙ-Cₙ₊ₘ also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, and so on; in the same way, n membered to n+m membered represents that the number of atoms on the ring ranges from n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring and 12 membered ring. Any range from n to n+m is also included. For example, 3-12 membered ring includes 3-6 membered ring, 3-9 membered ring, 5-6 membered ring, 5-7 membered ring, 6-7 membered ring, 6-8 membered ring, 6-10 membered ring, and so on.

The term "leaving group" refers to a functional group or atom that can be substituted by another functional group or atom through a substitution reaction (for example, a nucleophilic substitution reaction). For example, representative leaving groups include trifluoromethanesulfonate; chlorine, bromine, and iodine; sulfonate such as mesylate, tosylate, p-bromobenzenesulfonate, p-toluenesulfonate, and so on; and acyloxy, such as acetoxy, trifluoroacetoxy, and so on.

The term "protecting group" includes, but is not limited to, "amino protecting group", "hydroxy protecting group" or "thiol protecting group". The term "amino protecting group" refers to a protecting group suitable for preventing a side reaction at an amino nitrogen position. Representative amino protecting groups include, but are not limited to, formyl; acyl, such as alkanoyl (e.g., acetyl, trichloroacetyl or trifluoroacetyl); alkoxycarbonyl, such as tert-butoxycarbonyl (Boc); arylmethoxycarbonyl, such as benzyloxycarbonyl (Cbz) and 9-fluorenylmethyloxycarbonyl (Fmoc); arylmethyl, such as benzyl (Bn), trityl (Tr), 1,1-di-(4'-methoxyphenyl)methyl; silyl, such as trimethylsilyl (TMS), tert-butyldimethylsilyl (TBS), and so on. The term "hydroxyl protecting group" refers to a protecting group suitable for preventing a side reaction of a hydroxyl. Representative hydroxy protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm) and diphenylmethyl (DPM); and silyl, such as trimethylsilyl (TMS), tert-butyldimethylsilyl (TBS), and so on.

The compound of the present disclosure can be prepared by varioussynthetic methods well known to those skilled in the art, including the specific embodiments listed below, the embodiments formed in combination with other chemical synthesis methods, and equivalent alternatives well known to those skilled in the art. Preferred embodiments include, but are not limited to, the embodiments of the present disclosure.

The solvents used in the present disclosure are commercially available. The present disclosure uses the following abbreviations: aq represents aqueous; HATU represents O-(7-aza-1H-benzotriazole-1-yl)-*N,N,N',N'*-tetramethyluroniumhexafluorophosphate; EDCI represents N-(3-dimethylaminopropyl)-N'-ethylcarbodiimide hydrochloride; m-CPBA represents 3-chloroperoxybenzoic acid; eq represents equivalent; CDI represents carbonyldiimidazole; DCM represents dichloromethane; PE represents petroleum ether; DIAD represents diisopropylazodicarboxylate; DMF represents N,N-dimethylformamide; DMSO represents dimethyl sulfoxide; EtOAc represents ethyl acetate; EtOH represents ethanol; MeOH represents methanol; CBz represents benzyloxycarbonyl, which is an amine protecting group; BOC represents tert-butyloxycarbonyl, which is an amine protecting group; HOAc represents acetic acid; NaCNBH₃ represents sodium cyanoborohydride; r.t. represents room temperature; O/N represents overnight; THF represents tetrahydrofuran; Boc₂O represents di-tert-butyl dicarbonate; TFA represents trifluoroacetic acid; SOCl₂ represents thionyl chloride; CS₂ represents carbon disulfide; TsOH represents p-toluenesulfonic acid; NFSI represents N-fluoro-N-(phenylsulphonyl)benzenesulfonamide; NCS represents 1-chloropyrrolidine-2,5-dione; *n*-Bu₄NF represents tetrabutylammonium fluoride; iPrOH represents 2-propanol; mp represents melting point; LDA represents lithium diisopropylamide; LiHMDS represents lithium hexamethyldisilazide; Xantphos represents 4,5-bis(diphenylphosphino)-9,9-dimethyl-xanthne; LiAlH₄ represents lithium aluminum hydride; Pd₂(dba)₃ represents tris(dibenzylideneacetone)dipalladium; Pd(dppf)Cl₂ represents [1,1'-bis(diphenylphosphino)ferrocene]dichloropalladium; DIEA represents N,N-diisopropylethylamine; Pd(PPh₃)₄ represents tetrakistriphenylphosphine palladium; IPA represents isopropanol; and DEA represents diethylamine.

The compounds are named according to conventional naming principles in the field or using ChemDraw^{®} software, and commercially available compounds are expressed in names in a supplier catalog.

### TECHNICAL EFFECTS

Compared with the control compounds, the compounds of the present disclosure witness a significant increase of the exposure amount of active drugs, the peak blood concentration and the action duration. The compounds of the present disclosure exhibit a larger reduction magnitude of intraocular pressure and a longer intraocular pressure reduction action duration. In the model of acute ocular hypertension, the compounds of the present disclosure all exhibited good hypotensive effects at different test doses and also showed certain dose correlation, and the hypotensive amplitude and the action duration were both superior to K-115. The metabolite concentration of the compounds of the present disclosure was 0.934 ng/mL in 4 hours after the administration at a high dose of 8mg/mL; and in 8 hours after the administration, the metabolite concentration of the compounds was below the detection limit, indicating high system safety.

### DETAILED DESCRIPTION OF THE EMBODIMENTS

The following examples will describe the present disclosure in detail, but are not intended to impose any unfavorable limitation on the present disclosure. The present disclosure has been described in detail herein, and its specific examples are also disclosed. It will be obvious for those skilled in the art that various changes and modifications may be made to the examples of the present disclosure without departing from the spirit and scope of the present disclosure.

### Synthesis of fragments of the technical concentrate

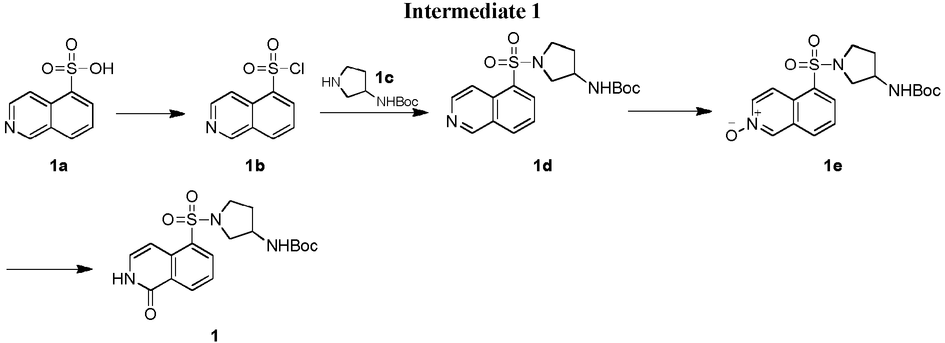

### Step 1

To a solution of compound **1a** (15.0 g, 71.7 mmol) in thionyl chloride (164.0 g, 1.38 mol) was added N,N-dimethylformamide (0.55 mL), stirred for 12 hours at 80 °C. After the reaction was completed, the reaction mixture was concentrated under reduced pressure to obtain a crude compound **1b**. MS-ESI calculated value [M+H]⁺228, actually measured value 228.

### Step 2

At 0 °C, to a solution of compound 1b (18.9 g, 62.0 mmol) in dichloromethane (150 mL) was added compound 1c (12.7 g, 68.2 mmol) and N,N-diisopropylethylamine (124 mL) under a nitrogen atmosphere, stirred for 0.5 hours at 25 °C. After the reaction was completed, the reaction solution, after being concentrated under reduced pressure, was diluted with water (100 mL), and extracted with dichloromethane (60 mL × 3), and the combined organic phase was washed with a saturated sodium chloride solution, dried with anhydrous sodium sulfate, and concentrated under reduced pressure after filtration. The residue was purified by silica gel column chromatography to obtain compound **1d.** MS-ESI calculated value [M+H]⁺378, actually measured value 378.

### Step 3

At 0 °C, to a solution of compound **1d** (8 g, 20.3 mmol) in dichloromethane (100 mL) was added chloroperoxybenzoic acid (6.48 g, 30.4 mmol, purity: 81%) in batch, stirred for 2 hours at 25 °C. After the reaction was completed, the reaction mixture was quenched with saturated sodium bicarbonate solution (100 mL), extracted with dichloromethane (60 mL×3), and the organic phase was dried by anhydrous sodium sulfate, then filtered, concentrated under reduced pressure to obtain compound 1e. MS-ESI calculated value [M+H]⁺394, actually measured value 394.

### Step 4

Compound **1e** (11.1 g, 28.2 mmol) was dissolved in acetic anhydride (109 g, 1.07 mol), and after stirred for 2 hours at 130 °C, acetic anhydride was removed by concentration. Tetrahydrofuran (100 mL) and 30% aqueous sodium hydroxide solution (150 mL) were added, stirred for 30 minutes at 25 °C. After removal of tetrahydrofuran by concentration under reduced pressure, a 6 M hydrochloric acid solution was added dropwise to the system to adjust the pH of the reaction solution to 7, extracted with ethyl acetate (50 mL ×3), and the combined organic phase dried with anhydrous sodium sulfate, filtered, and concentrated under reduced pressure. The residue was purified by silica gel column chromatography to obtain intermediate 1. ¹H NMR (400 MHz, DMSO-*d*₆) δ = 8.52 (d, *J* = 7.8 Hz, 1 H), 8.21 (dd, *J* = 7.8, 1.3 Hz, 1 H), 7.64 (t, *J* = 7.8 Hz, 1 H), 7.37 (d, *J* = 7.8 Hz, 1 H), 7.23 (d, *J* = 7.6 Hz, 1 H), 3.91 (d, *J* = 5.4 Hz, 1 H), 3.20-3.28 (m, 3 H), 3.03 (dd, *J* = 9.8, 5.0 Hz, 1 H), 1.92-2.05 (m, 1 H), 1.68-1.77 (m, 1 H), 1.34 (s, 9 H). MS-ESI calculated value [M+Na]⁺416, actually measured value 416.

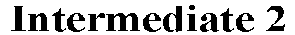

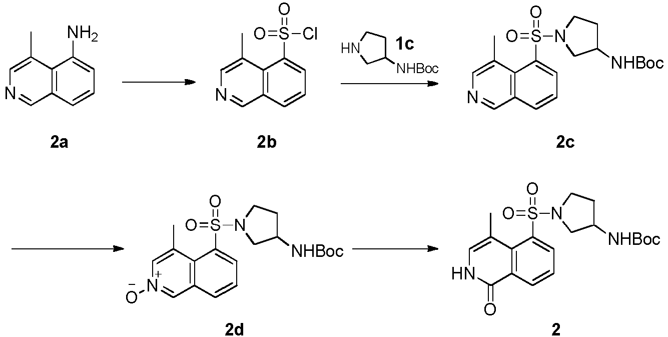

### Step 1

To a solution of compound **2a** (5.0 g, 36.7 mmol) in concentrated hydrochloric acid (50 mL, 12 M) was added slowly the solution of sodium nitrite (2.29 g, 33.2 mmol) in water (8 mL) at -5 °C, stirred for 1 hour at -5 °C. To a solution of cuprous chloride (156 mg, 1.58 mmol) and cupric chloride (2.34 g, 17.4 mmol) in glacial acetic acid (80 mL) was introduced sulphur dioxide until saturation, and then the previous reaction solution was slowly added dropwise to the solution containing sulfur dioxide at -5 °C, stirred for 1 hour at 20 °C. After the reaction was completed, water (150 mL) was added to the reaction solution, extracted with dichloromethane (150 mL×4). The combined organic phase was washed with saturated brine (500 mL×1), dried with anhydrous sodium sulfate, filtered, the filtrate was concentrated under reduced pressure to obtain a residue, and the residue crude **2b** was directly used for the next step without purification. MS-ESI calculated value [M+H]⁺ 242, actually measured value 242.

### Step 2

Compound **2c** was obtained referring to step 2 of intermediate **1.**

### Step 3

Compound **2d** was obtained referring to step 3 of intermediate **1.**

### Step 4

Intermediate **2** was obtained referring to step 4 of intermediate **1.** MS-ESI calculated value [M+H]⁺408, actually measured value 408.

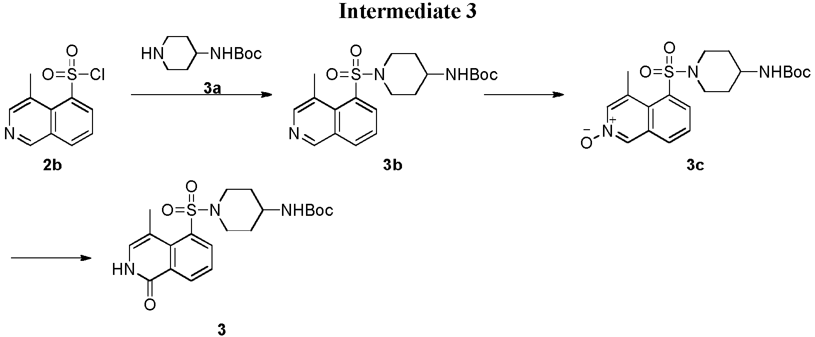

### Step 1

Compound **3b** was obtained referring to step 2 of intermediate **1.** MS-ESI calculated value [M+H]⁺ 406, actually measured value 406.

### Step 2

Compound **3c** was obtained referring to step 3 of intermediate **1.** MS-ESI calculated value [M+H]⁺ 422, actually measured value 422.

### Step 3

Intermediate **3** was obtained referring to step 4 of intermediate 1. MS-ESI calculated value [M+H]⁺ 422, actually measured value 422.

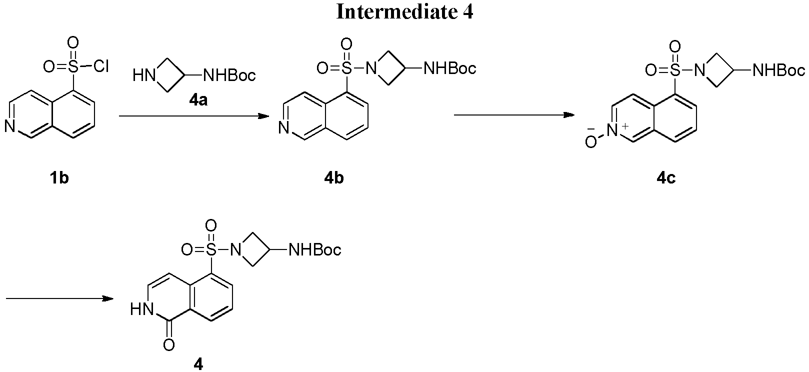

### Step 1

Compound **4b** was obtained referring to step 2 of intermediate 1. MS-ESI calculated value [M+H]⁺ 364, actually measured value 364.

### Step 2

Compound **4c** was obtained referring to step 3 of intermediate 1. MS-ESI calculated value [M+H]⁺ 380, actually measured value 380.

### Step 3

Intermediate 4 was obtained referring to step 4 of intermediate **1.** MS-ESI calculated value [M+Na]⁺ 402, actually measured value 402.

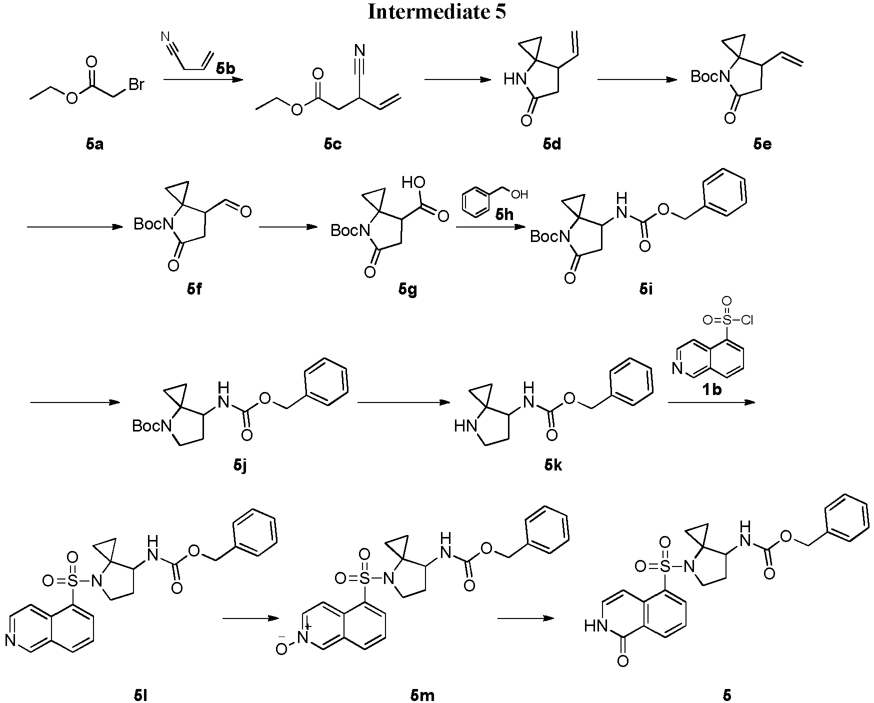

### Step 1

To a solution of diisopropylamine (36.4 g, 359 mmol) in tetrahydrofuran (1.5 mL) was added dropwise n-butyl lithium (2.5 M, 131.2 mL) at -78 °C under the nitrogen atmosphere; after stirred for 1 hour at -78 °C, compound **5b** (20.1 g, 299 mmol) was added dropwise to the reaction solution at -78 °C under the nitrogen atmosphere; after stirred for 1 hour at -78 °C, compound **5a** (50 g, 299 mmol) was added dropwise to the reaction solution at -78 °C under the nitrogen atmosphere, stirred for 1 hour at -78 °C under the nitrogen atmosphere. After the reaction was completed, quenched with saturated aqueous ammonium chloride solution (2 L) and extracted with ethyl acetate (500 mL ×3). The combined organic phase was washed with saturated brine (1 L×1) and dried with anhydrous sodium sulfate, filtered, the filtrate was concentrated under reduced pressure, and the crude product was separated and purified by silica gel column chromatography (petroleum ether/ethyl acetate) to obtain compound **5c.** ¹H NMR (400MHz, CDCl₃) δ = 5.83-5.70 (m, 1H), 5.50 (dd, *J* = 1.5, 16.9 Hz, 1H), 5.36-5.30 (m, 1H), 4.22-4.17 (m, 2H), 3.77-3.70 (m, 1H), 2.81-2.71 (m, 1H), 2.68-2.59 (m, 1H), 1.29-1.26 (m, 3H).

### Step 2

To a solution of compound **5c** (60.7 g, 396 mmol) in tetrahydrofuran (1.2 L) was added tetraisopropyltitanate (113 g, 396 mmol) under the nitrogen atmosphere, and added dropwise ethyl magnesium bromide (89.8 g, 674 mmol) at 20 °C under the nitrogen atmosphere, stirred for 3 hours at 20 °C. After the reaction was completed, quenched with water (300 mL) and filtered, the filtrate was concentrated under reduced pressure, and the crude product was separated and purified by silica gel column chromatography (petroleum ether/ethyl acetate) to obtain compound **5d.** MS-ESI calculated value [M+H]⁺ 138, actually measured value 138.

### Step 3

To a solution of compounds **5d** (81.0g, 590.5 mmol) in acetonitrile (500 mL) was added 4-dimethylaminopyridine (7.21g, 59.1 mmol) and di-tert-butyl dicarbonate (155 g, 709 mmol) successively , stirred for 3 hours at 20 °C. After the reaction was completed, quenched with water (1 L) and extracted with ethyl acetate (500 mL ×3). The combined organic phase was washed with saturated brine (500 mL×1) and dried with anhydrous sodium sulfate, filtered, the filtrate was concentrated under reduced pressure, and the crude product was separated and purified by silica gel column chromatography (petroleum ether/ethyl acetate) to obtain compound **5e.** MS-ESI calculated value [M+H]⁺ 238, actually measured value 238.

### Step 4

To a solution of compound **5e** (10.8 g, 45.5 mmol) in methanol (48 mL) and water (72 mL) was added sodium periodate (29.2 g, 136.4 mmol) and osmium tetraoxide (173 mg, 682 µmol) at 20 °C, stirred for 2 hours at 20 °C. After the reaction was completed, water (100 mL) was added to the reaction solution and the reaction solution was extracted with ethyl acetate (100 mL ×3). The combined organic phase was washed with saturated brine (100 mL×1)and dried with anhydrous sodium sulfate, filtered, the filtrate was concentrated under reduced pressure, and the crude product was separated and purified by silica gel column chromatography (petroleum ether/ethyl acetate) to obtain compound **5f.**

### Step 5

To a solution of compound **5f** (4.00 g, 16.7 mmol) in tert-butanol (50 mL) and tetrahydrofuran (50 mL) was added isopentene (14.6 g, 208 mmol), sodium hypochlorite (1.66 g, 18.4 mmol), sodium phosphate monobasic monohydrate (4.61 g, 33.4 mmol) and water (35 mL) successively at 0 °C, stirred for 12 hours at 20 °C. After the reaction was completed, the pH of the reaction solution was adjusted to 4 with 1N aqueous hydrochloric acid solution and the reaction solution was extracted with ethyl acetate (50 mL ×3). The combined organic phase was washed with saturated brine (100 mL×1) and dried with anhydrous sodium sulfate, and filtered, the filtrate was concentrated under reduced pressure to obtain a residual. Water (50 mL) was added to the residue, aqueous Na₂CO₃ solution was used to adjust the pH to 10, and ethyl acetate (50 mL ×1) was used for extraction to collect aqueous phase. The pH of the aqueous phase was adjusted to 4 with the 1N aqueous hydrochloric acid solution and the aqueous phase was extracted with ethyl acetate (50 mL ×3). The combined organic phase was washed with saturated brine (100 mL×1), dried with anhydrous sodium sulfate and filtered, and the filtrate was concentrated under reduced pressure to obtain compound **5g.**

### Step 6

To a solution of compound **5g** (3.10 g, 12.1 mmol) in toluene (30 mL) was added N,N-diisopropylethylamine (2.04 g, 15.8 mmol), diphenylphosphorylazide (4.34g, 15.8 mmol, 3.42 ml) and a 4A molecular sieve (2.00 g) at 0 °C, stirred for 0.5 hours at 20 °C, and stirred for another 0.5 hour at 90 °C, then cooled to 20 °C. Compound **5h** (1.44 g, 13.4 mmol) was added to the reaction solution at 20 °C, stirred the reaction solution for 12 hours at 20 °C. After the reaction was completed, the reaction solution was filtered, water (30 mL) was added to the filtrate and extracted with ethyl acetate (30 mL ×2). The combined organic phase was washed with saturated brine (50 mL×1) and dried with anhydrous sodium sulfate, filtered, the filtrate was concentrated under reduced pressure, and the crude product was separated and purified by silica gel column chromatography (petroleum ether/ethyl acetate) to obtain compound **5i.**

### Step 7

To a solution of compound **5i** (500 mg, 1.39 mmol) in tetrahydrofuran (5 mL) was added borane-tetrahydrofuran (1 M, 13.9 mL) at 0 °C under the nitrogen atmosphere, stirred for 1 hour at 25 °C. After the reaction was completed, quenched with saturated aqueous ammonium chloride solution (20 mL), and extracted with ethyl acetate (20 mL ×3). The combined organic phase was washed with saturated brine (20 mL×1) and dried with anhydrous sodium sulfate, filtered, the filtrate was concentrated under reduced pressure, and the crude product was separated and purified by preparative thin layer chromatography (petroleum ether/ethyl acetate) to obtain compound **5j.**

### Step 8

To a solution of Compound **5j** (295 mg, 852 µmol) in ethyl acetate (3 mL) was added ethyl acetate hydrochloride (4 M, 5 mL), stirred for 0.5 hour at 25 °C. After the reaction was completed, the reaction solution was concentrated under reduced pressure to obtain compound **5k.**

### Step 9

To a solution of compound **5k** (420 mg, 1.59 mmol) in dichloromethane (10 mL) was added compound **1b** (268 mg, 948 µmol) and N,N-diisopropylethylamine (489.98 mg, 3.79 mmol) at 0 °C under the nitrogen atmosphere, stirred the reaction mixture for 12 hours at 25 °C. After the reaction was completed, dichloromethane (10 mL) and water (15 mL) were added to the reaction solution, extracted with dichloromethane (10mL × 2); and the combined organic phase was washed with saturated brine (20 mL×1), dried with anhydrous sodium sulfate, filtered and concentrated under reduced pressure. The crude product was separated and purified by silica gel plate chromatography (petroleum ether/ethyl acetate) to obtain compound **5l**. MS-ESI calculated value [M+H]⁺ 438, □ □ □ ally measured value 438.

### Step 10

Compound **5m** was obtained referring to step 3 of intermediate 1. MS-ESI calculated value [M+H]⁺ 454, actually measured value 454.

### Step 11

Intermediate **5** was obtained referring to step 4 of intermediate 1. MS-ESI calculated value [M+H]⁺ 454, actually measured value 454.

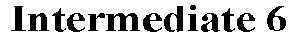

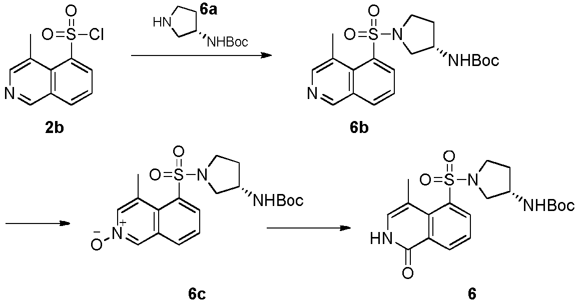

### Step 1

Compound 6b was obtained referring to step 2 of intermediate 1.

### Step 2

Compound 6c was obtained referring to step 3 of intermediate 1.

### Step 3

Intermediate 6 was obtained referring to step 4 of intermediate 1. MS-ESI calculated value [M+H]⁺408, actually measured value 408.

### Example 1: Compound 7

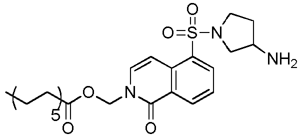

### Synthesis route:

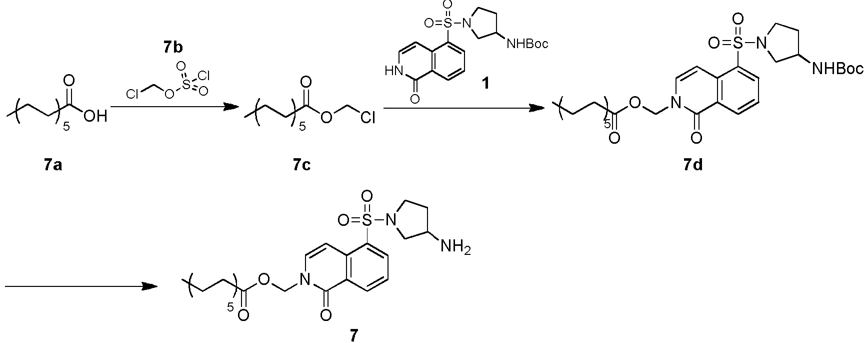

### Step 1

To a solution of compound **7a** (1 g, 4.99 mmol) in dichloromethane (30 mL) and water (30 mL) was added sodium bicarbonate (1.68 g, 12.0 mmol) and tetrabutylammonium fluoride (1 M, 499 µL), then a solution of compound **7b** (824 mg, 4.99 mmol) in dichloromethane (10 mL) was added to the reaction system under the condition of stirring at 0 °C, stirred for 16 hours at 30 °C. The organic phase was separated, dried with anhydrous magnesium sulfate and filtered, concentrated under reduced pressure to obtain compound **7c.**

### Step 2

At 0 °C, to a solution of intermediate **1** (150 mg, 324 µmol) in N,N-dimethylformamide (6 mL) was added sodium hydride (15.6 mg, 389 µmol, purity: 60%) under nitrogen atmosphere, then added compound **7c** (113 mg, 454 µmol) after stirred for 0.5 hour, warmed to 25 °C stirred for 2 hours. Ice water (30mL) was slowly added to the reaction solution, extracted with ethyl acetate (20 mL ×3), and the organic phase was dried with anhydrous sodium sulfate, filtered, concentrated under reduced pressure. The residue was purified by sillica gel plate to obtain compound **7d.** MS-ESI calculated value [M+Na]⁺628, actually measured value 628.

### Step 3

To a solution of compound **7d** (96.0 mg, 154 µmol) in dichloromethane (20 mL) was added trimethylsllytrifluoromethanesulphonate (68.6 mg, 309 µmol), then stirred for 1 hour at 0-5 °C. 2,6-dimethylpyridine (49.6 mg, 462 µmol) was added dropwise, stirred for another 1 hour at 0-5 °C. Water (30 mL) was added to the reaction solution, extracted with dichloromethane (20 mL ×3) , and the organic phase was dried with anhydrous sodium sulfate, filtered, concentrated under reduced pressure. The residue was purified by preparative high performance liquid (formic acid condition) to obtain a formate of compound **7.** ¹H NMR (400MHz, CD₃OD) δ = 8.70 *(d, J* = 7.8 Hz, 1 H), 8.52 (s, 1 H),8.39 (dd, *J* = 7.8, 1.3 Hz, 1 H), 7.74 *(t, J* = 7.8 Hz, 1 H), 7.67 (d, *J* = 7.8 Hz, 1 H), 7.51 (d, *J* = 7.8 Hz, 1 H), 6.00 (s, 2 H), 3.81-3.90 (m, 1 H), 3.57-3.66 (m, 1 H), 3.49-3.56 (m, 1 H), 3.42 (dd, *J* = 10.8, 4.1 Hz, 1 H), 3.30 (d, *J* = 6.2 Hz, 1 H), 2.39 (t, *J* = 7.4 Hz, 2H), 2.26-2.36 (m, 1 H), 1.92-2.03 (m, 1 H), 1.55-1.69 (m, 1 H), 1.55-1.69 (m, 2 H), 1.20-1.34 (m, 16 H), 0.91 *(t, J* = 6.8 Hz, 3 H). MS-ESI calculated value [M+H]⁺506, actually measured value 506.

The formate of compound **7** can be adjusted the pH of the reaction solution to 8-9 by adding a saturated aqueous sodium carbonate solution to the system, and then, after the extraction with ethyl acetate, the reaction solution was concentrated to obtain compound **7.**

### Example 2: Compound 8

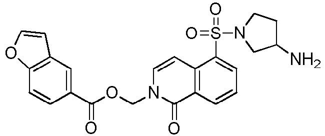

### Synthesis route:

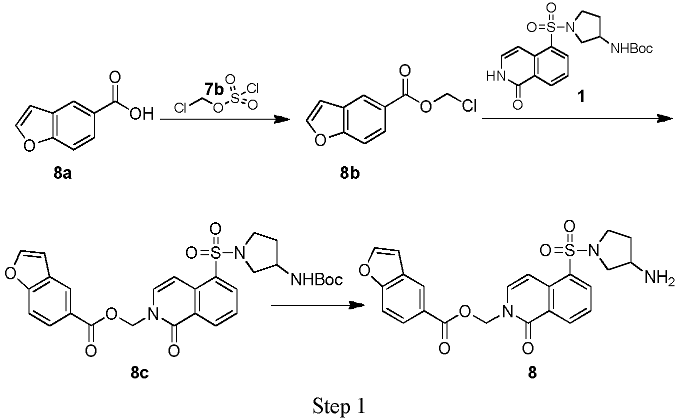

Compound 8b was obtained referring to step 1 of Example 1. ¹H NMR (400MHz, CDCl₃) δ = 8.27 (d, *J* = 1.6 Hz, 1 H), 7.94 (dd, *J* = 8.8, 1.7 Hz, 1 H), 7.60 (d, *J* = 2.4 Hz, 1 H), 7.44 (d, *J* = 8.8 Hz, 1 H), 6.74 (dd, *J* = 2.2, 0.7 Hz, 1 H), 5.89 (s, 2 H).

### Step 2

Compound **8c** was obtained referring to step 2 of Example **1.** MS-ESI calculated value [M+Na]⁺590, actually measured value 590.

### Step 3

A formate of compound **8** was obtained referring to step 3 of Example 1. ¹H NMR (400MHz, CD₃OD) δ = 8.72 (d, *J* = 7.8 Hz, 1 H), 8.48 (s, 1 H), 8.37-8.42 (m, 2 H), 8.04 (dd, *J* = 8.8, 1.7 Hz, 1 H), 7.88 (d, *J* = 2.2 Hz, 1 H), 7.82 (d, *J* = 7.8 Hz, 1 H), 7.73 (t, *J* = 7.8 Hz, 1 H), 7.58 (dd, *J* = 19.8, 8.4 Hz, 2 H), 6.97 (dd, *J* = 2.2, 0.7 Hz, 1 H), 6.27 (s, 2 H), 3.72-3.83 (m, 1 H), 3.49-3.63 (m, 2 H), 3.35 (d, *J* = 1.4 Hz, 1 H), 2.20-2.33 (m, 1 H), 1.85-1.97 (m, 1 H). MS-ESI calculated value [M+H]⁺468, actually measured value 468.

The formate of compound **8** can be adjusted the pH of the reaction solution to 8-9 by adding a saturated aqueous sodium carbonate solution to the system, and then, after the extraction with ethyl acetate, the reaction solution was concentrated to obtain compound **8.**

### Example 3: Compound 9

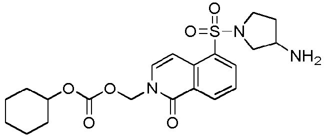

### Synthesis route:

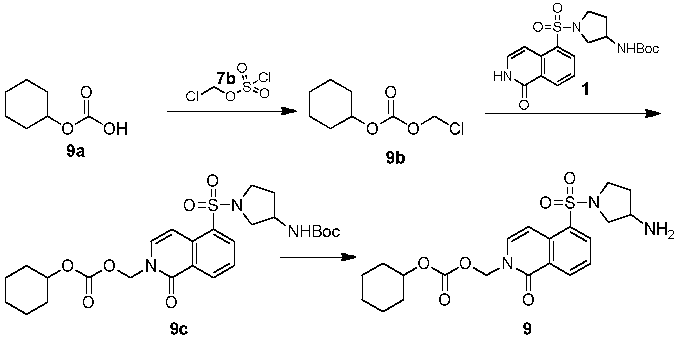

### Step 1

Compound **9b** was obtained referring to step 1 of Example 1. ¹H NMR (400MHz, CDCl₃) δ = 5.72 (s, 2 H), 4.63-4.77 (m, 1 H), 1.93 (dd, *J* = 12.5, 3.9 Hz, 2 H), 1.75 (dd, *J* = 9.2, 3.9 Hz, 2 H), 1.45-1.58 (m, 3 H), 1.23-1.42 (m, 3 H).

### Step 2

Compound **9c** was obtained referring to step 2 of Example 1. MS-ESI calculated value [M+Na]⁺572, actually measured value 572.

### Step 3

A formate of compound **9** was obtained referring to step 3 of Example **1.** ¹H NMR (400MHz, CD₃OD) δ = 8.68 (d, *J* = 8.0 Hz, 1 H), 8.46 (s, 1 H), 8.39 (dd, *J* = 7.8, 1.2 Hz, 1 H), 7.73 (t, *J* = 7.8 Hz, 1 H), 7.67 (d, *J* = 7.8 Hz, 1 H), 7.51 *(d, J* = 7.8 Hz, 1 H), 6.00 (s, 2 H), 4.62-4.71 (m, 1 H), 3.77-3.87 (m, 1 H), 3.50-3.64 (m, 2 H), 3.34-3.40 (m, 2 H), 2.24-2.36 (m, 1 H), 1.86-2.03 (m, 3 H), 1.75 (dt, *J* = 6.4, 3.1 Hz, 2 H), 1.24-1.61 (m, 6 H). MS-ESI calculated value [M+H]⁺450, actually measured value 450.

The formate of compound **9** can be adjusted the pH of the reaction solution to 8-9 by adding saturated aqueous sodium carbonate solution to the system, and then, after the extraction with ethyl acetate, the reaction solution was concentrated to obtain compound **9.**

### Example 4: Compound 10

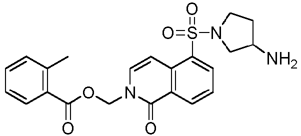

### Synthesis route:

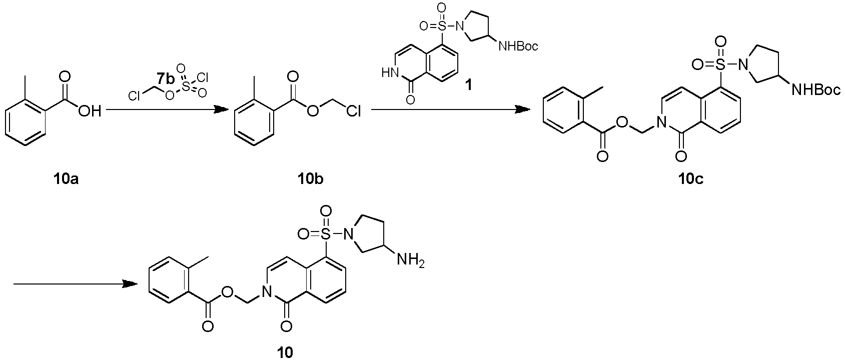

### Step 1

Compound **10b** was obtained referring to step 1 of Example 1. ¹H NMR (400MHz, CDCl₃) δ = 7.99 (dd, *J* = 8.2, 1.3 Hz, 1 H), 7.47 (td, *J* = 7.4, 1.4 Hz, 1 H), 7.25-7.32 (m, 2 H), 5.96 (s, 2 H), 2.65 (s, 3 H).

### Step 2

Compound **10c** was obtained referring to step 2 of Example 1. MS-ESI calculated value [M+Na]⁺564, actually measured value 564.

### Step 3

A formate of compound **10** was obtained referring to step 3 of Example 1. ¹H NMR (400MHz, CD₃OD) δ = 8.69 (d, *J* = 7.8 Hz, 1 H), 8.37 (dd, *J* = 7.6, 1.2 Hz, 1 H), 7.91 (d, *J* = 7.8 Hz, 1 H), 7.79 (d, *J* = 7.8 Hz, 1 H), 7.69-7.76 (m, 1 H), 7.53 *(d, J* = 7.8 Hz, 1 H), 7.45 (td, *J* = 7.4, 1.2 Hz, 1 H), 7.22-7.33 (m, 2 H), 6.21 (s, 2 H), 3.81-3.92 (m, 1 H), 3.51-3.64 (m, 2 H), 3.41 (dd, *J* = 10.8, 4.2 Hz, 1 H), 3.32-3.33 (m, 1 H), 2.57 (s, 3 H), 2.24-2.40 (m, 1 H), 1.90-2.04 (m, 1 H). MS-ESI calculated value [M+H]⁺442, actually measured value 442.

The formate of compound **10** can be adjusted the pH of the reaction solution to 8-9 by adding a saturated aqueous sodium carbonate solution to the system, and then, after the extraction with ethyl acetate, the reaction solution was concentrated to obtain compound **10.**

### Example 5: Compound 11

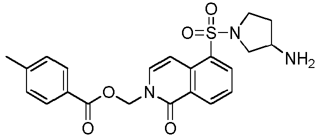

### Synthesis route:

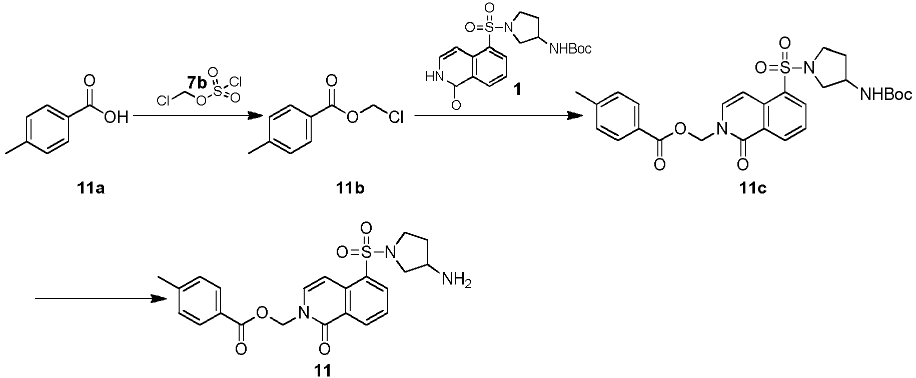

### Step 1

Compound **11b** was obtained referring to step 1 of Example **1.** ¹H NMR (400MHz, CDCl₃) δ = 7.94-7.98 (m, 2 H), 7.25 (d, *J* = 8.4 Hz, 2H), 5.93 (s, 2 H), 2.40 (s, 3 H).

### Step 2

Compound **11c** was obtained referring to step 2 of Example **1.** MS-ESI calculated value [M+Na]⁺564, actually measured value 564.

### Step 3

A formate of compound **11** was obtained referring to step 3 of Example **1.** ¹H NMR (400MHz, CD₃OD) δ = 8.72 (d, *J* = 8.6 Hz, 1 H), 8.39 (d, *J* = 7.6 Hz, 1 H), 7.94 (d, *J* = 8.0 Hz, 2 H), 7.68-7.84 (m, 2 H), 7.54 (d, *J* = 7.8 Hz, 1 H), 7.31 *(d, J* = 7.8 Hz, 2 H), 6.23 (s, 2 H), 3.84 (s, 1 H), 3.49-3.64 (m, 2 H), 3.39 (d, *J* = 15.2 Hz, 2 H), 2.42 (s, 3 H), 2.30 (s, 1 H), 1.97 (s, 1 H). MS-ESI calculated value [M+H]⁺442, actually measured value 442.

The formate of compound **11** can be adjusted the pH of the reaction solution to 8-9 by adding a saturated aqueous sodium carbonate solution to the system, and then, after the extraction with ethyl acetate, the reaction solution was concentrated to obtain compound **11.**

### Example 6: Compound 12

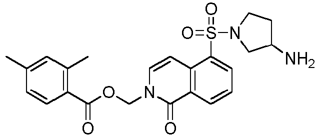

### Synthesis route:

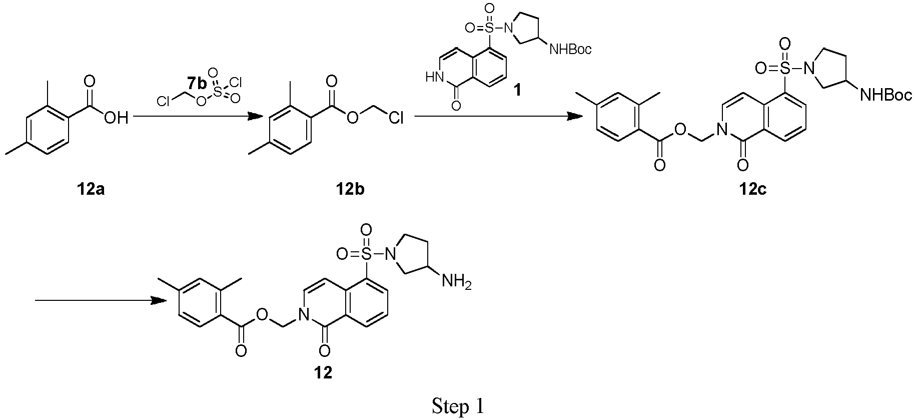

Compound **12b** was obtained referring to step 1 of Example **1.** ¹H NMR (400MHz, CDCl₃) δ = 7.92 (d, *J* = 7.8 Hz, 1 H), 7.05-7.15 (m, 2 H), 5.95 (s, 2 H), 2.63 (s, 3 H), 2.39 (s, 3 H).

### Step 2

Compound **12c** was obtained referring to step 2 of Example **1.** MS-ESI calculated value [M+Na]⁺578, actually measured value 578.

### Step 3

Compound **12** was obtained referring to step 3 of Example **1.** ¹H NMR (400MHz, CD₃OD) δ = 8.73 (d, *J* = 7.8 Hz, 1 H), 8.40 (dd, *J* = 7.8, 1.3 Hz, 1 H), 7.83 (dd, *J* = 18.2, 8.0 Hz, 2 H), 7.74 (t, *J* = 7.8 Hz, 1 H), 7.55 (d, *J* = 7.8 Hz, 1 H), 7.14 (s, 1 H), 7.09 (d, *J* = 8.4 Hz, 1 H), 6.20 (s, 2 H), 3.79-3.89 (m, 1 H), 3.50-3.65 (m, 2 H), 3.35-3.45 (m, 2 H), 2.56 (s, 3 H), 2.35 (s, 3 H), 2.24-2.34 (m, 1 H), 1.90-2.03 (m, 1 H). MS-ESI calculated value [M+H]⁺456, actually measured value 456.

### Example 7: Compound 13

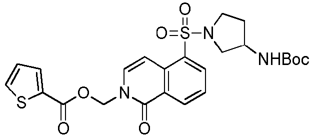

### Synthesis route:

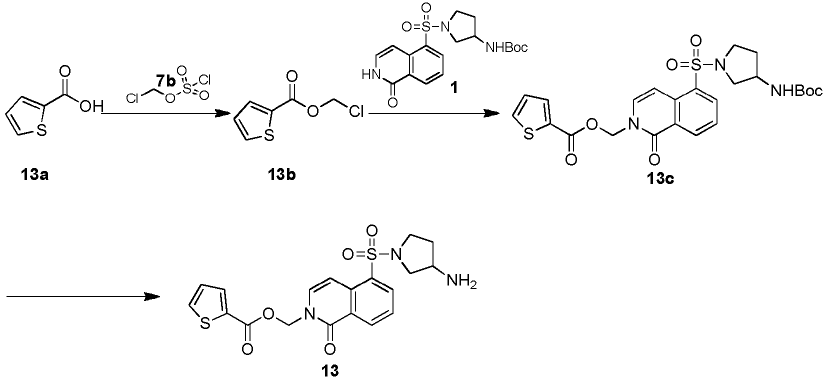

### Step 1

Compound **13b** was obtained referring to step 1 of Example 1. ¹H NMR (400MHz, CDCl₃) δ = 7.79-7.85 (m, 1 H), 7.59 (dd, *J* = 4.8, 1.2 Hz, 1 H), 7.04-7.10 (m, 1 H), 5.79-5.90 (m, 2 H)

### Step 2

Compound **13c** was obtained referring to step 2 of Example 1. MS-ESI calculated value [M+Na]⁺556, actually measured value 556.

### Step 3

A formate of compound **13** was obtained referring to step 3 of Example **1.** ¹H NMR (400 MHz, CD₃OD) δ = 8.71 (d, *J* = 7.8 Hz, 1 H), 8.40 (s, 1 H),8.39 (dd, *J* = 7.8, 1.3 Hz, 1 H), 7.89 (dd, *J* = 3.8, 1.2 Hz, 1 H), 7.83 (dd, *J* = 5.0, 1.2 Hz, 1 H), 7.69-7.79 (m, 2 H), 7.54 *(d, J* = 7.8 Hz, 1 H), 7.19 (dd, *J* = 5.0, 3.9 Hz, 1 H), 6.21 (s, 2 H), 3.70-3.83 (m, 1 H), 3.48-3.65 (m, 2 H), 3.33-3.35 (m, 2 H), 2.21-2.35 (m, 1 H), 1.84-1.99 (m, 1 H). MS-ESI calculated value [M+H]⁺434, actually measured value 434.

The formate of compound **13** can be adjusted the pH of the reaction solution to 8-9 by adding a saturated aqueous sodium carbonate solution to the system, and then, after the extraction with ethyl acetate, the reaction solution was concentrated to obtain compound **13.**

### Example 8: Compound 14

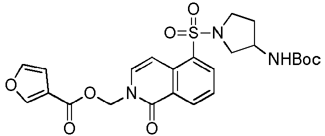

### Synthesis route:

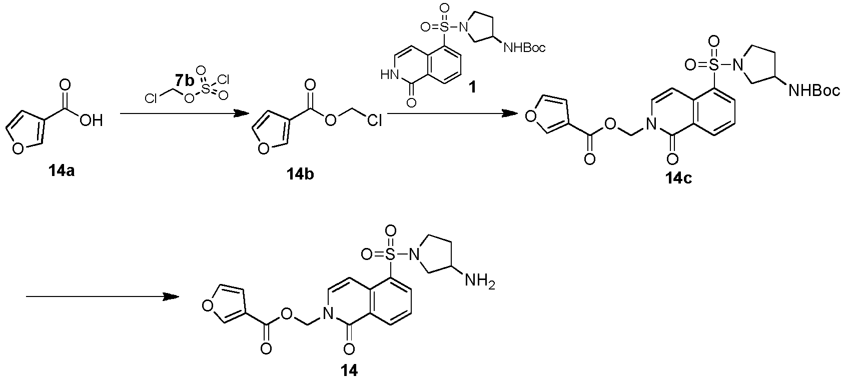

### Step 1

Compound 14b was obtained referring to step 1 of Example 1. ¹H NMR (400MHz, CDCl₃) δ = 7.98-8.05 (m, 1 H), 7.37-7.42 (m, 1 H), 6.71 (dd, *J* = 1.8, 0.7 Hz, 1 H), 5.81 (s, 2 H).

### Step 2

Compound **14c** was obtained referring to step 2 of Example 1. MS-ESI calculated value [M+Na]⁺540, actually measured value 540.

### Step 3

A formate of compound 14 was obtained referring to step 3 of Example 1. ¹H NMR (400MHz, CD₃OD) δ = 8.70 (d, *J* = 7.8 Hz, 1 H), 8.49 (s, 1 H), 8.39 (dd, *J* = 7.8, 1.3 Hz, 1 H), 8.23-8.26 (m, 1 H), 7.69-7.78 (m, 2 H), 7.62 (t, *J* = 1.8 Hz, 1 H), 7.53 (d, *J* = 7.8 Hz, 1 H), 6.80 (dd, *J* = 1.8, 0.7 Hz, 1 H), 6.17 (s, 2 H), 3.74-3.83 (m, 1 H), 3.50-3.62 (m, 2 H), 3.34-3.38 (m, 1 H), 3.29-3.32 (m, 1 H), 2.20-2.34 (m, 1 H), 1.85-1.99 (m, 1 H). MS-ESI calculated value [M+H]⁺418, actually measured value 418.

The formate of compound **14** can be adjusted the pH of the reaction solution to 8-9 by adding a saturated aqueous sodium carbonate solution to the system, and then, after the extraction with ethyl acetate, the reaction solution was concentrated to obtain compound **14.**

### Example 9: Compound 15

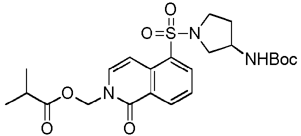

### Synthesis route:

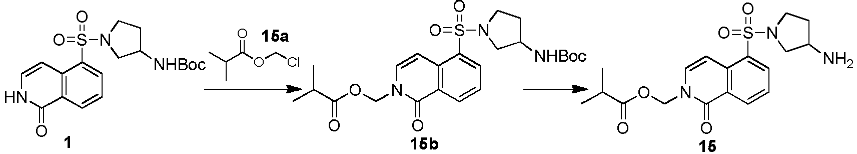

### Step 1

Compound **15b** was obtained referring to step 2 of Example 1. MS-ESI calculated value [M+Na]⁺516, actually measured value 516.

### Step 2

A formate of compound **15** was obtained referring to step 3 of Example 1. ¹H NMR (400MHz, CD₃OD) δ = 8.67 (d, *J* = 7.8 Hz, 1 H), 8.39 (s, 1 H), 8.37 (dd, *J* = 7.8, 1.3 Hz, 1 H), 7.72 (t, *J* = 7.8 Hz, 1 H), 7.66 (d, *J* = 7.8 Hz, 1 H), 7.50 *(d, J* = 7.8 Hz, 1 H), 5.99 (s, 2 H), 3.76-3.89 (m, 1 H), 3.50-3.65 (m, 2 H), 3.33 *(d, J* = 1.6 Hz, 2 H), 2.62 (spt, *J* = 7.0 Hz, 1 H), 2.22-2.37 (m, 1 H), 1.90-2.02 (m, 1 H), 1.17 (d, *J* = 7.0 Hz, 6 H), 0.00-0.00 (m, 1 H). MS-ESI calculated value [M+H]⁺394, actually measured value 394.

The formate of compound **15** can be adjusted the pH of the reaction solution to 8-9 by adding a saturated aqueous sodium carbonate solution to the system, and then, after the extraction with ethyl acetate, the reaction solution was concentrated to obtain compound 15.

### Example 10: Compound 16

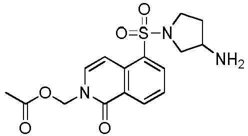

### Synthesis route:

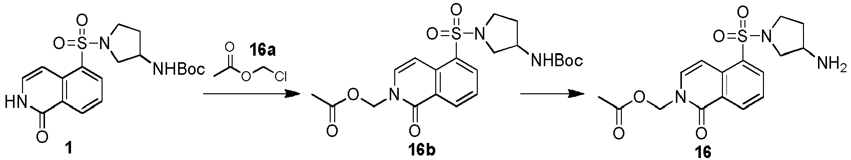

### Step 1

Compound 16b was obtained referring to step 2 of Example 1. ¹H NMR (400MHz, CDCl₃) δ = 8.72 (dd, *J* = 8.0, 1.2 Hz, 1 H), 8.32 (dd, *J* = 7.8, 1.2 Hz, 1 H), 7.59 (t, *J* = 8.0 Hz, 1 H), 7.34-7.48 (m, 2 H), 5.95 (s, 2 H), 4.66 (s, 1 H), 4.18 (s, 1 H), 3.37-3.61 (m, 2 H), 3.27 (s, 2 H), 2.09-2.18 (m, 4 H), 1.84 *(d, J* = 5.6 Hz, 1 H), 1.42 (s, 9 H).

### Step 2

A formate of compound 16 was obtained referring to step 3 of Example 1. ¹H NMR (400MHz, CD₃OD) δ = 8.68 (d, *J* = 7.6 Hz, 1 H), 8.44 (s, 1 H), 8.37 (dd, *J* = 7.6, 1.3 Hz, 1 H), 7.72 (t, *J* = 8.0 Hz, 1 H), 7.65 (d, *J* = 8.0 Hz, 1 H), 7.50 (d, *J* = 8.0 Hz, 1 H), 5.97 (s, 2 H), 3.75-3.88 (m, 1 H), 3.46-3.63 (m, 2 H), 3.32-3.43 (m, 2 H), 2.21-2.36 (m, 1 H), 2.09 (s, 3 H), 1.86-2.01 (m, 1 H). MS-ESI calculated value [M+H]⁺366, actually measured value 366.

The formate of compound **16** can be adjusted the pH of the reaction solution to 8-9 by adding a saturated aqueous sodium carbonate solution to the system, and then, after the extraction with ethyl acetate, the reaction solution was concentrated to obtain compound **16.**

### Example 11: Compound 17

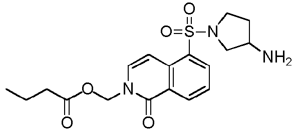

### Synthesis route:

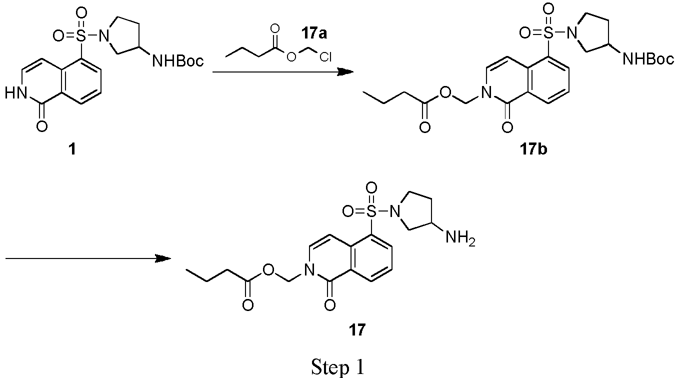

Compound **17b** was obtained referring to step 2 of Example **1.** ¹H NMR (400MHz, CDCl₃) δ = 8.66-8.78 (m, 1 H), 8.31 (dd, *J* = 7.8, 1.4 Hz, 1 H), 7.59 (t, *J* = 7.8 Hz, 1 H), 7.36-7.48 (m, 2 H), 5.96 (s, 2 H), 4.68 (s, 1 H), 4.14-4.30 (m, 1 H), 3.36-3.64 (m, 2 H), 3.27 (s, 2 H), 2.36 (t, *J* = 7.4 Hz, 2 H), 2.14 (td, *J* = 13.8, 6.8 Hz, 1 H), 1.78-1.88 (m, 1 H), 1.63-1.70 (m, 2 H), 1.42 (s, 9 H), 0.94 *(t, J* = 7.4 Hz, 3 H).

### Step 2

A formate of compound **17** was obtained referring to step 3 of Example 1. ¹H NMR (400MHz, CD₃OD) δ = 8.67 (d, *J* = 7.8 Hz, 1 H), 8.48 (s, 1 H), 8.37 (dd, *J* = 7.8, 1.2 Hz, 1 H), 7.71 (t, *J* = 7.8 Hz, 1 H), 7.65 (d, *J* = 7.8 Hz, 1 H), 7.49 (d, *J* = 7.8 Hz, 1 H), 5.98 (s, 2 H), 3.73-3.89 (m, 1 H), 3.46-3.64 (m, 2 H), 3.32-3.41 (m, 2 H), 2.36 *(t, J* = 7.3 Hz, 2 H), 2.18-2.30 (m, 1 H), 1.84-1.98 (m, 1 H), 1.64 (sxt, *J* = 7.4 Hz, 2 H), 0.93 *(t, J* = 7.4 Hz, 3 H). MS-ESI calculated value [M+H]⁺ 394, actually measured value 394.

The formate of compound **17** can be adjusted the pH of the reaction solution to 8-9 by adding a saturated aqueous sodium carbonate solution to the system, and then, after the extraction with ethyl acetate, the reaction solution was concentrated to obtain compound **17.**

### Example 12: Compound 18

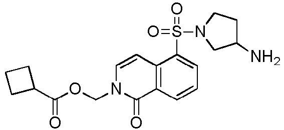

### Synthesis route:

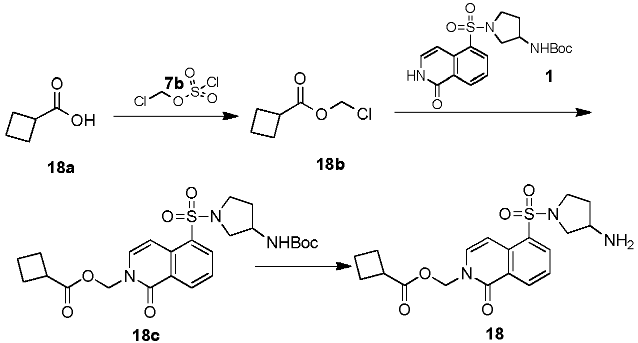

### Step 1

Compound **18b** was obtained referring to step 1 of Example 1.

### Step 2

Compound **18c** was obtained referring to step 2 of Example **1.** ¹H NMR (400MHz, CDCl₃) δ = 8.72 (d, *J* = 7.8 Hz, 1 H), 8.28-8.35 (m, 1 H), 7.59 *(t, J* = 7.8 Hz, 1 H), 7.37-7.48 (m, 2 H), 5.90-6.03 (m, 1 H), 5.96 (s, 1 H), 4.64 (s, 1 H), 4.17 (d, *J* = 9.4 Hz, 1 H), 3.38-3.60 (m, 2 H), 3.15-3.36 (m, 3 H), 2.13-2.37 (m, 5 H), 1.81-2.02 (m, 3 H), 1.43 (s, 9 H).

### Step 3

A formate of compound **18** was obtained referring to step 3 of Example 1. ¹H NMR (400MHz, CD₃OD) δ = 8.67 (d, *J* = 7.8 Hz, 1 H), 8.38 (s, 1 H), 8.37 (dd, *J* = 7.8, 1.2 Hz, 1 H), 7.71 (t, *J* = 7.8 Hz, 1 H), 7.65 (d, *J* = 8.0 Hz, 1 H), 7.49 (d, *J* = 8.0 Hz, 1 H), 5.98 (s, 2 H), 3.74-3.87 (m, 1 H), 3.45-3.64 (m, 2 H), 3.32-3.38 (m, 2 H), 3.14-3.27 (m, 1 H), 2.14-2.36 (m, 5 H), 1.83-2.07 (m, 3 H). MS-ESI calculated value [M+H]⁺406, actually measured value 406.

The formate of compound **18** can be adjusted the pH of the reaction solution to 8-9 by adding a saturated aqueous sodium carbonate solution to the system, and then, after the extraction with ethyl acetate, the reaction solution was concentrated to obtain compound **18.**

### Example 13: Compound 19

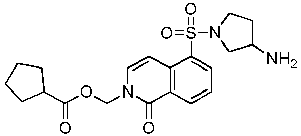

### Synthesis route:

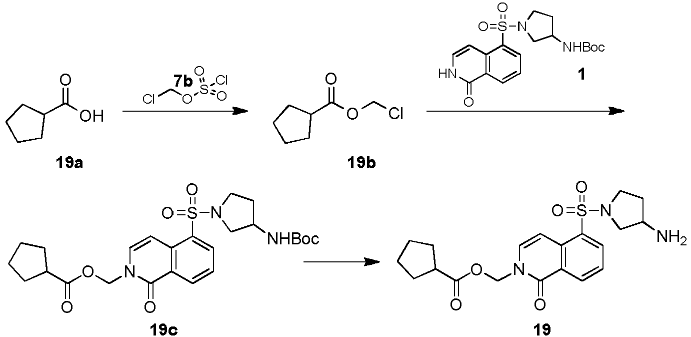

### Step 1

Compound **19b** was obtained referring to step 1 of Example 1.

### Step 2

Compound **19c** was obtained referring to step 2 of Example 1. MS-ESI calculated value [M+Na]⁺542, actually measured value 542.

### Step 3

A formate of compound **19** was obtained referring to step 3 of Example 1. ¹H NMR (400MHz, CD₃OD) δ = 8.68 (d, *J* = 7.6 Hz, 1 H), 8.37 (dd, *J* = 7.6, 1.4 Hz, 2 H), 7.72 (t, *J* = 8.0 Hz, 1 H), 7.65 (d, *J* = 7.8 Hz, 1 H), 7.49 (d, *J* = 8.4 Hz, 1 H), 5.97 (s, 2 H), 4.62 (s, 1 H), 3.78-3.87 (m, 1 H), 3.55-3.65 (m, 1 H), 3.51 (dd, *J* = 10.8, 6.4 Hz, 1H), 3.38 (dd, *J* = 10.8, 4.0 Hz, 1 H), 2.76-2.86 (m, 1 H), 2.19-2.35 (m, 1 H), 1.84-2.01 (m, 3 H), 1.73-1.83 (m, 2 H), 1.53-1.72 (m, 4 H). MS-ESI calculated value [M+H]⁺420, actually measured value 420.

The formate of compound **19** can be adjusted the pH of the reaction solution to 8-9 by adding a saturated aqueous sodium carbonate solution to the system, and then, after the extraction with ethyl acetate, the reaction solution was concentrated to obtain compound **19.**

### Example 14: Compound 20

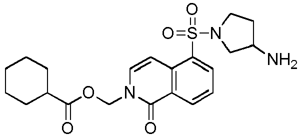

### Synthesis route:

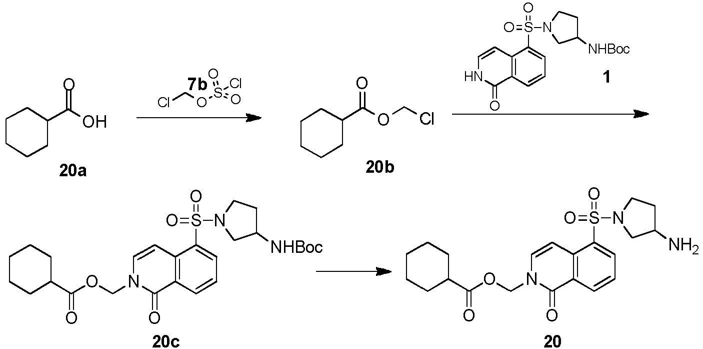

### Step 1

Compound **20b** was obtained referring to step 1 of Example 1.

### Step 2

Compound **20c** was obtained referring to step 2 of Example 1.

### Step 3

A formate of compound **20** was obtained referring to step 3 of Example **1.** ¹H NMR (400MHz, CD₃OD) δ = 8.69 (d, *J* = 7.8 Hz, 1 H), 8.40 (s, 1 H), 8.39 (dd, *J* = 7.8, 1.2 Hz, 1 H), 7.73 (t, *J* = 7.8 Hz, 1 H), 7.66 (d, *J* = 8.0 Hz, 1 H), 7.51 (d, *J* = 8.0 Hz, 1 H), 5.99 (s, 2 H), 4.58 (s, 2 H), 3.71-3.83 (m, 1 H), 3.46-3.65 (m, 2 H), 2.40 (tt, *J* = 11.0, 3.6 Hz, 1 H), 2.21-2.33 (m, 1 H), 1.85-2.00 (m, 3 H), 1.71-1.82 (m, 2 H), 1.65 (d, *J* = 8.8 Hz, 1 H), 1.22-1.50 (m, 5 H). MS-ESI calculated value [M+H]⁺434, actually measured value 434.

The formate of compound **20** can be adjusted the pH of the reaction solution to 8-9 by adding a saturated aqueous sodium carbonate solution to the system, and then, after the extraction with ethyl acetate, the reaction solution was concentrated to obtain compound **20.**

### Example 15: Compound 21

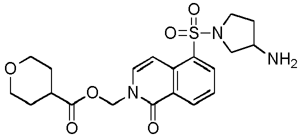

### Synthesis route:

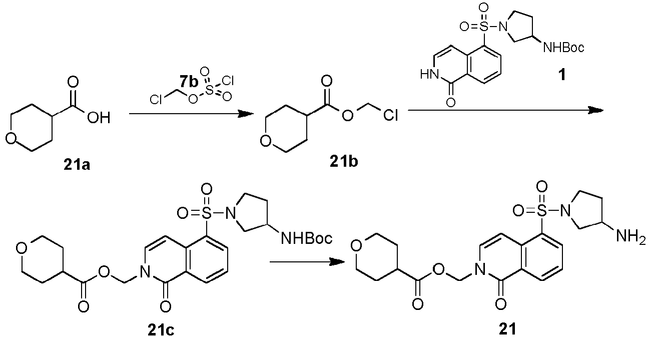

### Step 1

Compound **21b** was obtained referring to step 1 of Example **1.**

### Step 2

Compound **21c** was obtained referring to step 2 of Example **1.** ¹H NMR (400 MHz, CDCl₃) δ = 8.70 (dd, *J* = 8.0, 1.1 Hz, 1 H), 8.26-8.35 (m, 1 H), 7.59 *(t, J* = 7.8 Hz, 1 H), 7.34-7.45 (m, 2 H), 5.97 (s, 2 H), 4.59-4.83 (m, 1 H), 4.06-4.29 (m, 1 H), 3.94 (dt, *J* = 11.4, 3.5 Hz, 2 H), 3.48-3.54 (m, 1 H), 3.34-3.42 (m, 2 H), 3.16-3.33 (m, 2 H), 2.56-2.69 (m, 1 H), 2.08-2.20 (m, 1 H), 1.74-1.84 (m, 6 H), 1.34-1.43 (m, 9 H).

### Step 3

A formate of compound **21** was obtained referring to step 3 of Example **1.** ¹H NMR (400MHz, CD₃OD) δ = 8.67 (d, *J* = 8.2 Hz, 1 H), 8.45 (s, 1 H), 8.37 (d, *J* = 7.8 Hz, 1 H), 7.71 (t, *J* = 8.0 Hz, 1 H), 7.65 (d, *J* = 7.8 Hz, 1 H), 7.49 *(d, J* = 8.0 Hz, 1 H), 7.45-7.53 (m, 1 H), 6.00 (s, 2 H), 3.89 (dt, *J* = 11.7, 3.5 Hz, 2 H), 3.72-3.82 (m, 1 H), 3.48-3.63 (m, 2 H), 3.43 (td, *J* = 11.4, 2.4 Hz, 2 H), 3.35 (d, *J* = 3.6 Hz, 1 H), 2.59-2.73 (m, 1 H), 2.18-2.33 (m, 1 H), 1.92 (td, *J* = 13.5, 5.8 Hz, 1 H), 1.61-1.87 (m, 5 H). MS-ESI calculated value [M+H]⁺436, actually measured value 436.

The formate of compound **21** can be adjusted the pH of the reaction solution to 8-9 by adding a saturated aqueous sodium carbonate solution to the system, and then, after the extraction with ethyl acetate, the reaction solution was concentrated to obtain compound **21.**

### Example 16: Compound 22

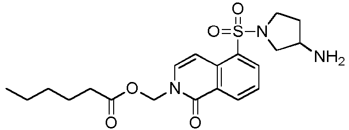

### Synthesis route:

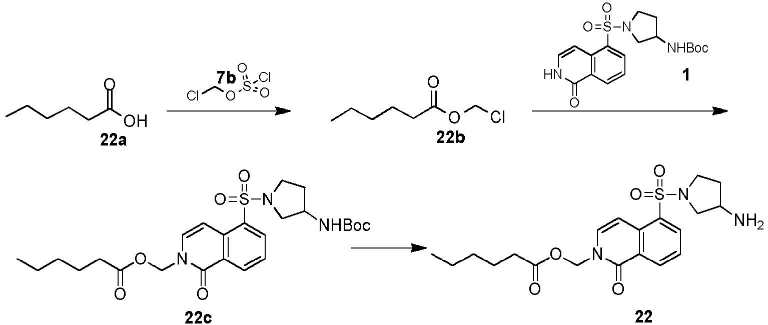

### Step 1

Compound **22b** was obtained referring to step 1 of Example **1.**

### Step 2

Compound **22c** was obtained referring to step 2 of Example **1.** ¹H NMR (400 MHz, CDCl₃) δ = 8.72 (dd, *J* = 8.0, 0.8 Hz, 1 H), 8.32 (dd, *J* = 7.8, 1.4 Hz, 1 H), 7.59 *(t, J* = 7.8 Hz, 1 H), 7.34-7.48 (m, 2 H), 5.96 (s, 2 H), 4.67 (s, 1 H), 4.18 (s, 1 H), 3.36-3.64 (m, 2 H), 3.27 (s, 2 H), 2.37 (t, *J* = 7.6 Hz, 2 H), 2.14 (dq, *J* = 14.4, 6.6 Hz, 1 H), 1.75-1.91 (m, 1 H), 1.65-1.68 (m, 1 H), 1.59-1.62 (m, 1 H), 1.42 (s, 9 H), 1.27-1.33 (m, 4 H), 0.78-0.89 (m, 3 H).

### Step 3

A formate of compound **22** was obtained referring to step 3 of Example **1.** ¹H NMR (400MHz, CD₃OD) δ = 8.67 (d, *J* = 8.0 Hz, 1 H), 8.48 (s, 1 H), 8.37 (d, *J* = 7.4 Hz, 1 H), 7.71 (t, *J* = 7.8 Hz, 1 H), 7.64 (d, *J* = 8.0 Hz, 1 H), 7.49 (d, *J* = 7.8 Hz, 1 H), 5.98 (s, 2 H), 4.57 (s, 1 H), 3.71-3.74 (m, 1 H), 3.46-3.64 (m, 2 H), 2.37 *(t, J* = 7.6 Hz, 2 H), 2.23-2.24 (m, 1 H), 1.88-1.89 (m, 1 H), 1.53-1.71 (m, 2 H), 1.27-1.30 (m, 5 H), 0.85 *(t, J* = 6.8 Hz, 3 H). MS-ESI calculated value [M+H]⁺ 422, actually measured value 422.

The formate of compound **22** can be adjusted the pH of the reaction solution to 8-9 by adding a saturated aqueous sodium carbonate solution to the system, and then, after the extraction with ethyl acetate, the reaction solution was concentrated to obtain compound 22.

### Example 17: Compound 23

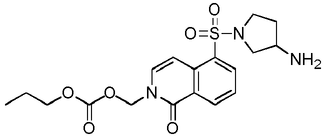

### Synthesis route:

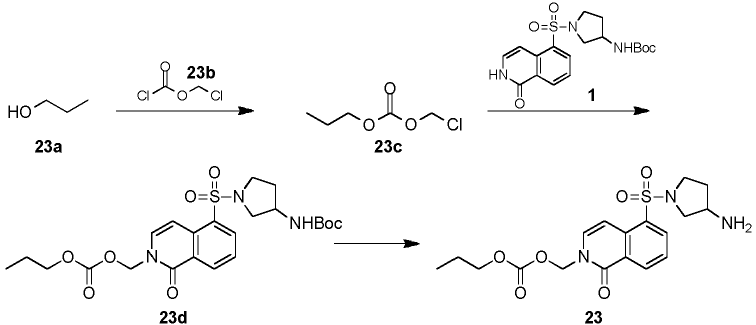

### Step 1

To a solution of compound **23b** (1.0 g, 7.76 mmol) and pyridine (920 mg, 11.6 mmol) in dichloromethane (10 mL) was added compound **23a** (466 mg, 7.76 mmol) dropwise at 0 °C, stirred for 16 hours at 30 °C. Hydrochloric acid (1M, 10mL) was added to the reaction solution, stirred for 5 minutes; the organic phase was separated, washed with water (10mL), dried with anhydrous magnesium sulfate, filtered and concentrated to obtain compound **23c.** ¹H NMR (400 MHz, CDCl₃) δ = 5.73 (s, 2 H), 4.19 *(t, J* = 6.6 Hz, 2 H), 1.73 (sxt, *J* = 7.2 Hz, 2 H), 0.97 (t, *J* = 7.2Hz, 3 H).

### Step 2

Compound **23d** was obtained referring to step 2 of Example **1.** MS-ESI calculated value [M+Na]⁺532, actually measured value 532.

### Step 3

A formate of compound **23** was obtained referring to step 3 of Example **1.** ¹H NMR (400MHz, CD₃OD) δ = 8.67 (d, *J* = 8.0 Hz, 1 H), 8.38 (s, 1 H), 8.37 (d, *J* = 7.8 Hz, 1 H), 7.71 (t, *J* = 7.8 Hz, 1 H), 7.65 (d, *J* = 8.0 Hz, 1 H), 7.50 (d, *J* = 8.0 Hz, 1 H), 5.99 (s, 2 H), 4.58 (s, 1 H), 4.13 *(t, J* = 6.6 Hz, 2 H), 3.76-3.88 (m, 1 H), 3.46-3.64 (m, 2 H), 3.34-3.40 (m, 1 H), 2.20-2.34 (m, 1 H), 1.94 (td, *J* = 13.6, 5.8 Hz, 1 H), 1.68 (sxt, *J* = 7.2 Hz, 2 H), 0.94 *(t, J* = 7.6 Hz, 3 H). MS-ESI calculated value [M+H]⁺ 410, actually measured value 410.

The formate of compound **23** can be adjusted the pH of the reaction solution to 8-9 by adding a saturated aqueous sodium carbonate solution to the system, and then, after the extraction with ethyl acetate, the reaction solution was concentrated to obtain compound **23.**

### Example 18: Compound 24

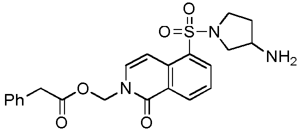

### Synthesis route:

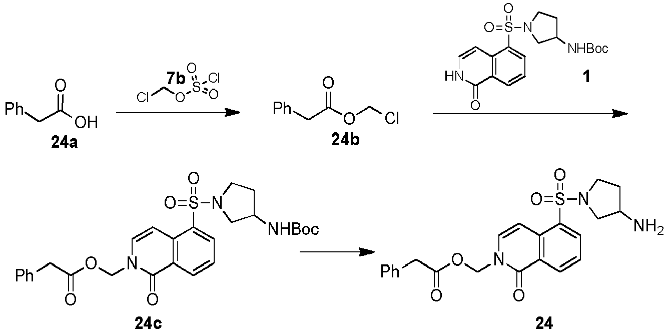

### Step 1

Compound **24b** was obtained referring to step 1 of Example **1.**

### Step 2

Compound **24c** was obtained referring to step 2 of Example **1.** ¹H NMR (400 MHz, CDCl₃) δ ppm 8.72 (d, *J* = 7.2 Hz, 1 H), 8.32 (dd, *J* = 7.8, 1.4 Hz, 1 H), 7.60 (t, *J* = 7.8 Hz, 1 H), 7.33-7.43 (m, 3 H), 7.27-7.32 (m, 4 H), 5.97 (s, 2 H), 4.53-4.75 (m, 1 H), 4.18 (s, 1 H), 3.70 (s, 2 H), 3.37-3.60 (m, 2 H), 3.25 (s, 2 H), 2.08-2.24 (m, 1 H), 1.77-1.91 (m, 1 H), 1.43 (s, 9 H).

### Step 3

A formate of compound **24** was obtained referring to step 3 of Example 1. ¹H NMR (400MHz, CD₃OD) δ = 8.66 (d, *J* = 7.8 Hz, 1 H), 8.45 (s, 1 H), 8.37 (dd, *J* = 7.8, 1.2 Hz, 1 H), 7.71 (t, *J* = 8.0 Hz, 1 H), 7.60 (d, *J* = 8.0 Hz, 1 H), 7.46 *(d, J* = 7.8 Hz, 1 H), 7.16-7.32 (m, 4 H), 6.00 (s, 2 H), 3.74-3.85 (m, 1 H), 3.70 (s, 2 H), 3.46-3.61 (m, 2 H), 3.33-3.38 (m, 1 H), 3.25-3.30 (m, 1 H), 2.18-2.33 (m, 1 H), 1.83-2.02 (m, 1 H). MS-ESI calculated value [M+Na]⁺464, actually measured value 464.

The formate of compound **24** can be adjusted the pH of the reaction solution to 8-9 by adding a saturated aqueous sodium carbonate solution to the system, and then, after the extraction with ethyl acetate, the reaction solution was concentrated to obtain compound **24.**

### Example 19: Compound 25

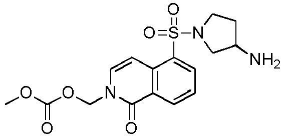

### Synthesis route:

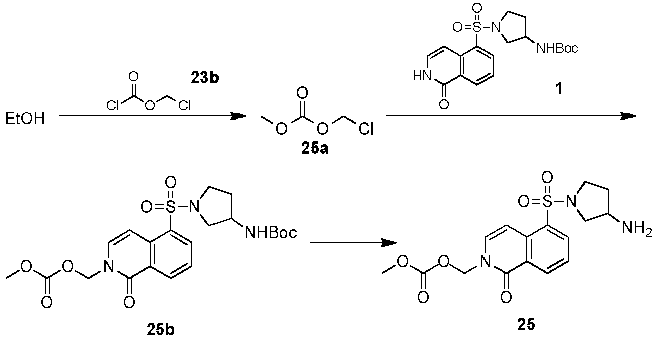

### Step 1

Compound **25a** was obtained referring to step 1 of Example **17.**

### Step 2

Compound **25b** was obtained referring to step 2 of Example 1. MS-ESI calculated value [M+Na]⁺504, actually measured value 504.

### Step 3

A formate of compound **25** was obtained referring to step 3 of Example **1.** ¹H NMR (400MHz, CD₃OD) δ = 8.68 (d, *J* = 7.8 Hz, 1 H), 8.38 (dd, *J* = 7.8, 1.2 Hz, 1 H), 7.72 (t, *J* = 8.0 Hz, 1 H), 7.65 (d, *J* = 7.8 Hz, 1 H), 7.50 *(d, J* = 8.0 Hz, 1 H), 5.99 (s, 2 H), 3.83-3.90 (m, 1 H), 3.80 (s, 3 H), 3.59-3.68 (m, 1 H), 3.44-3.59 (m, 2 H), 3.37-3.42 (m, 1 H), 2.23-2.37 (m, 1 H), 1.97 (td, *J* = 13.6, 6.0 Hz, 1 H). MS-ESI calculated value [M+H]⁺382, actually measured value 382.

The formate of compound **25** can be adjusted the pH of the reaction solution to 8-9 by adding a saturated aqueous sodium carbonate solution to the system, and then, after the extraction with ethyl acetate, the reaction solution was concentrated to obtain compound **25.**

### Example 20: Compound 26

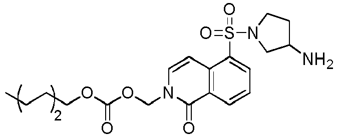

### Synthesis route:

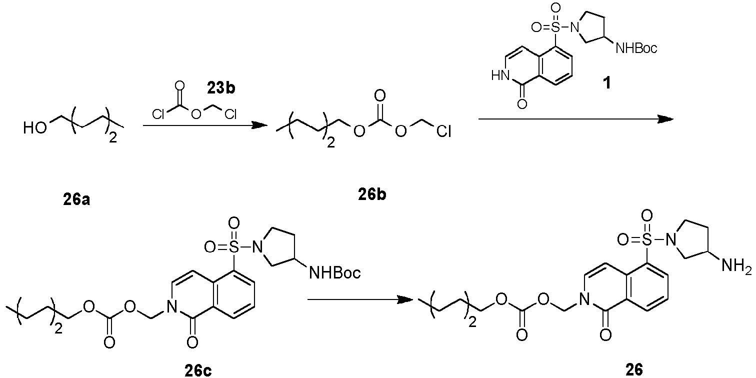

### Step 1

Compound **26b** was obtained referring to step 1 of Example **17.**

### Step 2

Compound **26c** was obtained referring to step 2 of Example **1.** ¹H NMR (400 MHz, CDCl₃) δ = 8.72 (d, *J* = 8.0 Hz, 1 H), 8.32 (d, *J* = 7.8 Hz, 1 H), 7.59 (t, *J* = 8.0 Hz, 1 H), 7.43 (s, 2 H), 5.98 (s, 2 H), 4.65 (s, 1 H), 4.18 (t, *J* = 6.8 Hz, 2 H), 3.46 (d, *J* = 18.0 Hz, 2 H), 3.28 (s, 2 H), 2.14 (dq, *J* = 13.6, 7.2 Hz, 1 H), 1.84 (s, 1 H), 1.64-1.72 (m, 2 H), 1.43 (s, 9 H), 1.27-1.37 (m, 6 H), 0.88 *(t, J* = 6.8 Hz, 3 H).

### Step 3

A formate of compound **26** was obtained referring to step 3 of Example **1.** ¹H NMR (400MHz, CD₃OD) δ = 8.67 (d, *J* = 8.0 Hz, 1 H), 8.45 (s, 1 H), 8.37 (dd, J = 7.8, 1.2 Hz, 1 H), 7.71 (t, J = 8.0 Hz, 1 H), 7.65 (d, J = 7.8 Hz, 1 H), 7.50 (d, *J* = 8.0 Hz, 1 H), 5.99 (s, 2 H), 4.17 *(t, J* = 6.6 Hz, 2 H), 3.76-3.85 (m, 1 H), 3.48-3.63 (m, 2 H), 3.32-3.40 (m, 2 H), 2.22-2.39 (m, 1 H), 1.89-1.99 (m, 1 H), 1.60-1.72 (m, 2 H), 1.30-1.40 (m, 6 H), 0.83-0.94 (m, 3 H). MS-ESI calculated value [M+H]⁺ 452, actually measured value 452.

The formate of compound **26** can be adjusted the pH of the reaction solution to 8-9 by adding a saturated aqueous sodium carbonate solution to the system, and then, after the extraction with ethyl acetate, the reaction solution was concentrated to obtain compound **26.**

### Example 21: Compound 27

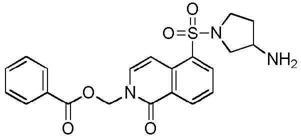

### Synthesis route:

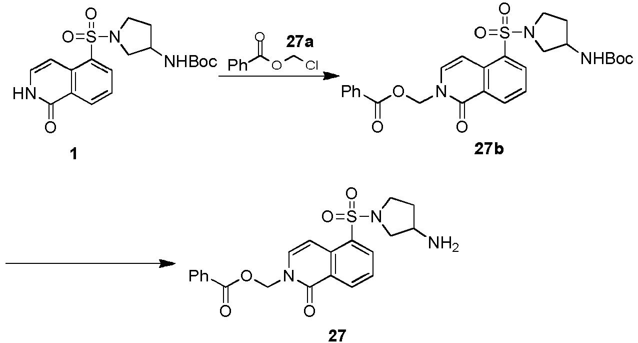

### Step 1

Compound **27b** was obtained referring to step 2 of Example **1.** MS-ESI calculated value [M+Na]⁺550, actually measured value 550.

### Step 2

A formate of compound **27** was obtained referring to step 3 of Example **1.** ¹H NMR (400 MHz, CDCl₃) δ = 8.73 (d, *J* = 8.0 Hz, 1 H), 8.35 (d, *J* = 7.8 Hz, 1 H), 8.09 (d, *J* = 7.4 Hz, 2H), 7.54-7.64 (m, 3 H), 7.45 *(dt, J* = 7.8, 3.8 Hz, 3 H), 6.22 (s, 2 H), 3.62 (s, 1 H), 3.46-3.55 (m, 2 H), 3.31-3.42 (m, 1 H), 3.10 (dd, *J* = 9.9, 4.1 Hz, 1 H), 2.03-2.22 (m, 2 H). MS-ESI calculated value [M+H]⁺428, actually measured value 428.

The formate of compound **27** can be adjusted the pH of the reaction solution to 8-9 by adding a saturated aqueous sodium carbonate solution to the system, and then, after the extraction with ethyl acetate, the reaction solution was concentrated to obtain compound **27.**

### Example 22: Compound 28

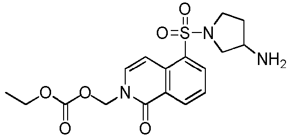

### Synthesis route:

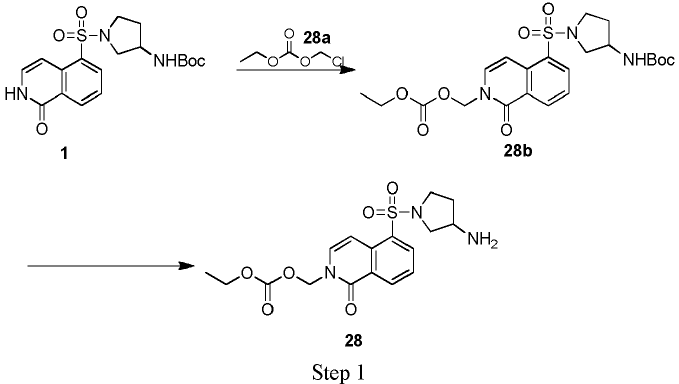

Compound **28b** was obtained referring to step 2 of Example 1. MS-ESI calculated value [M+Na]⁺518, actually measured value 518.

### Step 2

A formate of compound **28** was obtained referring to step 3 of Example 1. ¹H NMR (400 MHz, deuterated acetone) δ ppm 8.63 *(d, J* = 7.4 Hz, 1 H), 8.43 (dd, *J* = 7.8, 1.4 Hz, 1 H), 8.12 (s, 1 H), 7.64-7.74 (m, 2 H), 7.49 (d, *J* = 8.0 Hz, 1 H), 6.03 (d, *J* = 1.0 Hz, 2 H), 4.22 (q, *J* = 7.0 Hz, 2 H), 4.16 (t, *J* = 4.6 Hz, 1 H), 3.59 (dd, *J* = 9.8, 5.8 Hz, 1 H), 3.45-3.52 (m, 2 H), 3.22 (dd, *J* = 9.8, 3.9 Hz, 1 H), 2.11-2.16 (m, 1 H), 1.76 (td, *J* = 12.4, 5.2 Hz, 1 H), 1.27 *(t, J* = 7.0 Hz, 3 H). MS-ESI calculated value [M+H]⁺396, actually measured value 396.

The formate of compound **28** can be adjusted the pH of the reaction solution to 8-9 by adding a saturated aqueous sodium carbonate solution to the system, and then, after the extraction with ethyl acetate, the reaction solution was concentrated to obtain compound **28.**

### Example 23: Compound 29

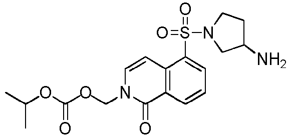

### Synthesis route:

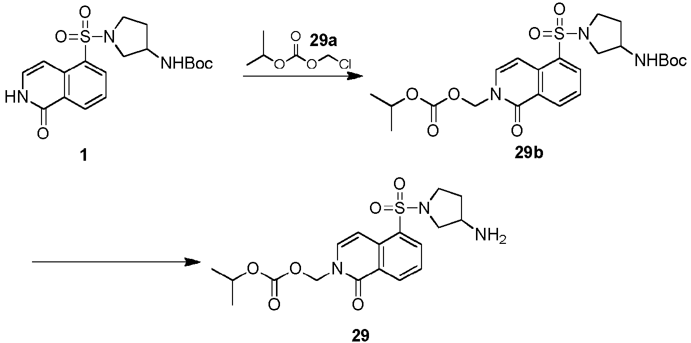

### Step 1

Compound **29b** was obtained referring to step 2 of Example 1. MS-ESI calculated value [M+Na]⁺532, actually measured value 532.

### Step 2

A formate of compound **29** was obtained referring to step 3 of Example 1. ¹H NMR (400MHz, CD₃OD) δ = 8.69 (d, *J* = 7.8 Hz, 1 H), 8.49 (s, 1 H), 8.39 (dd, *J* = 7.8, 1.3 Hz, 1 H), 7.73 (t, *J* = 7.8 Hz, 1 H), 7.67 (d, *J* = 7.8 Hz, 1 H), 7.51 (d, *J* = 7.8 Hz, 1 H), 5.99 (s, 2 H), 3.76-3.88 (m, 1 H), 3.49-3.66 (m, 2 H), 3.28-3.40 (m, 3 H), 2.22-2.38 (m, 1 H), 1.87-2.01 (m, 1 H), 1.30 (d, *J* = 6.4 Hz, 7 H). MS-ESI calculated value [M+H]⁺410, actually measured value 410.

The formate of compound **29** can be adjusted the pH of the reaction solution to 8-9 by adding a saturated aqueous sodium carbonate solution to the system, and then, after the extraction with ethyl acetate, the reaction solution was concentrated to obtain compound **29.**

### Example 24: Compound 30

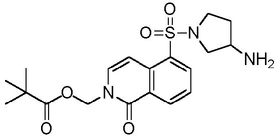

### Synthesis route:

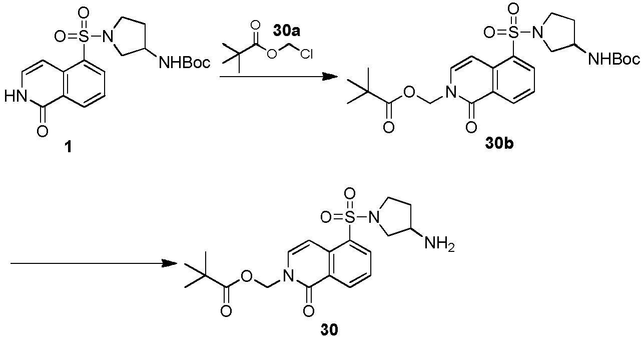

### Step 1

Compound **30b** was obtained referring to step 2 of Example **1.** MS-ESI calculated value [M+Na]⁺530, actually measured value 530.

### Step 2

A formate of compound **30** was obtained referring to step 3 of Example **1.** ¹H NMR (400 MHz, CDCl₃) δ ppm 8.63 (d, *J* = 7.8 Hz, 1 H), 8.25 (d, *J* = 7.6 Hz, 1 H), 8.21 (s, 1 H),7.51 (t, *J* = 7.8 Hz, 1 H), 7.34-7.40 (m, 1 H), 7.29-7.34 (m, 1 H), 5.87 (s, 2 H), 3.69 (s, 1 H), 3.50 (s, 1 H), 3.42 (s, 1 H), 3.26 (s, 2 H), 2.10 (s, 1 H), 1.85 (s, 1 H), 1.13 (s, 9 H). MS-ESI calculated value [M+H]⁺408, actually measured value 408.

The formate of compound **30** can be adjusted the pH of the reaction solution to 8-9 by adding a saturated aqueous sodium carbonate solution to the system, and then, after the extraction with ethyl acetate, the reaction solution was concentrated to obtain compound **30.**

### Example 25: Compound 31

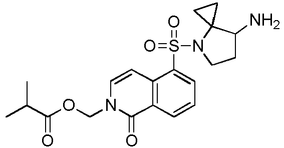

### Synthesis route:

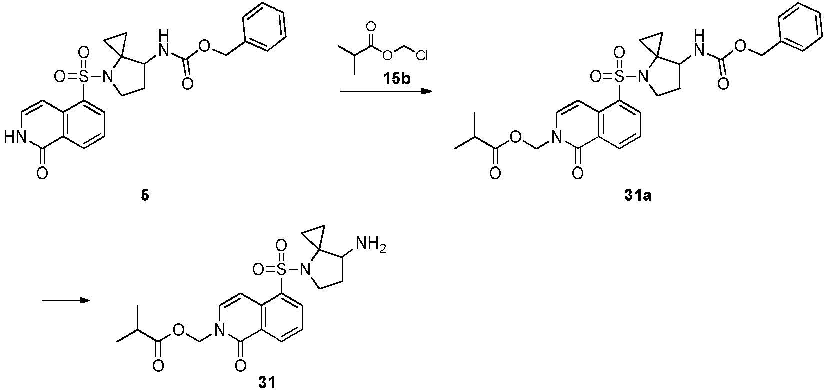

### Step 1

Compound **31a** was obtained referring to step 2 of Example 1. MS-ESI calculated value [M+H]⁺ 554, actually measured value 554.

### Step 2

Trifluoroacetic acid (1.0 mL) was added to a microwave tube and compound **31a** (79 mg, 143 µmol) was added to the microwave tube, microwaving for 1 hour at 60 °C. The solvent was removed by concentration under reduced pressure, and the crude product was purified by high performance liquid chromatography (acid, formic acid system) to obtain a formate of compound **31.** ¹H NMR (400 MHz, CD₃OD) δ = 8.66 (d, *J =* 8.1 Hz, 1H), 8.50 (s, 1H), 8.45-8.39 (m, 1H), 7.73-7.63 (m, 2H), 7.17 (d, *J =* 7.9 Hz, 1H), 5.97 (s, 2H), 4.12-4.01 (m, 1H), 3.66-3.55 (m, 1H), 3.41 (br d, *J =* 4.5 Hz, 1H), 2.65-2.57 (m, 1H), 2.56-2.46 (m, 1H), 2.13-2.02 (m, 1H), 1.23-1.17 (m, 1H), 1.15 (d, *J=* 7.0 Hz, 7H), 0.80 (t, *J =* 8.9 Hz, 2H). MS-ESI calculated value [M+H]⁺ 420, actually measured value 420.

The formate of compound **31** can be adjusted the pH of the reaction solution to 8-9 by adding a saturated aqueous sodium carbonate solution to the system, and then, after the extraction with ethyl acetate, the reaction solution was concentrated to obtain compound **31.**

### Example 26: Compound 32

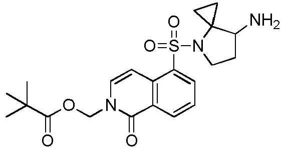

### Synthesis route:

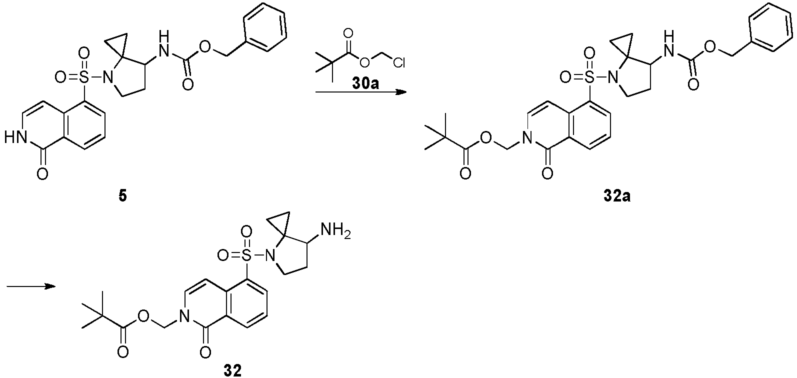

### Step 1

Compound **32a** was obtained referring to step 2 of Example 1. MS-ESI calculated value [M+Na]⁺590, actually measured value 590.

### Step 2

A formate of compound 32 was obtained referring to step 2 of Example **25.** ¹H NMR (400 MHz, CD₃OD) δ = 8.67 (d, J = 7.6 Hz, 1H), 8.47 (s, 1H), 8.45-8.41 (m, 1H), 7.74-7.61 (m, 2H), 7.19 (d, J = 7.8 Hz, 1H), 5.97 (s, 2H), 4.12-3.99 (m, 1H), 3.68-3.54 (m, 1H), 3.36-3.33 (m, 1H), 2.59-2.42 (m, 1H), 2.08-1.94 (m, 1H), 1.19 (s, 9H), 1.18-1.06 (m, 2H), 0.88-0.74 (m, 2H). MS-ESI calculated value [M+H]+ 434, actually measured value 434.

The formate of compound **32** can be adjusted the pH of the reaction solution to 8-9 by adding a saturated aqueous sodium carbonate solution to the system, and then, after the extraction with ethyl acetate, the reaction solution was concentrated to obtain compound **32.**

### Example 27: Compound 33

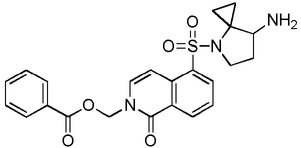

### Synthesis route:

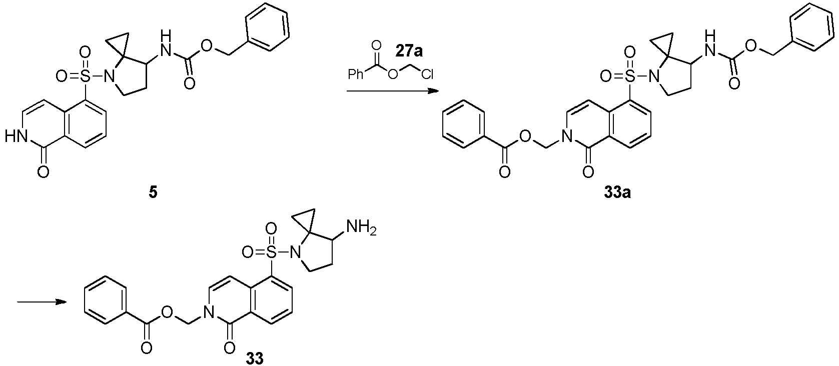

### Step 1

Compound **33a** was obtained referring to step 2 of Example 1. MS-ESI calculated value [M+H]⁺588, actually measured value 588.

### Step 2

A formate of compound 33 was obtained referring to step 2 of Example **25.** ¹H NMR (400 MHz, CD₃OD) δ = 8.66 (d, *J=* 7.9 Hz, 1H), 8.48 (s, 1H), 8.43-8.37 (m, 1H), 8.05-7.99 (m, 2H), 7.78 (d, *J =* 7.9 Hz, 1H), 7.68 (t, *J =* 7.9 Hz, 1H), 7.64-7.57 (m, 1H), 7.50-7.43 (m, 2H), 7.19 (d, *J* = 7.8 Hz, 1H), 6.22 (s, 2H), 4.12-4.00 (m, 1H), 3.65-3.55 (m, 1H), 3.47-3.39 (m, 1H), 2.59-2.44 (m, 1H), 2.15-2.03 (m, 1H), 1.26-1.10 (m, 2H), 0.83-0.74 (m, 2H). MS-ESI calculated value [M+H]⁺ 454, actually measured value 454.

The formate of compound **33** can be adjusted the pH of the reaction solution to 8-9 by adding a saturated aqueous sodium carbonate solution to the system, and then, after the extraction with ethyl acetate, the reaction solution was concentrated to obtain compound **33.**

### Example 28: Compound 34

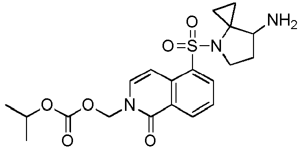

### Synthesis route:

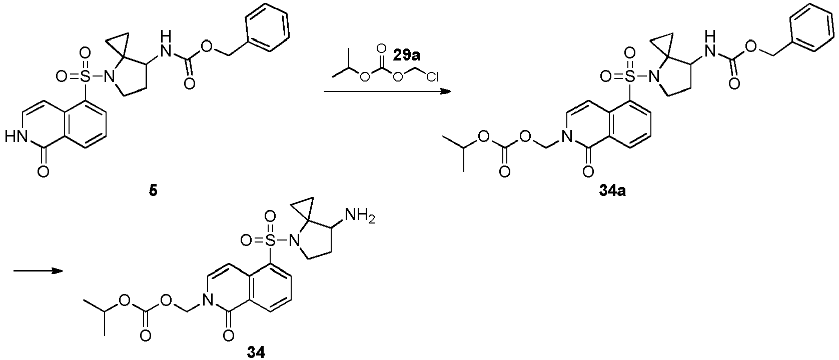

### Step 1

Compound **34a** was obtained referring to step 2 of Example 1. MS-ESI calculated value [M+Na]⁺592, actually measured value 592.

### Step 2

A formate of compound **34** was obtained referring to step 2 of Example **25.** ¹H NMR (400 MHz, CD₃OD) δ = 8.66 (d, *J=* 8.1 Hz, 1H), 8.50 (br s, 1H), 8.46-8.40 (m, 1H), 7.72-7.63 (m, 2H), 7.23 (d, *J =* 7.8 Hz, 1H), 5.97 (s, 2H), 4.92-4.90 (m, 1H), 4.02-3.92 (m, 1H), 3.66-3.55 (m, 1H), 3.29-3.20 (m, 1H), 2.51-2.36 (m, 1H), 2.00-1.88 (m, 1H), 1.28 (d, *J* = 6.4 Hz, 6H), 1.18-1.02 (m, 2H), 0.95-0.87 (m, 1H), 0.79-0.72 (m, 1H). MS-ESI calculated value [M+H]⁺ 436, actually measured value 436.

The formate of compound **34** can be adjusted the pH of the reaction solution to 8-9 by adding a saturated aqueous sodium carbonate solution to the system, and then, after the extraction with ethyl acetate, the reaction solution was concentrated to obtain compound **34.**

### Example 29: Compound 35

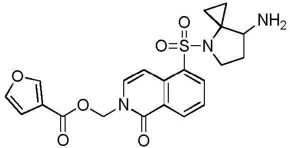

### Synthesis route:

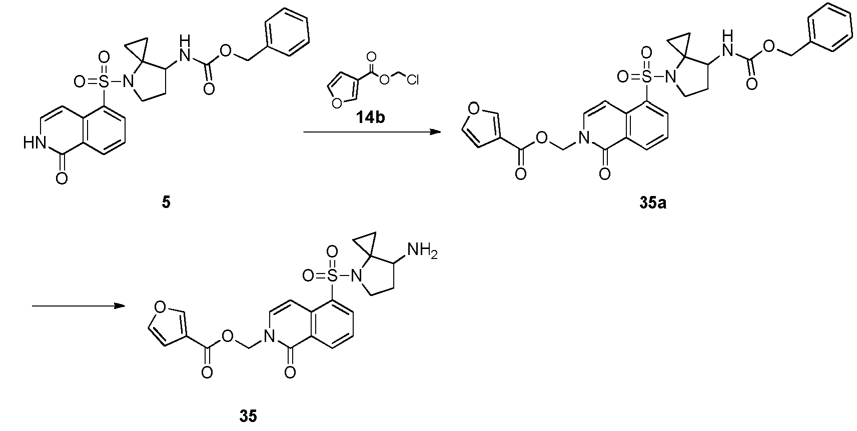

### Step 1

Compound **35a** was obtained referring to step 2 of Example **1.** MS-ESI calculated value [M+Na]⁺ 600, actually measured value 600.

### Step 2

A formate of compound **35** was obtained referring to step 2 of Example **25.** ¹H NMR (400MHz, CD₃OD) δ = 8.68 (d, *J=* 7.9 Hz, 1H), 8.46 (br s, 1H), 8.44-8.41 (m, 1H), 8.23 (s, 1H), 7.78-7.67 (m, 2H), 7.60 (t, *J* = 1.7 Hz, 1H), 7.20 (d, *J* = 7.8 Hz, 1H), 6.79 (d, *J* = 1.2 Hz, 1H), 6.15 (s, 2H), 4.12-4.02 (m, 1H), 3.65-3.55 (m, 1H), 3.43-3.35 (m, 1H), 2.59-2.45 (m, 1H), 2.11-2.00 (m, 1H), 1.26-1.08 (m, 2H), 0.87-0.74 (m, 2H). MS-ESI calculated value [M+H]⁺ 444, actually measured value 444.

The formate of compound **35** can be adjusted the pH of the reaction solution to 8-9 by adding a saturated aqueous sodium carbonate solution to the system, and then, after the extraction with ethyl acetate, the reaction solution was concentrated to obtain compound **35.**

### Example 30: Compound 36

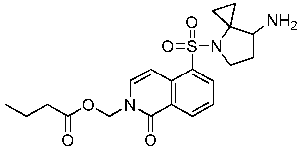

### Synthesis route:

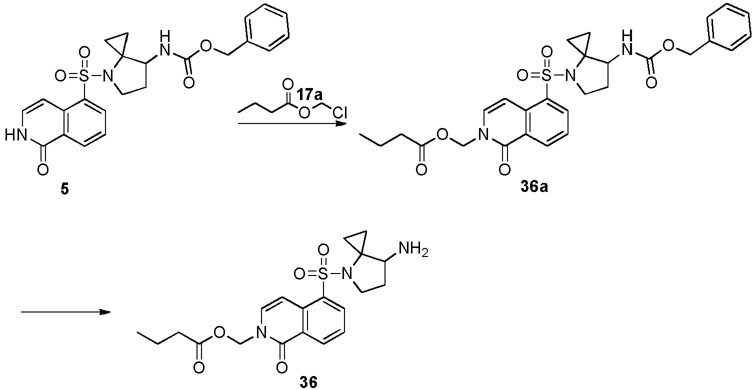

### Step 1

Compound **36a** was obtained referring to step 2 of Example 1. MS-ESI calculated value [M+H]⁺ 554, actually measured value 554.

### Step 2

A formate of compound 36 was obtained referring to step 2 of Example 25. ¹H NMR (400MHz, CD₃OD) δ = 8.67 (d, *J=* 7.8 Hz, 1H), 8.53 (br s, 1H), 8.49-8.43 (m, 1H), 7.74-7.63 (m, 2H), 7.25 (d, *J =* 7.9 Hz, 1H), 5.99 (s, 2H), 4.02-3.89 (m, 1H), 3.68-3.57 (m, 1H), 3.27-3.19 (m, 1H), 2.48-2.40 (m, 1H), 2.38 (t, *J* = 7.3 Hz, 2H), 1.99-1.88 (m, 1H), 1.72-1.60 (m, 2H), 1.17-1.02 (m, 2H), 0.99-0.91 (m, 4H), 0.80-0.72 (m, 1H). MS-ESI calculated value [M+H]⁺ 420, actually measured value 420.

The formate of compound **36** can be adjusted the pH of the reaction solution to 8-9 by adding saturated aqueous sodium carbonate solution to the system, and then, after the extraction with ethyl acetate, the reaction solution was concentrated to obtain compound **36.**

### Example 31: Compound 37

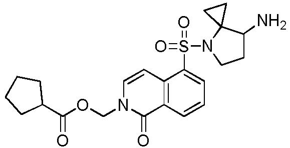

### Synthesis route:

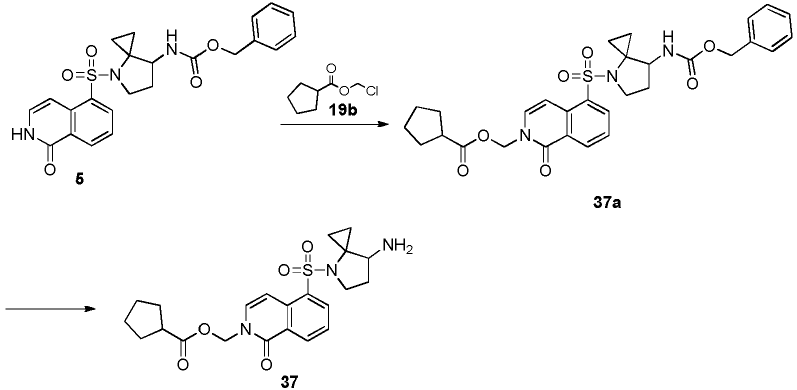

### Step 1

Compound **37a** was obtained referring to step 2 of Example 1. MS-ESI calculated value [M+H]⁺ 580, actually measured value 580.

### Step 2

A formate of compound **37** was obtained referring to step 2 of Example **25.** ¹H NMR (400MHz, CD₃OD) δ = 8.66 (d, *J=* 8.0 Hz, 1H), 8.46 (br s, 1H), 8.43-8.40 (m, 1H), 7.74-7.62 (m, 2H), 7.16 (d, *J =* 7.8 Hz, 1H), 5.97 (s, 2H), 4.14-4.04 (m, 1H), 3.64-3.55 (m, 1H), 3.48-3.41 (m, 1H), 2.86-2.76 (m, 1H), 2.61-2.47 (m, 1H), 2.16-2.05 (m, 1H), 1.94-1.84 (m, 2H), 1.83-1.73 (m, 2H), 1.72-1.53 (m, 4H), 1.25-1.11 (m, 2H), 0.85-0.73 (m, 2H). MS-ESI calculated value [M+H]⁺ 446, actually measured value 446.

The formate of compound **37** can be adjusted the pH of the reaction solution to 8-9 by adding a saturated aqueous sodium carbonate solution to the system, and then, after the extraction with ethyl acetate, the reaction solution was concentrated to obtain compound **37.**

### Example 32: Compound 38

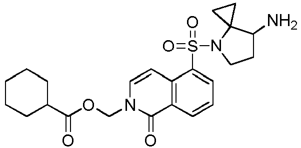

### Synthesis route:

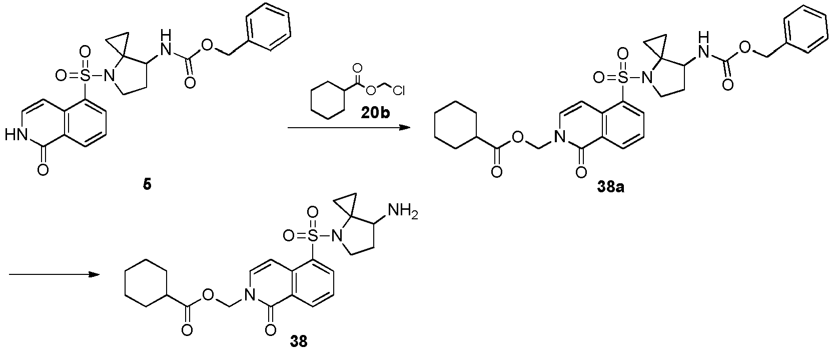

### Step 1

Compound **38a** was obtained referring to step 2 of Example **1.** MS-ESI calculated value [M+H]⁺ 594, actually measured value 594.

### Step 2

A formate of compound **38** was obtained referring to step 3 of Example **25.** ¹H NMR (400MHz, CD₃OD) δ = 8.64 (d, *J=* 8.0 Hz, 1H), 8.47-8.40 (m, 1H), 7.74-7.59 (m, 2H), 7.29 (d, *J =* 7.9 Hz, 1H), 5.97 (s, 2H), 3.88-3.77 (m, 1H), 3.67-3.56 (m, 1H), 3.08-3.02 (m, 1H), 2.44-2.26 (m, 2H), 1.88 (d, *J=* 13.4 Hz, 2H), 1.82-1.68 (m, 3H), 1.63 (br d, *J=* 9.5 Hz, 1H), 1.49-1.38 (m, 2H), 1.34-1.27 (m, 3H), 1.09-0.94 (m, 3H), 0.75-0.67 (m, 1H). MS-ESI calculated value [M+H]⁺ 460, actually measured value 460.

The formate of compound **38** can be adjusted the pH of the reaction solution to 8-9 by adding a saturated aqueous sodium carbonate solution to the system, and then, after the extraction with ethyl acetate, the reaction solution was concentrated to obtain compound **38.**

### Example 33: Compound 39

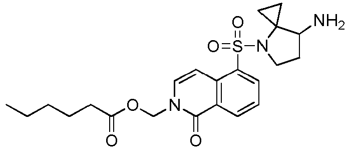

### Synthesis route:

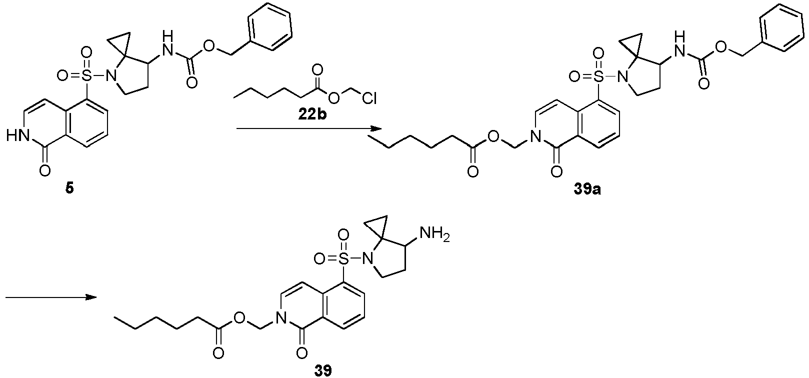

### Step 1

Compound **39a** was obtained referring to step 2 of Example 1. MS-ESI calculated value [M+H]⁺ 582, actually measured value 582.

### Step 2

Compound **39** was obtained referring to step 2 of Example **25.** ¹H NMR (400MHz, CD₃OD) δ = 8.66-8.59 (m, 1H), 8.47-8.39 (m, 1H), 7.72-7.60 (m, 2H), 7.37-7.28 (m, 1H), 5.97 (s, 2H), 3.83-3.57 (m, 2H), 3.01-2.93 (m, 1H), 2.37 (t, *J* = 7.4 Hz, 2H), 2.32-2.21 (m, 1H), 1.80-1.67 (m, 1H), 1.65-1.56 (m, 2H), 1.27 (dd, *J* = 3.6, 7.3 Hz, 4H), 1.16-1.06 (m, 1H), 1.03-0.95 (m, 1H), 0.95-0.89 (m, 1H), 0.84 (t, *J* = 7.0 Hz, 3H), 0.73-0.63 (m, 1H). MS-ESI calculated value [M+H]⁺ 448, actually measured value 448.

The formate of compound **39** can be adjusted the pH of the reaction solution to 8-9 by adding a saturated aqueous sodium carbonate solution to the system, and then, after the extraction with ethyl acetate, the reaction solution was concentrated to obtain compound **39.**

### Example 34: Compound 40

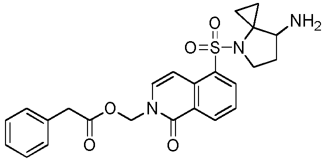

### Synthesis route:

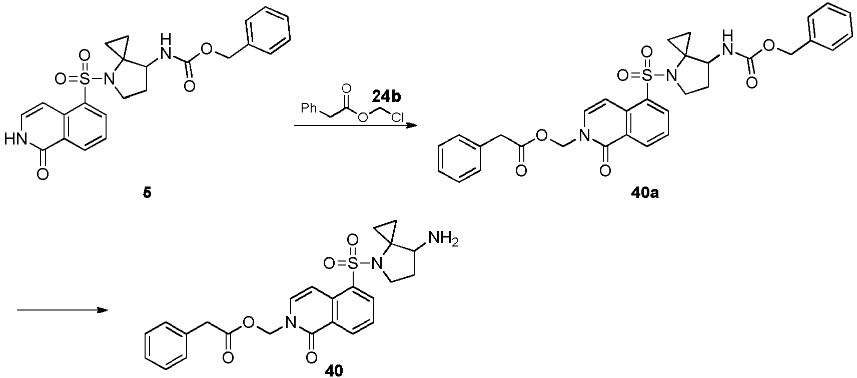

### Step 1

Compound **40a** was obtained referring to step 2 of Example **1.** MS-ESI calculated value [M+H]⁺ 602, actually measured value 602.

### Step 2

A formate of compound **40** was obtained referring to step 2 of Example **25.** ¹H NMR (400MHz, CD₃OD) δ = 8.64 (d, *J* = 7.8 Hz, 1H), 8.48 (br s, 1H), 8.44-8.39 (m, 1H), 7.69 (t, *J* = 7.9 Hz, 1H), 7.60 (d, *J* = 7.9 Hz, 1H), 7.29-7.17 (m, 4H), 7.13 (d, *J* = 7.9 Hz, 1H), 5.99 (s, 2H), 4.12-4.00 (m, 1H), 3.70 (s, 2H), 3.64-3.52 (m, 1H), 3.44-3.36 (m, 1H), 2.57-2.45 (m, 1H), 2.16-1.99 (m, 1H), 1.21-1.09 (m, 2H), 0.83-0.73 (m, 2H). MS-ESI calculated value [M+H]⁺ 468, actually measured value 468.

The formate of compound **40** can be adjusted the pH of the reaction solution to 8-9 by adding a saturated aqueous sodium carbonate solution to the system, and then, after the extraction with ethyl acetate, the reaction solution was concentrated to obtain compound **40.**

### Example 35: Compound 41

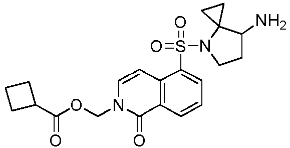

### Synthesis route:

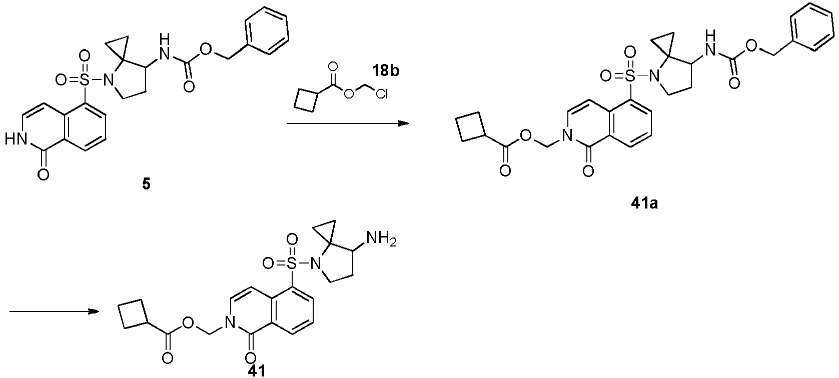

### Step 1

Compound **41a** was obtained referring to step 2 of Example **1.** MS-ESI calculated value [M+H]⁺ 566, actually measured value 566.

### Step 2

A formate of compound **41** was obtained referring to step 2 of Example **25.** ¹H NMR (400MHz, CD₃OD) δ = 8.66 (d, *J=* 7.9 Hz, 1H), 8.50 (br s, 1H), 8.45-8.39 (m, 1H), 7.73-7.63 (m, 2H), 7.18 (d, *J =* 7.9 Hz, 1H), 5.97 (s, 2H), 4.10-3.99 (m, 1H), 3.67-3.55 (m, 1H), 3.39-3.33 (m, 1H), 3.27-3.18 (m, 1H), 2.57-2.44 (m, 1H), 2.33-2.14 (m, 4H), 2.09-1.95 (m, 2H), 1.94-1.81 (m, 1H), 1.24-1.07 (m, 2H), 0.90-0.71 (m, 2H). MS-ESI calculated value [M+H]⁺ 432, actually measured value 432.

The formate of compound **41** can be adjusted the pH of the reaction solution to 8-9 by adding a saturated aqueous sodium carbonate solution to the system, and then, after the extraction with ethyl acetate, the reaction solution was concentrated to obtain compound **41.**

### Example 36: Compound 42

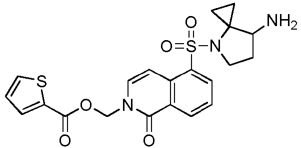

### Synthesis route:

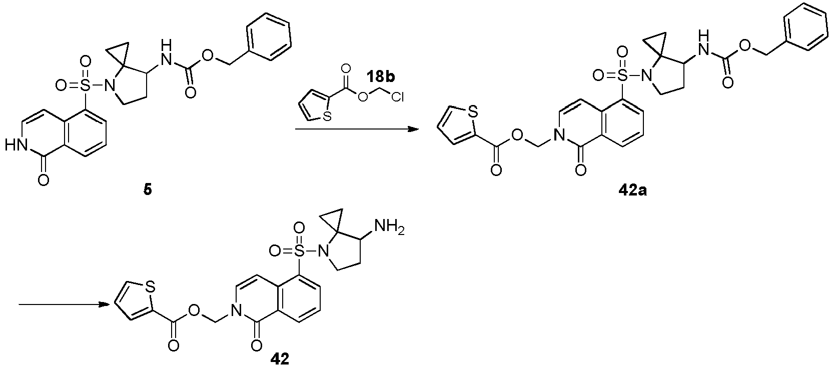

### Step 1

Compound **42a** was obtained referring to step 2 of Example 1. MS-ESI calculated value [M+H]⁺594, actually measured value 594.

### Step 2

A formate of compound **42** was obtained referring to step 2 of Example **25.** ¹H NMR (400MHz, CD₃OD) δ = 8.68 (d, *J=* 7.6 Hz, 1H), 8.46 (s, 1H), 8.44-8.41 (m, 1H), 7.90-7.86 (m, 1H), 7.83-7.79 (m, 1H), 7.76 (d, *J =* 8.1 Hz, 1H), 7.70 (t, *J* = 7.9 Hz, 1H), 7.23-7.14 (m, 2H), 6.18 (s, 2H), 4.11-4.01 (m, 1H), 3.65-3.56 (m, 1H), 3.40-3.36 (m, 1H), 2.59-2.45 (m, 1H), 2.12-1.98 (m, 1H), 1.27-1.06 (m, 2H), 0.88-0.72 (m, 2H). MS-ESI calculated value [M+H]⁺ 460, actually measured value 460.

The formate of compound **42** can be adjusted the pH of the reaction solution to 8-9 by adding a saturated aqueous sodium carbonate solution to the system, and then, after the extraction with ethyl acetate, the reaction solution was concentrated to obtain compound **42.**

### Example 37: Compound 43

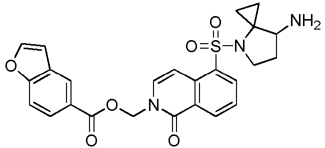

### Synthesis route:

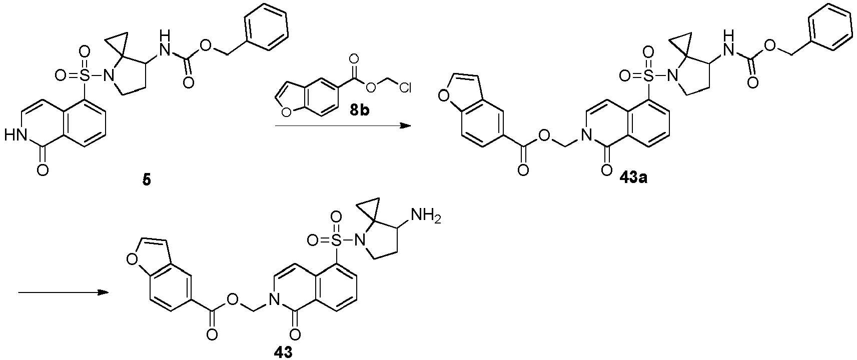

### Step 1

Compound 43a was obtained referring to step 2 of Example 1. MS-ESI calculated value [M+H]⁺ 628, actually measured value 628.

### Step 2

A formate of compound **43** was obtained referring to step 2 of Example **25.** ¹H NMR (400MHz, CD₃OD) δ = 8.69 (d, *J =* 7.9 Hz, 1H), 8.49 (s, 1H), 8.45-8.41 (m, 1H), 8.38 (d, *J =* 1.6 Hz, 1H), 8.05-8.00 (m, 1H), 7.86 (d,*J* = 2.3 Hz, 1H), 7.81 (d, *J =* 7.9 Hz, 1H), 7.70 (t, *J =* 7.9 Hz, 1H), 7.58 (d, *J =* 8.8 Hz, 1H), 7.23 (d, *J =* 7.9 Hz, 1H), 6.95 (d, *J =* 1.5 Hz, 1H), 6.24 (s, 2H), 4.10-3.96 (m, 1H), 3.68-3.54 (m, 1H), 3.36-3.32 (m, 1H), 2.55-2.42 (m, 1H), 2.08-1.94 (m, 1H), 1.26-1.04 (m, 2H), 0.89-0.72 (m, 2H). MS-ESI calculated value [M+H]⁺ 494, actually measured value 494.

The formate of compound **43** can be adjusted the pH of the reaction solution to 8-9 by adding a saturated aqueous sodium carbonate solution to the system, and then, after the extraction with ethyl acetate, the reaction solution was concentrated to obtain compound **43.**

### Example 38: Compound 44

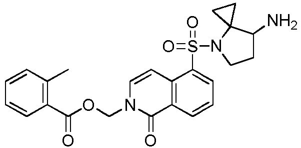

### Synthesis route:

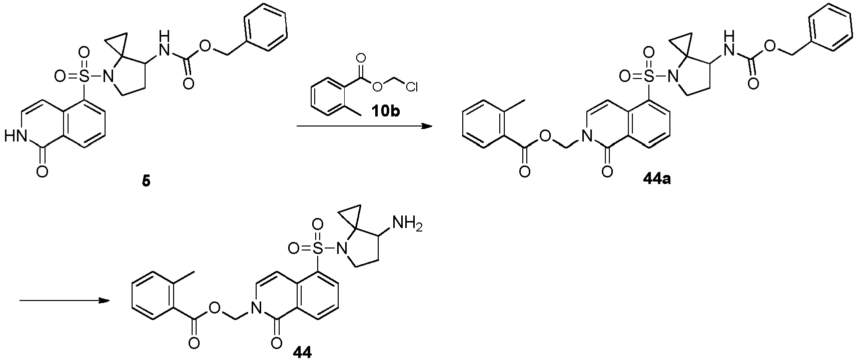

### Step 1

Compound **44a** was obtained referring to step 2 of Example 1. MS-ESI calculated value [M+H]⁺ 602, actually measured value 602.

### Step 2

A formate of compound **44** was obtained referring to step 2 of Example 25. ¹H NMR (400MHz, CD₃OD) δ = 8.69 (d, *J* = 8.0 Hz, 1H), 8.50 (s, 1H), 8.46-8.40 (m, 1H), 7.91 (d, *J* = 7.9 Hz, 1H), 7.80 (d, *J* = 7.9 Hz, 1H), 7.74-7.67 (m, 1H), 7.49-7.41 (m, 1H), 7.32-7.19 (m, 3H), 6.20 (s, 2H), 4.15-4.05 (m, 1H), 3.67-3.57 (m, 1H), 3.49-3.43 (m, 1H), 2.60-2.50 (m, 4H), 2.17-2.05 (m, 1H), 1.28-1.13 (m, 2H), 0.86-0.75 (m, 2H). MS-ESI calculated value [M+H]⁺ 468, actually measured value 468.

The formate of compound **44** can be adjusted the pH of the reaction solution to 8-9 by adding a saturated aqueous sodium carbonate solution to the system, and then, after the extraction with ethyl acetate, the reaction solution was concentrated to obtain compound **44.**

### Example 39: Compound 45

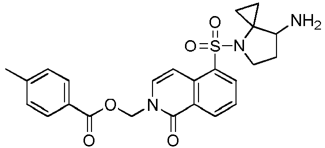

### Synthesis route:

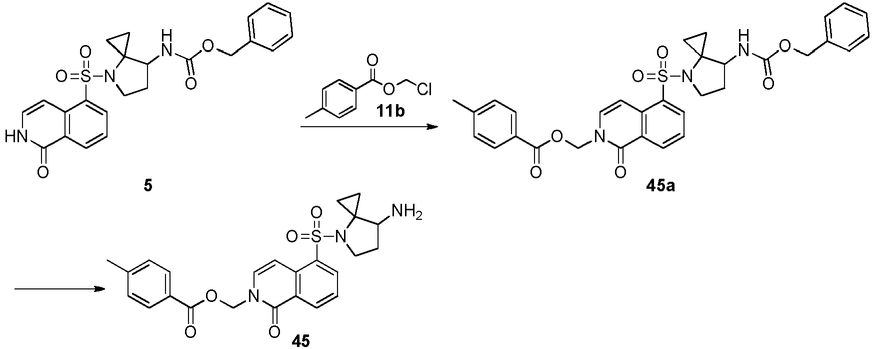

### Step 1

Compound **45a** was obtained referring to step 2 of Example **1.** MS-ESI calculated value [M+H]⁺ 602, actually measured value 602.

### Step 2

A formate of compound **45** was obtained referring to step 2 of Example **25.** ¹H NMR (400MHz, CD₃OD) δ = 8.69 (d, *J =* 8.0 Hz, 1H), 8.46 (s, 1H), 8.44-8.40 (m, 1H), 7.92 (d, *J =* 8.3 Hz, 2H), 7.78 (d, *J =* 8.0 Hz, 1H), 7.70 (t, *J =* 7.9 Hz, 1H), 7.29 (d, *J =* 8.2 Hz, 2H), 7.20 (d, *J =* 8.0 Hz, 1H), 6.20 (s, 2H), 4.12-4.01 (m, 1H), 3.65-3.54 (m, 1H), 3.43-3.36 (m, 1H), 2.58-2.46 (m, 1H), 2.39 (s, 3H), 2.11-1.99 (m, 1H), 1.26-1.08 (m, 2H), 0.86-0.74 (m, 2H). MS-ESI calculated value [M+H]⁺ 468, actually measured value 468.

The formate of compound **45** can be adjusted the pH of the reaction solution to 8-9 by adding a saturated aqueous sodium carbonate solution to the system, and then, after the extraction with ethyl acetate, the reaction solution was concentrated to obtain compound **45.**

### Example 40: Compound 46

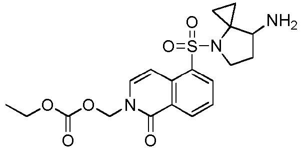

### Synthesis route:

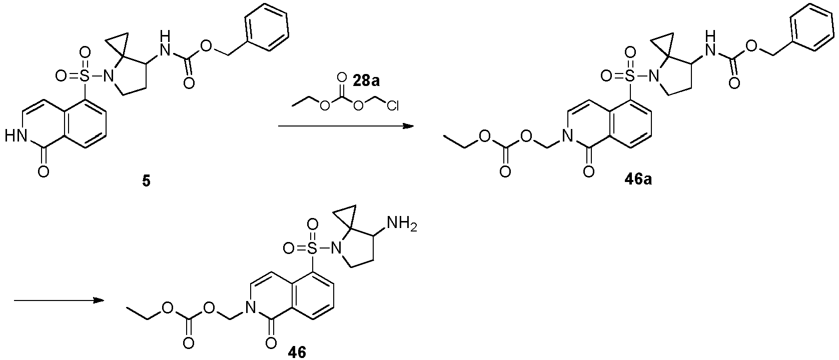

### Step 1

Compound **46a** was obtained referring to step 2 of Example **1.** MS-ESI calculated value [M+H]⁺ 556, actually measured value 556.

### Step 2

A formate of compound **46** was obtained referring to step 2 of Example **25.** ¹H NMR (400MHz, CD₃OD) δ = 8.66 (d, *J* = 7.9 Hz, 1H), 8.52 (s, 1H), 8.46-8.40 (m, 1H), 7.74-7.65 (m, 2H), 7.18 (d, *J* = 7.9 Hz, 1H), 5.99 (s, 2H), 4.27-4.19 (m, 2H), 4.11-4.02 (m, 1H), 3.67-3.56 (m, 1H), 3.45-3.39 (m, 1H), 2.59-2.47 (m, 1H), 2.14-2.02 (m, 1H), 1.29 (t, *J* = 7.1 Hz, 3H), 1.24-1.11 (m, 2H), 0.88-0.76 (m, 2H). MS-ESI calculated value [M+H]⁺ 422, actually measured value 422.

The formate of compound **46** can be adjusted the pH of the reaction solution to 8-9 by adding a saturated aqueous sodium carbonate solution to the system, and then, after the extraction with ethyl acetate, the reaction solution was concentrated to obtain compound **46.**

### Example 41: Compound 47

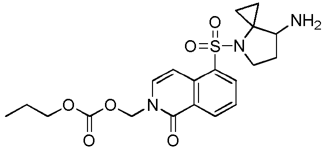

### Synthesis route:

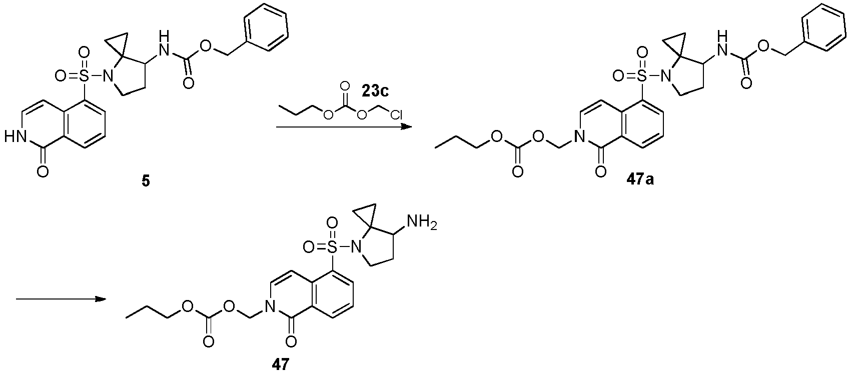

### Step 1

Compound **47a** was obtained referring to step 2 of Example **1.** MS-ESI calculated value [M+H]⁺ 570, actually measured value 570.

### Step 2

A formate of compound **47** was obtained referring to step 2 of Example **25.** ¹H NMR (400MHz, CD₃OD) δ = 8.66 (d, *J =* 7.9 Hz, 1H), 8.49 (s, 1H), 8.44-8.38 (m, 1H), 7.74-7.63 (m, 2H), 7.17 (d, *J =* 7.8 Hz, 1H), 6.01-5.94 (m, 2H), 4.13 (t, *J =* 6.6 Hz, 2H), 4.10-4.02 (m, 1H), 3.65-3.56 (m, 1H), 3.45-3.39 (m, 1H), 2.58-2.47 (m, 1H), 2.13-2.03 (m, 1H), 1.73-1.63 (m, 2H), 1.24-1.10 (m, 2H), 0.94 (t, *J* = 7.5 Hz, 3H), 0.80 (t, *J* = 9.0 Hz, 2H). MS-ESI calculated value [M+H]⁺ 436, actually measured value 436.

The formate of compound **47** can be adjusted the pH of the reaction solution to 8-9 by adding a saturated aqueous sodium carbonate solution to the system, and then, after the extraction with ethyl acetate, the reaction solution was concentrated to obtain compound **47.**

### Example 42: Compound 48

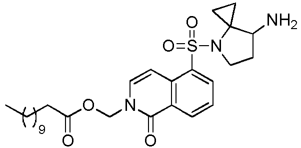

### Synthesis route:

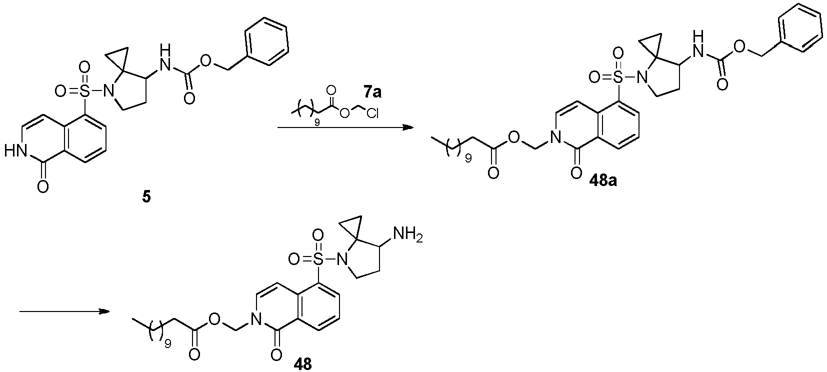

### Step 1

Compound **48a** was obtained referring to step 2 of Example 1. MS-ESI calculated value [M+H]⁺ 666, actually measured value 666.

### Step 2

A formate of compound **48** was obtained referring to step 2 of Example **25.** ¹H NMR (400MHz, CD₃OD) δ = 8.65 (d, *J =* 7.6 Hz, 1H), 8.48 (s, 1H), 8.43-8.39 (m, 1H), 7.74-7.60 (m, 2H), 7.16 (d, *J =* 7.9 Hz, 1H), 5.97 (s, 2H), 4.14-4.02 (m, 1H), 3.65-3.54 (m, 1H), 3.47-3.38 (m, 1H), 2.61-2.47 (m, 1H), 2.37 (t, *J =* 7.3 Hz, 2H), 2.14-2.03 (m, 1H), 1.66-1.54 (m, 2H), 1.30-1.15 (m, 18H), 0.89 (t, *J =* 7.0 Hz, 3H), 0.83-0.73 (m, 2H). MS-ESI calculated value [M+H]⁺532, actually measured value 532.

The formate of compound **48** can be adjusted the pH of the reaction solution to 8-9 by adding a saturated aqueous sodium carbonate solution to the system, and then, after the extraction with ethyl acetate, the reaction solution was concentrated to obtain compound **48.**

### Example 43: Compound 49

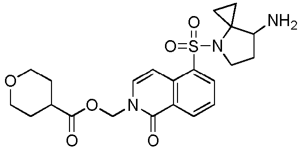

### Synthesis route:

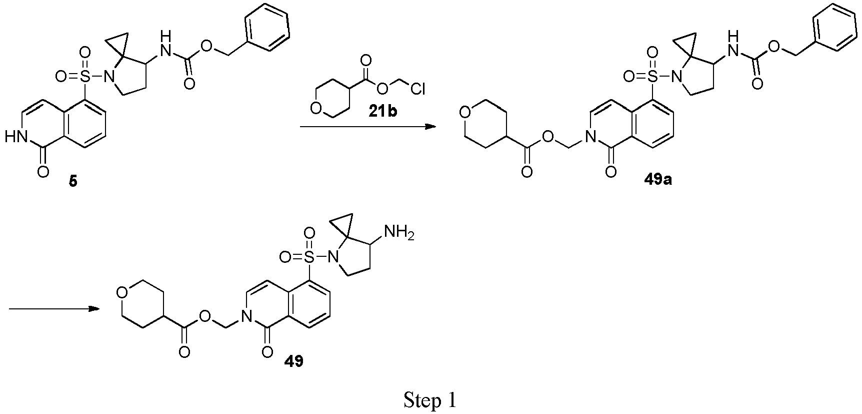

Compound **49a** was obtained referring to step 2 of Example 1. MS-ESI calculated value [M+H]⁺596, actually measured value 596.

### Step 2

A formate of compound **49** was obtained referring to step 2 of Example **25.** ¹H NMR (400MHz, CD₃OD) δ = 8.65 (d, *J* = 7.6 Hz, 1H), 8.49 (s, 1H), 8.43-8.40 (m, 1H), 7.75-7.61 (m, 2H), 7.16 (d, *J* = 7.8 Hz, 1H), 5.99 (s, 2H), 4.12-4.02 (m, 1H), 3.92-3.83 (m, 2H), 3.66-3.54 (m, 1H), 3.47-3.37 (m, 3H), 2.73-2.62 (m, 1H), 2.59-2.45 (m, 1H), 2.15-2.01 (m, 1H), 1.88-1.78 (m, 2H), 1.75-1.61 (m, 2H), 1.24-1.10 (m, 2H), 0.85-0.75 (m, 2H). MS-ESI calculated value [M+H]⁺462, actually measured value 462.

The formate of compound **49** can be adjusted the pH of the reaction solution to 8-9 by adding a saturated aqueous sodium carbonate solution to the system, and then, after the extraction with ethyl acetate, the reaction solution was concentrated to obtain compound **49.**

### Example 44: Compound 50

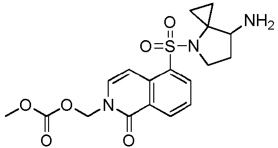

### Synthesis route:

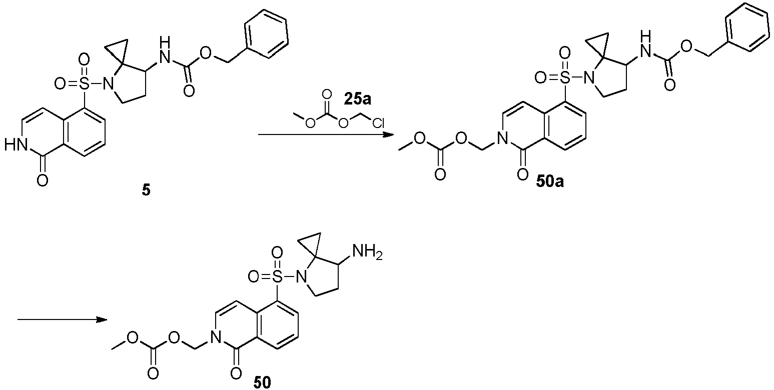

### Step 1

Compound **50a** was obtained referring to step 2 of Example **1.** MS-ESI calculated value [M+H]⁺542, actually measured value 542.

### Step 2

A formate of compound **50** was obtained referring to step 2 of Example **25.** ¹H NMR (400MHz, CD₃OD) δ = 8.64 (d, *J* = 7.9 Hz, 1H), 8.44 (s, 1H), 8.42-8.38 (m, 1H), 7.72-7.64 (m, 2H), 7.14 (d, *J* = 7.8 Hz, 1H), 5.98 (s, 2H), 4.14-4.05 (m, 1H), 3.80 (s, 3H), 3.64-3.56 (m, 1H), 3.48 (d, *J =* 5.1 Hz, 1H), 2.61-2.50 (m, 1H), 2.18-2.07 (m, 1H), 1.26-1.14 (m, 2H), 0.85-0.73 (m, 2H). MS-ESI calculated value [M+H]⁺ 408, actually measured value 408.

The formate of compound **50** can be adjusted the pH of the reaction solution to 8-9 by adding a saturated aqueous sodium carbonate solution to the system, and then, after the extraction with ethyl acetate, the reaction solution was concentrated to obtain compound **50.**

### Example 45: Compound 51

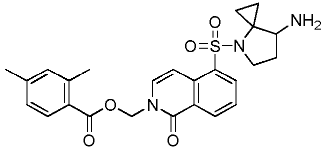

### Synthesis route:

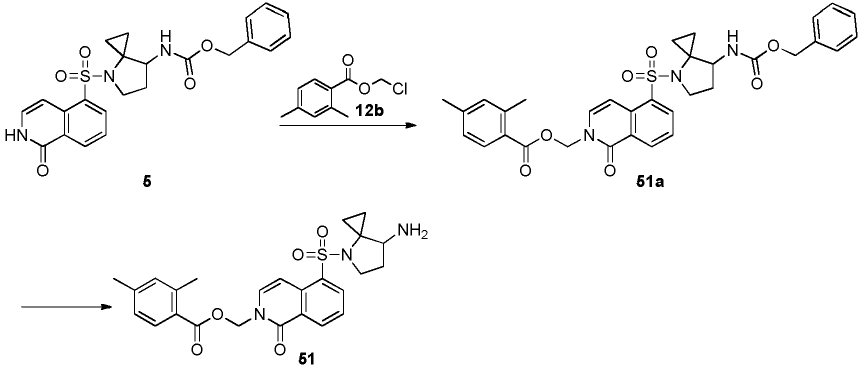

### Step 1

Compound **51a** was obtained referring to step 2 of Example 1. MS-ESI calculated value [M+H]⁺ 616, actually measured value 616.

### Step 2

A formate of compound **51** was obtained referring to step 2 of Example **25.** ¹H NMR (400MHz, CD₃OD) δ = 8.64 (d, *J =* 7.9 Hz, 1H), 8.49 (br s, 1H), 8.42-8.35 (m, 1H), 7.77 (t, *J =* 8.1 Hz, 2H), 7.70-7.62 (m, 1H), 7.18 (d, *J* = 7.8 Hz, 1H), 7.09-6.98 (m, 2H), 6.15 (s, 2H), 4.10-3.98 (m, 1H), 3.63-3.53 (m, 1H), 3.45-3.37 (m, 1H), 2.57-2.44 (m, 4H), 2.30 (s, 3H), 2.14-2.01 (m, 1H), 1.26-1.08 (m, 2H), 0.79 (t, *J* = 9.0 Hz, 2H). MS-ESI calculated value [M+H]⁺482, actually measured value 482.

The formate of compound **51** can be adjusted the pH of the reaction solution to 8-9 by adding a saturated aqueous sodium carbonate solution to the system, and then, after the extraction with ethyl acetate, the reaction solution was concentrated to obtain compound **51.**

### Example 46: Compound 52

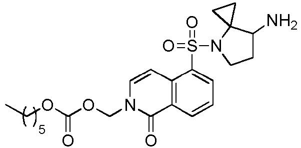

### Synthesis route:

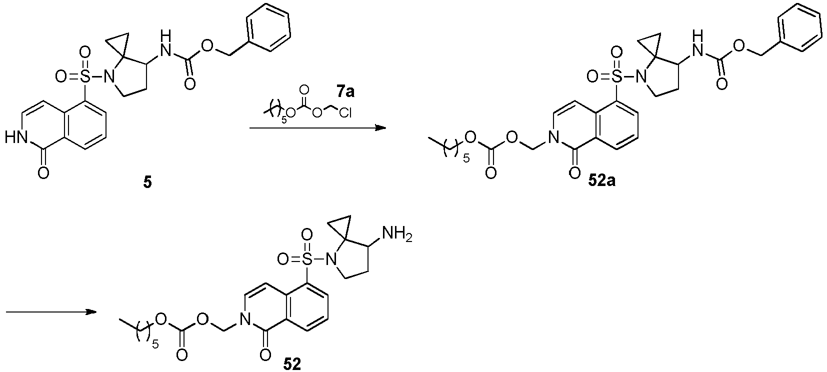

### Step 1

Compound **52a** was obtained referring to step 2 of Example **1.** MS-ESI calculated value [M+Na]⁺ 634, actually measured value 634.

### Step 2

A formate of compound **52** was obtained referring to step 2 of Example **25.** ¹H NMR (400MHz, CD₃OD) δ = 8.66 (d, *J* = 7.8 Hz, 1H), 8.61 (s, 1H), 8.42 (d, *J* = 7.8 Hz, 1H), 7.74-7.64 (m, 2H), 7.14 (d, *J* = 7.8 Hz, 1H), 6.03-5.92 (m, 2H), 4.22-4.05 (m, 3H), 3.59-3.61 (m, 1H), 3.50 (d, *J* = 4.8 Hz, 1H), 2.54-2.57 (m, 1H), 2.20-2.08 (m, 1H), 1.71-1.59 (m, 2H), 1.41-1.12 (m, 8H), 0.87 (t, *J* = 6.8 Hz, 3H), 0.84-0.67 (m, 2H). MS-ESI calculated value [M+H]⁺ 478, actually measured value 478.

The formate of compound **52** can be adjusted the pH of the reaction solution to 8-9 by adding a saturated aqueous sodium carbonate solution to the system, and then, after the extraction with ethyl acetate, the reaction solution was concentrated to obtain compound **52.**

### Example 47: Compound 53

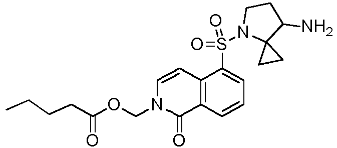

### Synthesis route:

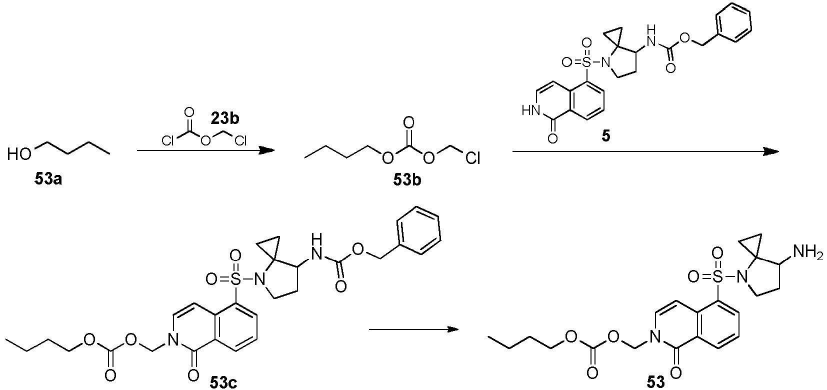

### Step 1

Compound **53b** was obtained referring to step 1 of Example **17.**

### Step 2

Compound **53c** was obtained referring to step 2 of Example **1.** MS-ESI calculated value [M+Na]⁺ 606, actually measured value 606.

### Step 3

A formate of compound **53** was obtained referring to step 2 of Example **25.** ¹H NMR (400MHz, CD₃OD) δ = 8.66 *(d, J* = 8.0 Hz, 1H), 8.48 (s, 1H),8.42 (dd, *J* = 1.1, 7.8 Hz, 1H), 7.75-7.62 (m, 2H), 7.17 (d, *J* = 7.8 Hz, 1H), 6.04-5.91 (m, 2H), 4.17 *(t, J* = 6.6 Hz, 2H), 4.05-4.07 (m, 1H), 3.65-3.53 (m, 1H), 3.41 (dd, *J* = 3.2, 7.6 Hz, 1H), 2.51-2.53 (m, 1H), 2.13-1.99 (m, 1H), 1.69-1.56 (m, 2H), 1.35-1.40 (m, 2H), 1.25-1.08 (m, 2H), 0.92 *(t, J* = 7.4 Hz, 3H), 0.85-0.75 (m, 2H). MS-ESI calculated value [M+H]⁺ 450, actually measured value 450.

The formate of compound **53** can be adjusted the pH of the reaction solution to 8-9 by adding a saturated aqueous sodium carbonate solution to the system, and then, after the extraction with ethyl acetate, the reaction solution was concentrated to obtain compound **53.**

### Example 48: Compound 54

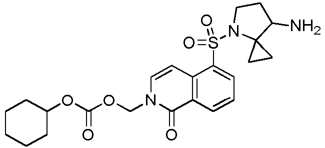

### Synthesis route:

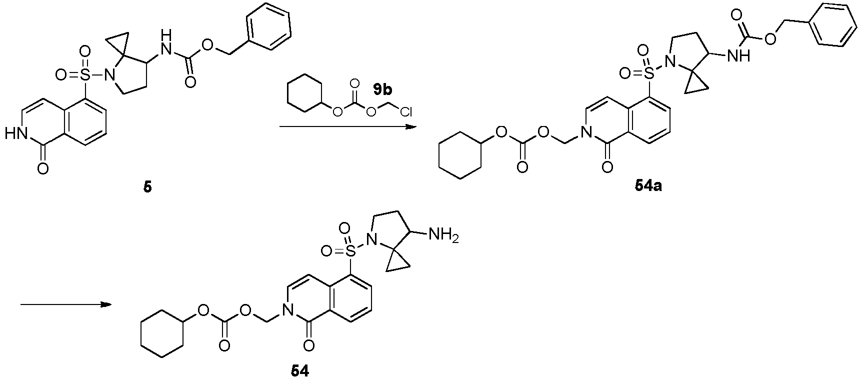

### Step 1

Compound **54a** was obtained referring to step 2 of Example 1. MS-ESI calculated value [M+Na]⁺ 610, actually measured value 610.

### Step 2

A formate of compound **54** was obtained referring to step 2 of Example 25. ¹H NMR (400MHz, CD₃OD) δ = 8.66-8.64 (d, *J* = 7.8 Hz, 1H), 8.50 (s, 1H),8.43-8.41 (d, *J* = 7.8 Hz, 1H), 8.34 (m, 1H), 7.76-7.63 (m, 2H), 7.14 (d, *J =* 7.8 Hz, 1H), 5.96-5.93 (m, 2H), 4.66- 4.61 (m, 1H), 3.94-3.59 (m, *J=* 6.4, 9.0, 11.8 Hz, 1H), 3.58-3.56 (m, *J =* 4.8 Hz, 1H), 3.56-3.24(m, *J =* 4.8 Hz, 1H), 3.24-3.22(m, *J =* 4.8 Hz, 1H),2.41-1.89 (dt, *J =* 8.4, 14.0 Hz, 2H), 1.87 - 1.72 (m, 2H), 1.71-1.36 (m, 7H), 1.36-1.39 (m, 2H), 1.14-1.12(m, *J =* 6.8 Hz, 1H), 0.90-0.75 (m, 1H). MS-ESI calculated value [M+H]⁺ 476, actually measured value 476.

The formate of compound **54** can be adjusted the pH of the reaction solution to 8-9 by adding a saturated aqueous sodium carbonate solution to the system, and then, after the extraction with ethyl acetate, the reaction solution was concentrated to obtain compound **54.**

### Example 49: Compound 55

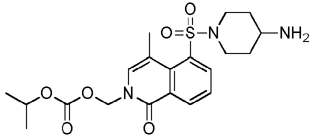

### Synthesis route:

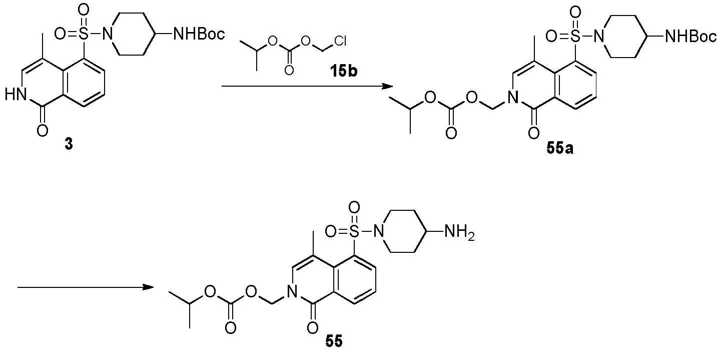

### Step 1

Compound **3** (150 mg, 231 µmol) and potassium carbonate (63.8 mg, 462 µmol) were dissolved in anhydrous tetrahydrofuran (5 mL), stirred for 1 hour at 60 °C. Then compound **15b** (70.4 mg, 462 µmol) was added to the reaction system at 60 °C, stirred for 12 hours at 60 °C. The solvent was removed by concentration under reduced pressure, and the crude product was purified by silica gel plate chromatography (ethyl acetate: ethanol) and concentrated under reduced pressure to obtain compound **55a.** MS-ESI calculated value [M+H]⁺ 538, actually measured value 538.

### Step 2

Compound **55** was obtained referring to step 3 of Example **1** (purified by neutral preparative high performance liquid chromatography). ¹H NMR (400 MHz, CD₃OD) δ = 8.68 (dd, *J* = 1.4, 8.1 Hz, 1H), 8.07 (dd, *J* = 1.5, 7.8 Hz, 1H), 7.63 (t, *J* = 7.9 Hz, 1H), 7.42 (d, *J* = 0.9 Hz, 1H), 5.93 (s, 2H), 4.93-4.89 (m, 1H), 3.84 *(d, J* = 12.9 Hz, 2H), 3.18-3.09 (m, 2H), 2.99-2.87 (m, 1H), 2.68 (d, *J* = 0.8 Hz, 3H), 2.01-1.90 (m, 2H), 1.55-1.42 (m, 2H), 1.28 (d, *J* = 6.3 Hz, 6H). MS-ESI calculated value [M+H]⁺ 438, actually measured value 438.

### Example 50: Compound 56

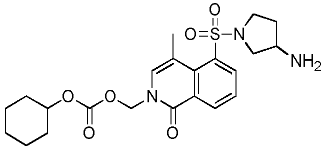

### Synthesis route:

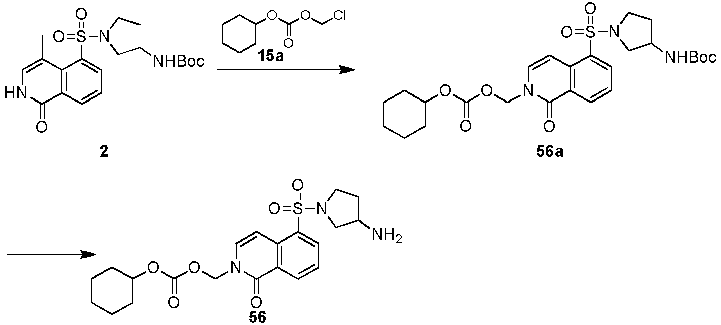

### Step 1

To a solution of compound **2** (435 mg, 1.07 mmol) in N,N-dimethylformamide (10 mL) was added potassium tert-butoxide (359 mg, 3.20 mmol) at 0 °C, stirred for 1 hour at 0 °C. Compound **15a** (226 mg, 1.17 mmol) was added to the reaction solution at 0 °C, stirred for 13 hours at 25 °C. Water (4 mL) was added to the reaction solution which was extracted with ethyl acetate (3 mL ×3), the combined organic phase was washed with saturated brine (5 mL ×3), dried with anhydrous sodium sulfate, filtered, concentrated under reduced pressure, and the crude product was separated and purified by silica gel column chromatography (petroleum ether/ethyl acetate) to obtain compound **56a.**

### Step 2

To a solution of compound **56a** (150 mg, 0.266 mmol) in anhydrous ethyl acetate (2 mL) added ethyl acetate hydrochloride (4M, 16.0 mmol), stirred for 3 hours at 20 °C. The solvent was removed by concentration under reduced pressure, and the crude product was prepared by high performance liquid chromatography (hydrochloride system) to obtain a hydrochloride of compound 56. ¹H NMR (400MHz, CD₃OD) δ8.68 (d, *J* = 8.0 Hz, 1 H), 8.46 (s, 1 H), 8.39 (dd, *J* = 7.8, 1.2 Hz, 1 H), 7.73 (t, *J* = 7.8 Hz, 1 H), 7.67 (d, *J* = 7.8 Hz, 1 H), 7.51 (d, *J* = 7.8 Hz, 1 H), 6.00 (s, 2 H), 4.62-4.71 (m, 1 H), 3.77-3.87 (m, 1 H), 3.50-3.64 (m, 2 H), 3.34-3.40 (m, 2 H), 2.24-2.36 (m, 1 H), 1.86-2.03 (m, 3 H), 1.75 (dt, *J* = 6.4, 3.1 Hz, 2 H), 1.24-1.61 (m, 6 H). MS-ESI calculated value [M+H]⁺ 450, actually measured value 450.

The hydrochloride of compound **56** can be adjusted the pH of the reaction solution to 8-9 by adding a saturated aqueous sodium carbonate solution to the system, and then, after the extraction with ethyl acetate, the reaction solution was concentrated to obtain compound **56.**

### Example 51: Compound 57

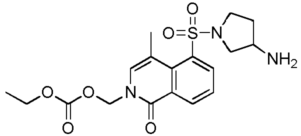

### Synthesis route:

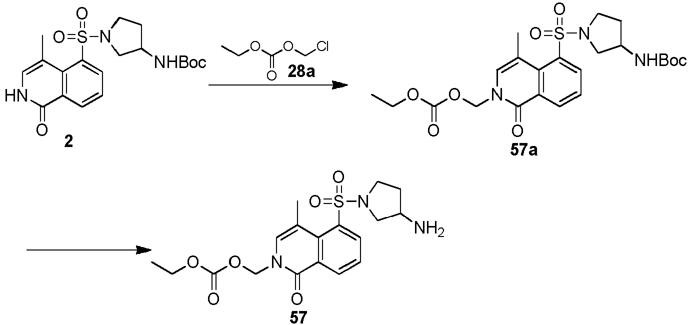

### Step 1

Compound **57a** was obtained referring to step 1 of Example 50. MS-ESI calculated value [M+H]⁺ 510, actually measured value 510.

### Step 2

A formate of compound 57 was obtained from crude product by high performance liquid chromatography (formic acid system) referring to step 3 of Example **1.** ¹H NMR (400MHz, CDCl₃) δ 8.69-8.63 (m, 1H), 8.25-8.20 (m, 1H), 7.64-7.61 (m, 1H), 7.59-7.40 (m, 1H), 5.96-5.74 (m, 2H), 4.24-4.20 (m, 2H), 3.70-3.66 (m, 3H), 3.54 (s, 1H), 3.20-3.18 (m, 1H), 2.68-2.66 (m, 3H), 2.28-2.27 (m, 1H), 1.92-1.91 (m, 1H), 1.31-1.26 (m, 3H). MS-ESI calculated value [M+H]⁺ 410, actually measured value 410.

The formate of compound **57** can be adjusted the pH of the reaction solution to 8-9 by adding a saturated aqueous sodium carbonate solution to the system, and then, after the extraction with ethyl acetate, the reaction solution was concentrated to obtain compound **57.**

### Example 52: Compound 58

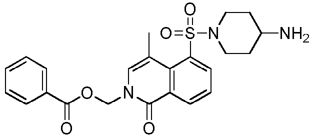

### Synthesis route:

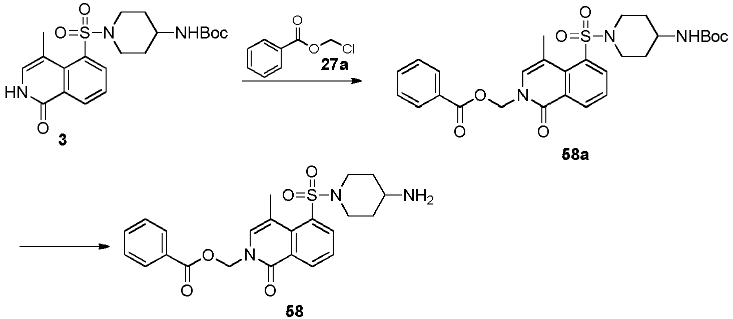

### Step 1

Compound **58a** was obtained referring to step 2 of Example 1. MS-ESI calculated value [M+H]⁺ 556, actually measured value 556.

### Step 2

Compound **58** was obtained from crude product by high performance liquid chromatography (neutral system) referring to step 3 of Example 1. ¹H NMR (400MHz, CD₃OD) δ = 8.72 (d, *J* = 8.1 Hz, 1H), 8.10 (d, *J* = 7.7 Hz, 1H), 8.04 (d, *J* = 8.1 Hz, 2H), 7.68-7.59 (m, 2H), 7.56 (s, 1H), 7.51-7.45 (m, 2H), 6.20 (s, 2H), 3.85 (d, *J* = 13.0 Hz, 2H), 3.14 *(t, J* = 11.2 Hz, 2H), 2.99-2.89 (m, 1H), 2.72 (s, 3H), 1.95 (d, *J* = 10.9 Hz, 2H), 1.55-1.42 (m, 2H). MS-ESI calculated value [M+H]⁺ 456, actually measured value 456.

### Example 53: Compound 59

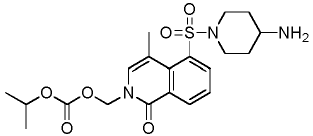

### Synthesis route:

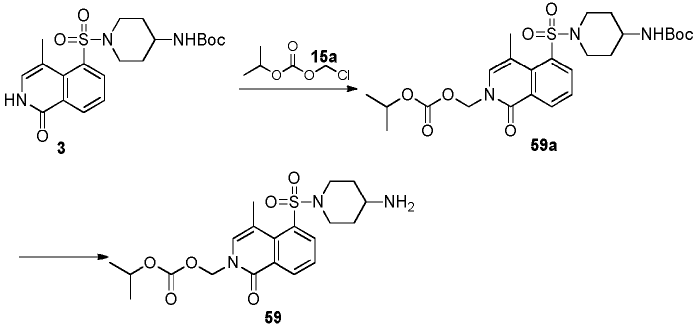

### Step 1

Compound **59a** was obtained referring to step 1 of Example **49**. MS-ESI calculated value [M+H]⁺ 538, actually measured value 538.

### Step 2

A formate of compound **59** was obtained referring to step 3 of Example **1.** ¹H NMR (400MHz, CD₃OD) δ = 8.73 *(d, J* = 7.7 Hz, 1H), 8.55 (s, 1H), 8.10 *(d, J* = 7.6 Hz, 1H), 7.66 *(t, J* = 7.8 Hz, 1H), 7.47 (s, 1H), 5.96 (s, 2H), 4.59 (m, 1H), 3.99 (d, *J* = 12.8 Hz, 2H), 3.46-3.37 (m, 1H), 3.23 *(t, J* = 12.3 Hz, 2H), 2.70 (s, 3H), 2.14 (d, *J* = 11.2 Hz, 2H), 1.85-1.61 (m, 2H), 1.30 (d, *J* = 6.1 Hz, 6H). MS-ESI calculated value [M+H]⁺ 438, actually measured value 438.

The formate of compound **59** can be adjusted the pH of the reaction solution to 8-9 by adding a saturated aqueous sodium carbonate solution to the system, and then, after the extraction with ethyl acetate, the reaction solution was concentrated to obtain compound **59.**

### Example 54: Compound 60

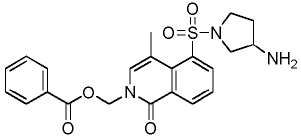

### Synthesis route:

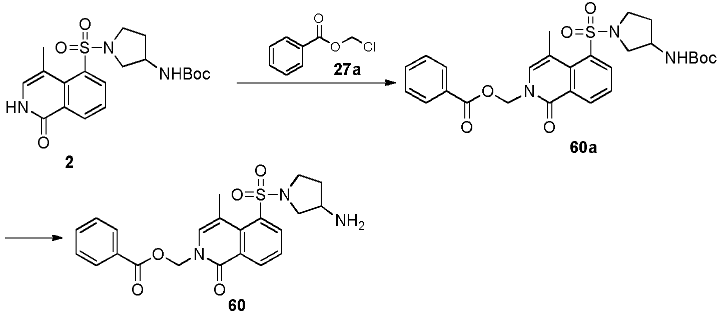

### Step 1

Compound **60a** was obtained referring to step 1 of Example **49.** MS-ESI calculated value [M+H]⁺ 542, actually measured value 542.

### Step 2

Compound **60** was obtained from crude product by high performance liquid chromatography (neutral system) referring to step 3 of Example 1. MS-ESI calculated value [M+H]⁺ 442, actually measured value 442. ¹H NMR (400MHz, CD₃OD) δ = 8.72 (dd, *J* = 1.41, 8.01 Hz, 1H), 8.25 (dd, *J* = 1.47, 7.70 Hz, 1H), 7.98-8.11 (m, 2H), 7.58-7.71 (m, 2H), 7.56 (d, *J* = 0.86 Hz, 1H), 7.43-7.52 (m, 2H), 6.21 (s, 2H), 3.63-3.76 (m, 3H), 3.51-3.60 (m, 1H), 3.21 (dd, *J* = 4.65, 9.17 Hz, 1H), 2.72 (d, *J* = 0.86 Hz, 3H), 2.24-2.35 (m, 1H), 1.92 (dd, *J* = 6.97, 13.33 Hz, 1H).

### Example 55: Compound 61

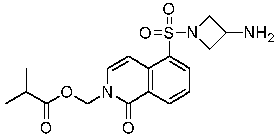

### Synthesis route:

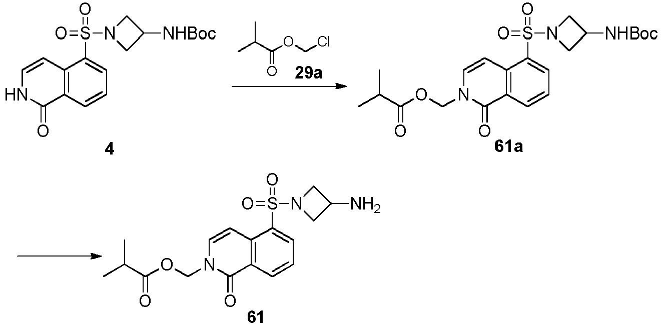

### Step 1

Compound **61a** was obtained referring to step 1 of Example **49.** MS-ESI calculated value [M+Na]⁺ 502, actually measured value 502.

### Step 2

A formate of compound **61** was obtained referring to step 3 of Example **1.** MS-ESI calculated value [M+Na]⁺ 402, actually measured value 402. ¹H NMR (400MHz, CDCl₃) δ = 8.84-8.56 (m, 1H), 8.30 (d, *J* = 6.5 Hz, 1H), 8.13 (s, 1H), 7.56 (s, 1H), 7.45 *(d, J* = 7.5 Hz, 1H), 7.28-7.23 (m, 1H), 5.94 (s, 2H), 4.10-3.96 (m, 2H), 3.85 (s, 2H), 3.79 (s, 2H), 3.72-3.37 (m, 1H), 2.74-2.46 (m, 1H), 1.25-1.04 (m, 6H).

The formate of compound **61** can be adjusted the pH of the reaction solution to 8-9 by adding a saturated aqueous sodium carbonate solution to the system, and then, after the extraction with ethyl acetate, the reaction solution was concentrated to obtain compound **61.**

### Example 56: Compound 62

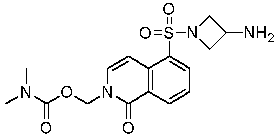

### Synthesis route:

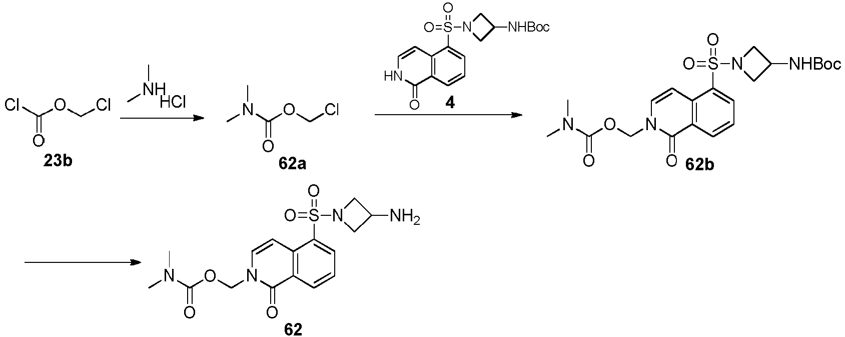

### Step 1

To a solution of dimethylamine hydrochloride (332 mg, 4.07 mmol) and potassium carbonate (1.23 g, 8.92 mmol) in anhydrous dichloromethane (5 mL) was added dropwise chloromethylchloroformate **23b** (500 mg, 3.88 mmol) at -78 °C, stirred for 3 hours at -78 °C. The reaction solution was filtered when being cold, and concentrated to obtain the crude product compound **62a** which was directly used for the next step of the reaction without purification. ¹H NMR (400MHz, CDCl₃) δ = 5.71 (s, 2H), 2.89 (d, *J* = 8.8 Hz, 6H).

### Step 2

Compound **62b** was obtained referring to step 1 of Example **49.** MS-ESI calculated value [M+H]⁺ 481, actually measured value 481.

### Step 3

A formate of compound **62** was obtained referring to step 3 of Example **1.** MS-ESI calculated value [M+H]⁺ 381, actually measured value 381. ¹H NMR (400MHz, CD₃OD) δ = 8.66 *(d, J* = 7.6 Hz, 1H), 8.37 (s, 1H), 8.36 (dd, *J* = 1.3, 7.7 Hz, 1H), 7.74-7.61 (m, 2H), 7.32 (d, *J* = 7.9 Hz, 1H), 5.96 (s, 2H), 4.13-4.06 (m, 2H), 3.99-3.87 (m, 1H), 3.87-3.77 (m, 2H), 2.91 (d, *J* = 2.7 Hz, 6H).

The formate of compound **62** can be adjusted the pH of the reaction solution to 8-9 by adding a saturated aqueous sodium carbonate solution to the system, and then, after the extraction with ethyl acetate, the reaction solution was concentrated to obtain compound **62.**

### Example 57: Compound 63

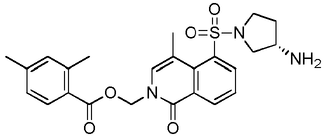

### Synthesis route:

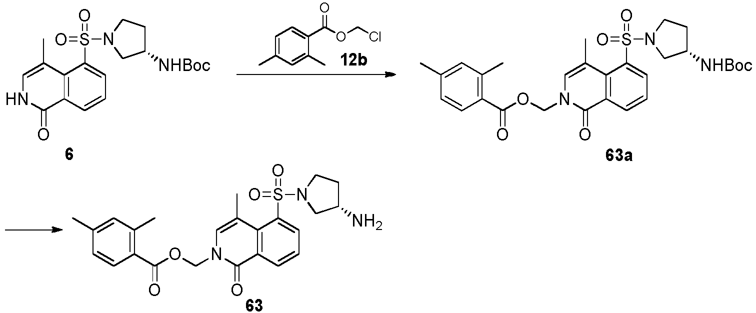

### Step 1

Compound **63a** was obtained referring to step 1 of Example **49.** MS-ESI calculated value [M+H]⁺ 570, actually measured value 570.

### Step 2

Compound **63** was obtained from crude product by high performance liquid chromatography (neutral system) referring to step 3 of Example **50.** MS-ESI calculated value [M+H]⁺ 470, actually measured value 470. ¹H NMR (400MHz, CD₃OD) δ = 8.71 (dd, *J* = 1.4, 8.0 Hz, 1H), 8.24 (dd, *J* = 1.5, 7.8 Hz, 1H), 7.81 (d, *J* = 7.9 Hz, 1H), 7.64 *(t, J* = 7.9 Hz, 1H), 7.54 (d, *J* = 0.9 Hz, 1H), 7.09 (s, 1H), 7.05 (d, *J* = 7.9 Hz, 1H), 6.14 (s, 2H), 3.77-3.62 (m, 3H), 3.59-3.50 (m, 1H), 3.20 (dd, *J* = 4.9, 9.5 Hz, 1H), 2.70 (d, *J* = 0.9 Hz, 3H), 2.53 (s, 3H), 2.32 (s, 3H), 2.30-2.23 (m, 1H), 1.96-1.86 (m, 1H).

### Example 58: Compound 64

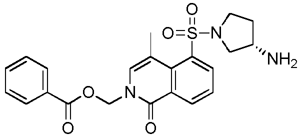

### Synthesis route:

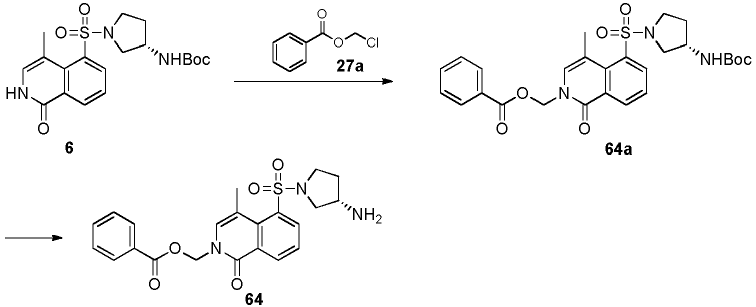

### Step 1

Compound **64a** was obtained referring to step 1 of Example **49.** MS-ESI calculated value [M+H]⁺ 542, actually measured value 542.

### Step 2

Compound **64** was obtained from crude product by high performance liquid chromatography (neutral system) referring to step 3 of Example **1.** MS-ESI calculated value [M+H]⁺ 442, actually measured value 442. ¹H NMR (400 MHz, CD₃OD) δ = 8.69-8.63 (m, 1H), 8.23-8.16 (m, 1H), 8.04-7.96 (m, 2H), 7.64-7.54 (m, 2H), 7.51 (s, 1H), 7.46-7.39 (m, 2H), 6.17 (s, 2H), 3.75-3.59 (m, 3H), 3.57-3.48 (m, 1H), 3.23-3.13 (m, 1H), 2.67 (s, 3H), 2.33-2.20 (m, 1H), 1.96-1.81 (m, 1H).

### Example 59: Control compound 1

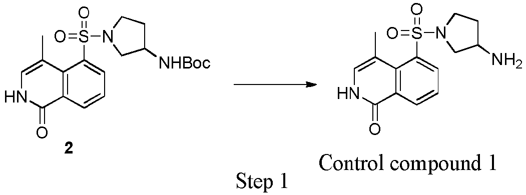

To a solution of intermediate **2** (360 mg, 0.82 mmol) in ethyl acetate (5 mL) was added dropwise an ethyl acetate hydrochloride solution (1.86 mL, 4M). After the reaction was completed, the precipitated solid was filtered, then washed with ethyl acetate (5 mL ×3), and dried to obtain a hydrochloride of control compound **1.** MS-ESI calculated value [M+H]⁺ 308, actually measured value 308.

The hydrochloride of control compound **1** can be adjusted the pH of the reaction solution to 8-9 by adding a saturated aqueous sodium carbonate solution to the system, and then, after the extraction with ethyl acetate, the reaction solution was concentrated to obtain control compound **1.**

### Example 60: Control compound 2

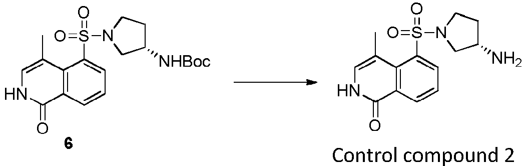

### Step 1

To a solution of intermediate **6** (4 g, 9.57 mmol) in tetrahydrofuran (40 mL) was added concentrated sulfuric acid (1.88 g, 19.15 mmol) dropwise at 0 °C. After the reaction was completed, the precipitated solid was filtered, and then control compound **2** was prepared by high performance liquid chromatography (neutral system). MS-ESI calculated value [M+H]⁺ 308, actually measured value 308. ¹H NMR (400 MHz, CD₃OD) δ 8.68 (d, 1H), 8.24-8.26 (m, 1H), 7.61-7.65 (m, 1H), 7.13 (s, 1H), 3.67-3.73 (m, 3H), 3.57-3.66 (m, 1H), 3.19-3.22 (m, 1H), 2.69 (s, 3H), 2.27-2.30 (m, 1H), 1.91-1.95 (m, 1H).

### Bioassay experiments

### Experimental example 1. Pharmacokinetic test in aqueous humor

### Experiment objective:

The compounds are prodrug molecules containing ester functional groups, which can be hydrolyzed into active drug molecules (technical concentrate) by the action of abundant ester hydrolases in the ocular tissue during eye-drop administration. In this experiment, the production rate of the active drug ingredients and the exposure amount of the active drug ingredients were detected.

### Experiment materials:

Male New Zealand rabbits, aged 3-6 months, weighted 2.0-5.0 kg, purchased from PizhouDongfang Breeding Co.Ltd.

### Preparation of eye-drop sample:

Solvents used were 1.2% hydroxypropyl methyl cellulose E5/ 20.5% Poloxamer P407/1.6% Poloxamer P188.

### Experiment operations:

The dose of eye-drop administraion was 0.5 mg/eye and eye-drop administration was performed on both eyes. Aqueous humor was collected 0.25 h. 0.5 h, 2 h, 4 h, 8 h, and 24 h after administration to prepare the aqueous humor sample. All samples were quantitatively detected for the content of the administered compound in aqueous humor of the experiment animals by liquid chromatography-coupled mass spectrometry (LC-MS) and mass spectrometry (MS) technology. The detected concentration values were subject to WinNonlin non-compartment model. According to the aqueous humor concentration-time data, parameters of the half-life period, peak concentration of the drug in the aqueous humor, peak time of the drug in the aqueous humor, unit exposure and the like were calculated.

**Table 1. Pharmacokinetic test results in aqueous humor of New Zealand rabbits**

| **Test sample (compounds obtained in various examples)** | **Peak concentration of drug in aqueous humor (nM)** | **Half-life period (h)** | **Peak time of drug in aqueous humor (h)** | **Unit exposure (nM.h)** |
|---|---|---|---|---|
| Formate of compound 30 | 1222 | 3.39 | 2.00 | 8322 |
| Formate of compound 31 | 3505 | 2.39 | 0.5 | 15911 |
| Formate of compound 42 | 3810 | 1.91 | 2.0 | 14826 |
| Hydrochloride of compound 56 | 3345 | 2.47 | 2.00 | 14124 |
| Compound 60 | 2600 | 2.83 | 0.5 | 14332 |
| Compound 63 | 7470 | 2.72 | 2.0 | 36581 |
| Hydrochloride of control compound 1 | 425 | 3.9 | 0.5 | 2508 |
| Control compound 2 | 868 | -- | 2.0 | 3555 |

| | | | | |
|---|---|---|---|---|
| "--": not detected. **Conclusion:** Experiment results showed that the test sample compounds (prodrug molecules) were not detected in the aqueous humor, and what were mainly detected were their active metabolites (technical concentrate molecules) after the ester hydrolysis. Compared with control compound 1 and control compound 2, the compounds of the present disclosure significantly increase the exposure amount of the active drugs and meanwhile significantly increase the peak blood concentration and the action duration. | | | | |

### Experimental example 2. Intraocular pressure reduction experiment in New Zealand rabbits with normal intraocular pressure

### Experiment objective:

Rabbits with normal intraocular pressure were adopted to screen intraocular pressure reduction actions of the potential compounds by eye-drop administration.

### Experiment materials:

Male New Zealand rabbits, aged 97-127 days, weighted 2.65-3.5 kg, purchased from PizhouDongfang Breeding Co.Ltd.

### Experiment operations:

The male New Zealand rabbits were randomly divided into 8 rabbits/per group using a computer-generated randomization method. Animals in each group were eye-dropped with different test samples in the right eye, and normal saline or solvent in the left eye, and the volume of the administration was both 50 µL/eye. The intraocular pressure of the animals was measured before administration and in 1, 2, 4, 6, 8 and 10 hours after the administration, respectively. Experimental results are shown in Table 2:

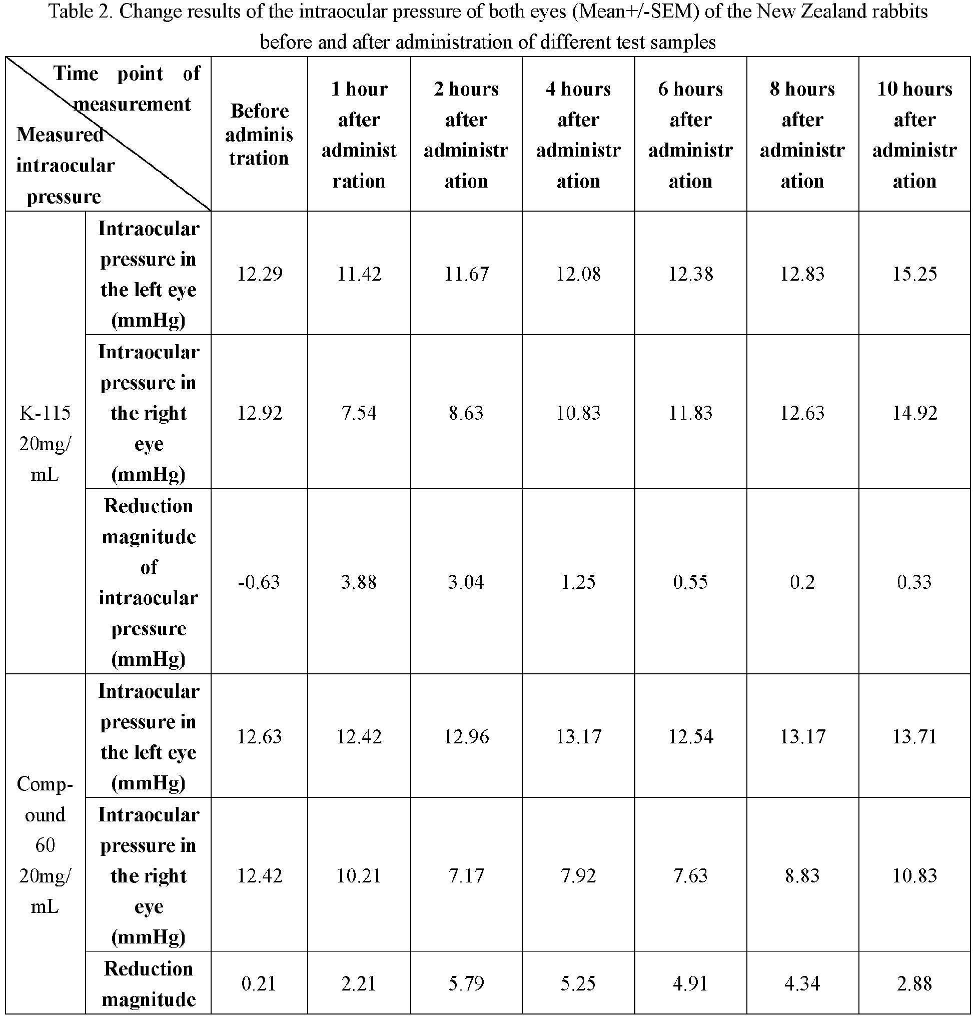

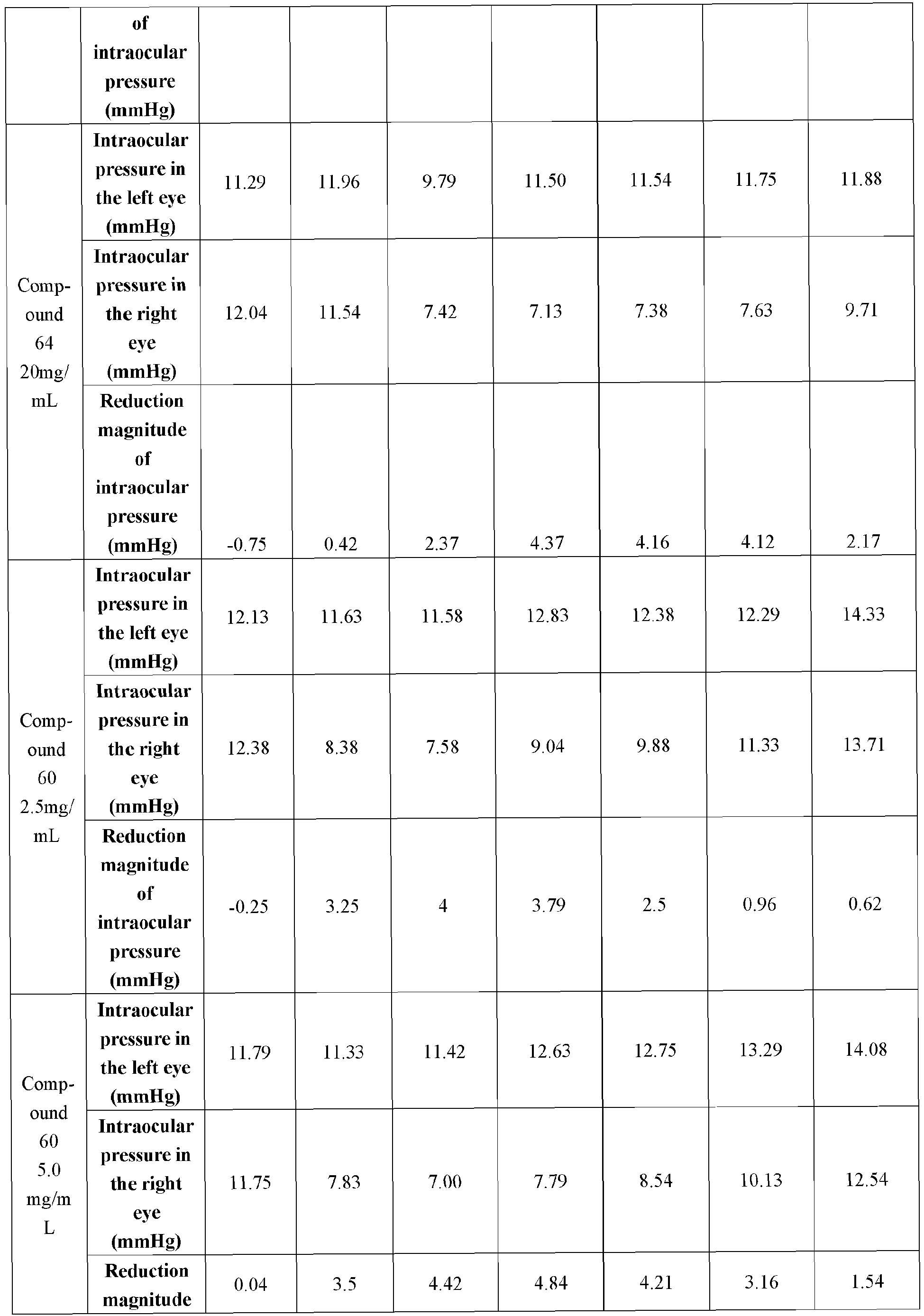

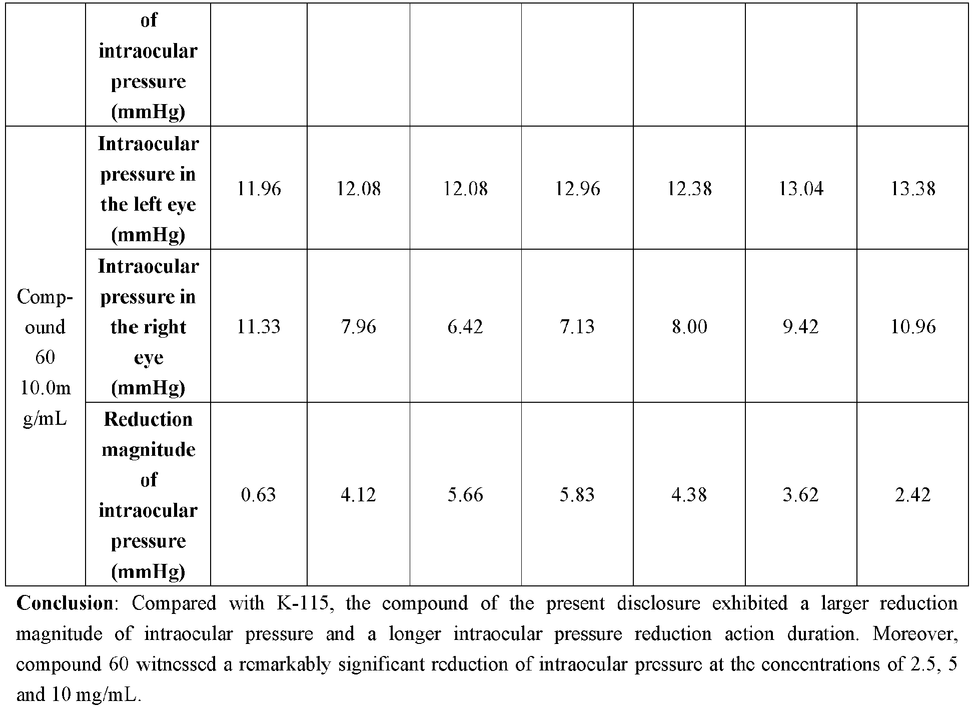

### Experimental example 3. Intraocular pressure reduction experiment in New Zealand rabbits with acute ocular hypertension

### Experiment objective:

Acute ocular hypertension of rabbits were induced by anterior chamber injection of a viscoelastic agent and the intraocular pressure reduction action of compound 60 and compound 63 at different concentrations were explored by eye-drop administration.

### Experiment materials:

Male New Zealand rabbits, aged 97-127 days, weighted 2.5-3.4 kg, purchased from PizhouDongfang Breeding Co.Ltd.

### Experiment operation 1:

Male New Zealand rabbits were randomly divided into 8 rabbits/per group according to body weight using a computer-generated randomization method. The animals in each group were injected with medical sodium hyaluronate gel in the anterior chamber of right eye in a single dose, 100 µL/eye, to induce ocular hypertension. In 5-15 minutes, 3 hours and 6 hours after right eye modeling, both eyes were dropped with a solvent, K-115 or test samples (compound 60 at different concentrations) at a volume of 50 µL/eye and intraocular pressure of the animals was measured before administration and in 1, 2, 4, 6, 8 and 10 hours after administration, respectively. Experimental results are shown in Table 3:

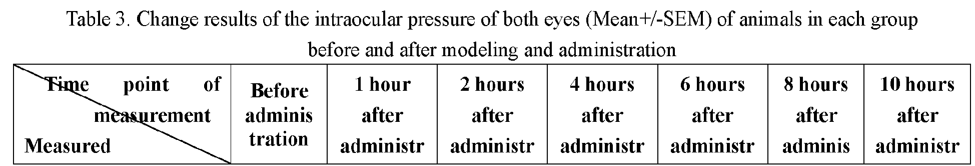

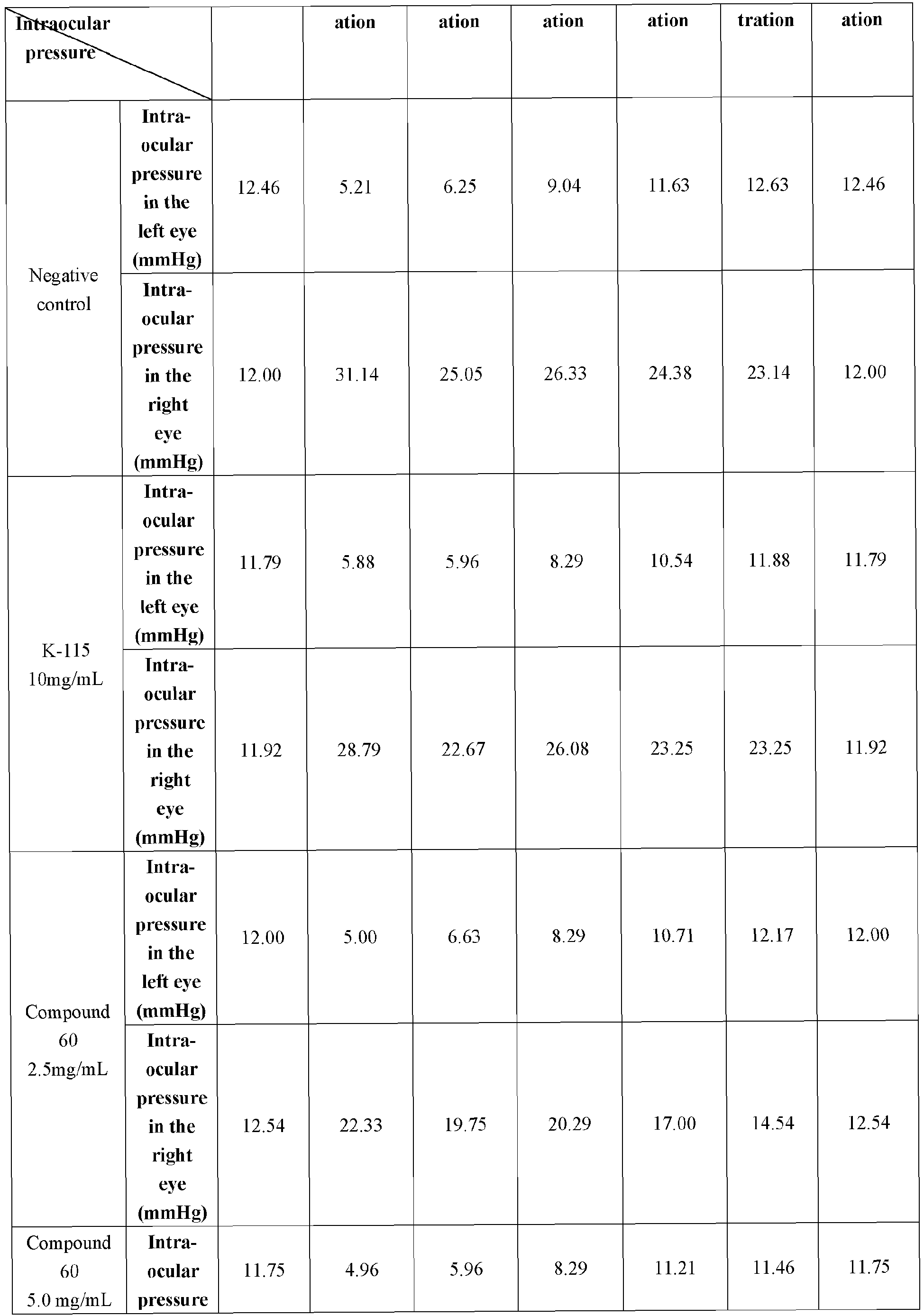

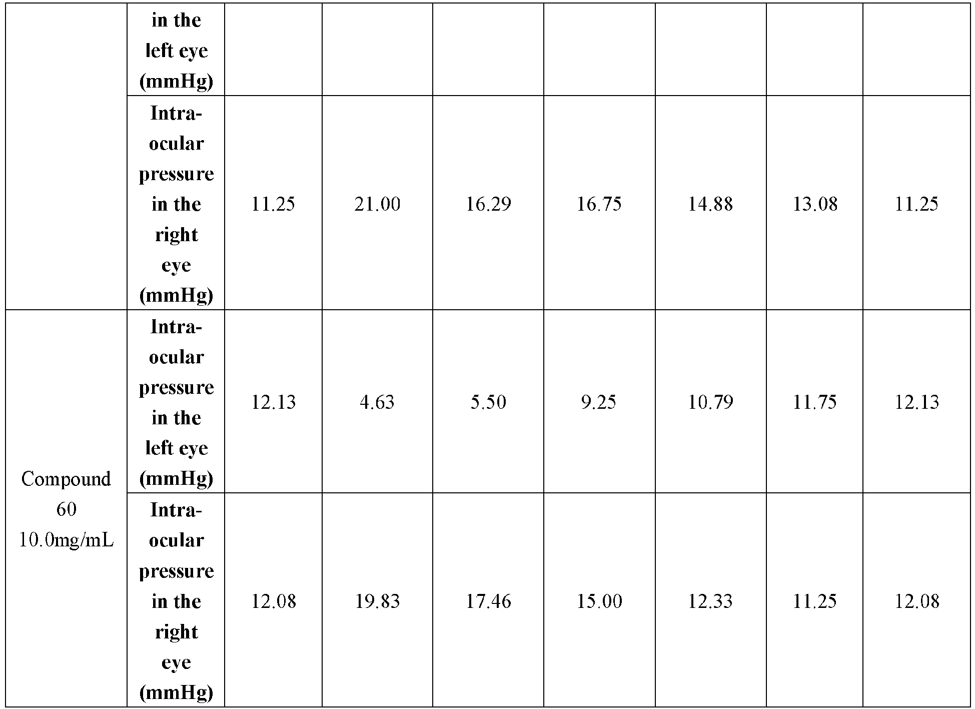

### Experiment operation 2:

50 male New Zealand rabbits were randomly divided into 5 groups according to body weight, with 10 rabbits/per group. Animals in 1-5 groups were injected with medical sodium hyaluronate gel in the anterior chamber of right eye in a single dose, 100 µL/eye, to induce ocular hypertension. In 5-15 minutes after modeling, the right eye was respectively dropped with a solvent, K-115 and test samples (compound 63 at different concentrations), the left eye was dropped with a solvent, the volume of the administration was all 50 µL/eye, and intraocular pressure in both eyes of the animals was measured before administration and in 2, 4, 6, 8 and 10 hours after administration, respectively. Experimental results are shown in Table 4:

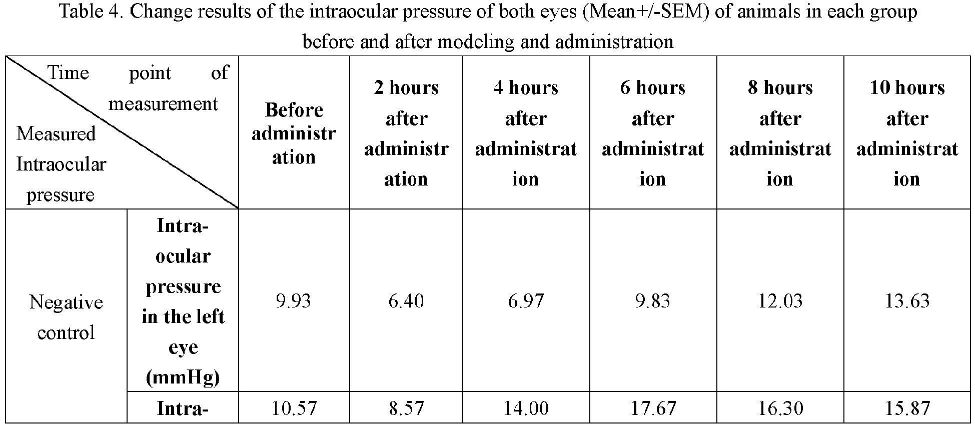

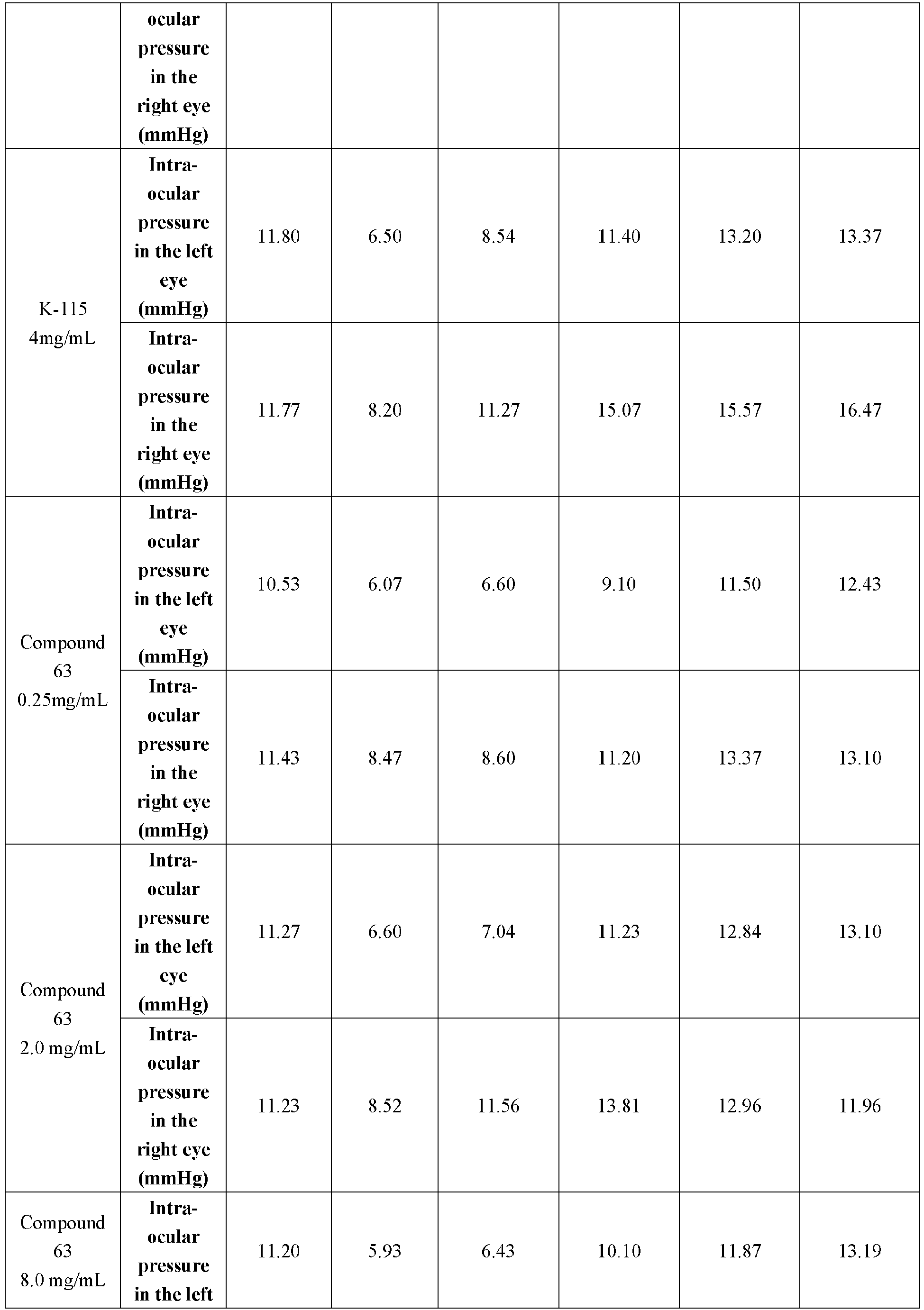

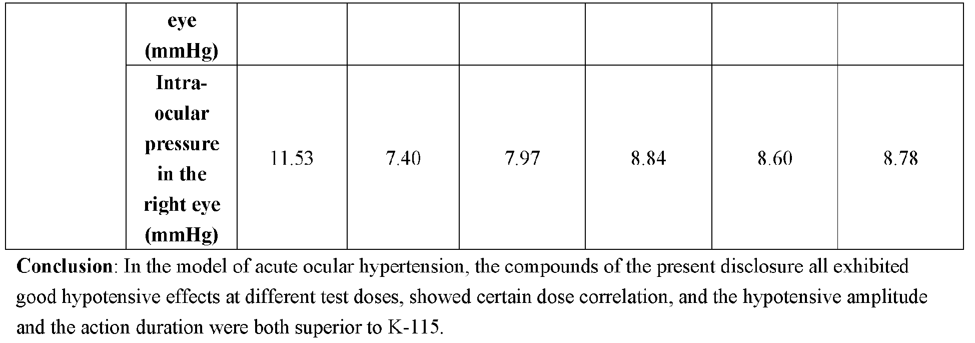

### Experimental example 4. Experiment on reduction of intraocular pressure and ocular toxicity of New Zealand rabbits with normal intraocular pressure by repeated eye-drop administration for 14 days

### Experiment objective:

The intraocular pressure reduction action and the potential ocular toxicity of compound **63** were explored by 14 days of repeated eye-drop administration in rabbits with normal intraocular pressure.

### Experiment materials:

Male New Zealand rabbits, aged 97-127 days, weighted 2.6-3.5 kg, purchased from PizhouDongfang Breeding Co.Ltd.

### Experiment operation 1:

The male New Zealand rabbits were randomly divided into 7 groups, with 6 rabbits per group. Animals were randomly divided according to body weight. Animals in 1-7 groups were eye-dropped with solvent/control sample/test sample in both eyes, the volume of the administration was all 50 µL/eye, once per day for 14 consecutive days, and the day of the administration was recorded as Day 1. On Day 1, intraocular pressure of the animals was measured before the administration and in 1, 2, 4, 6, 8 and 10 hours after the administration, respectively; on Days 2-14, intraocular pressure of the animals was measured in 1 hour after daily administration for the group administered with K-115, and intraocular pressure of the animal was measured in 4 hours after daily administration for the other groups. Experiment results are shown in Tables 5, 6 and 7:

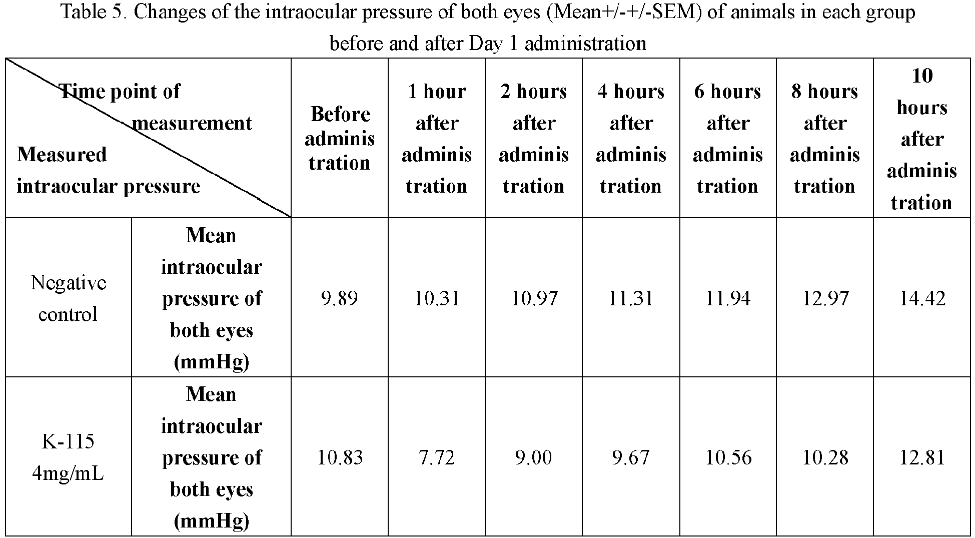

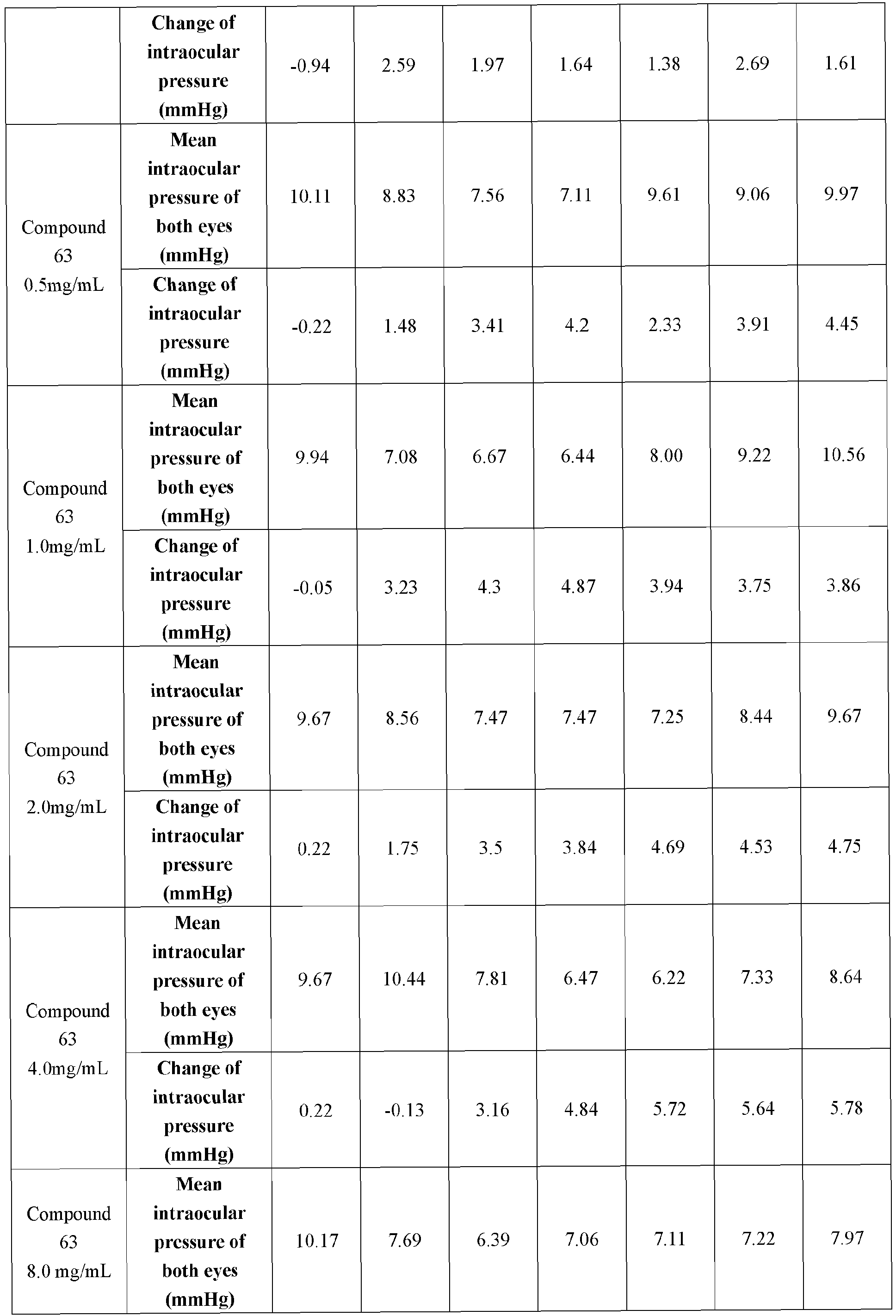

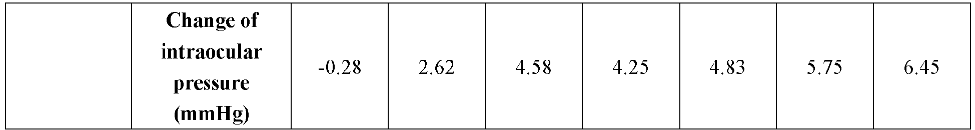

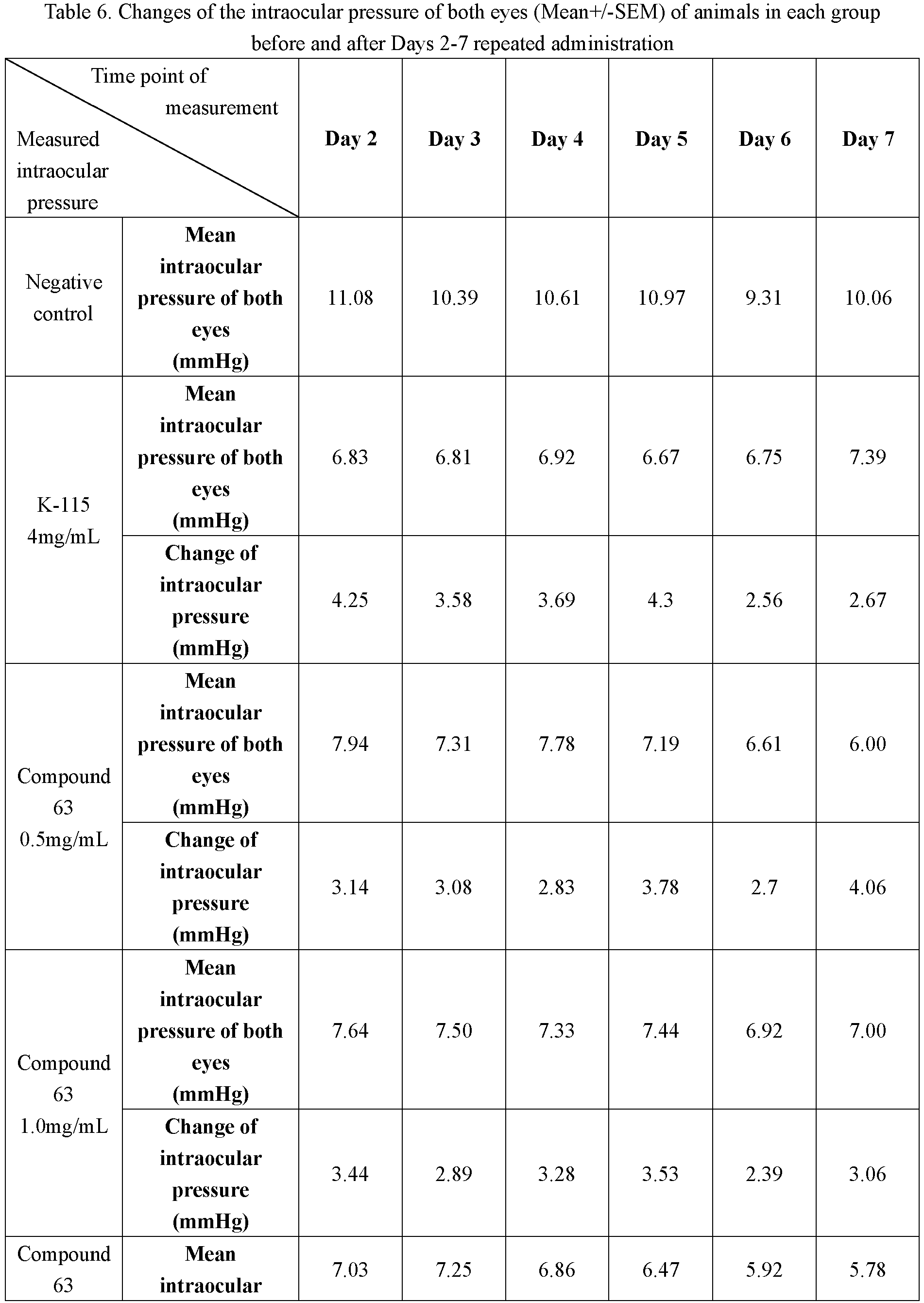

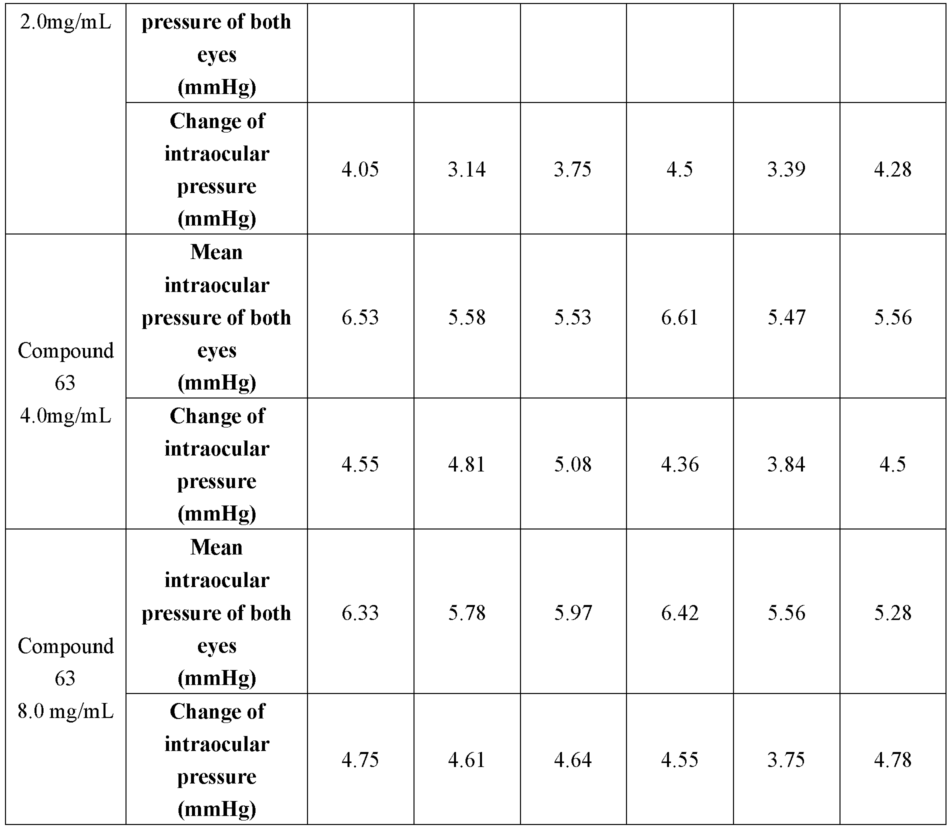

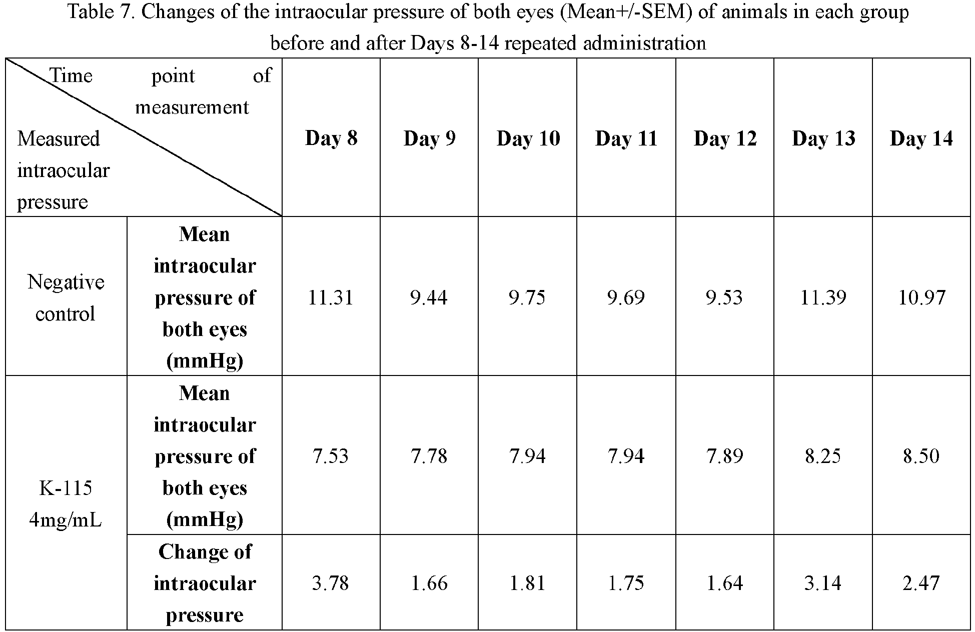

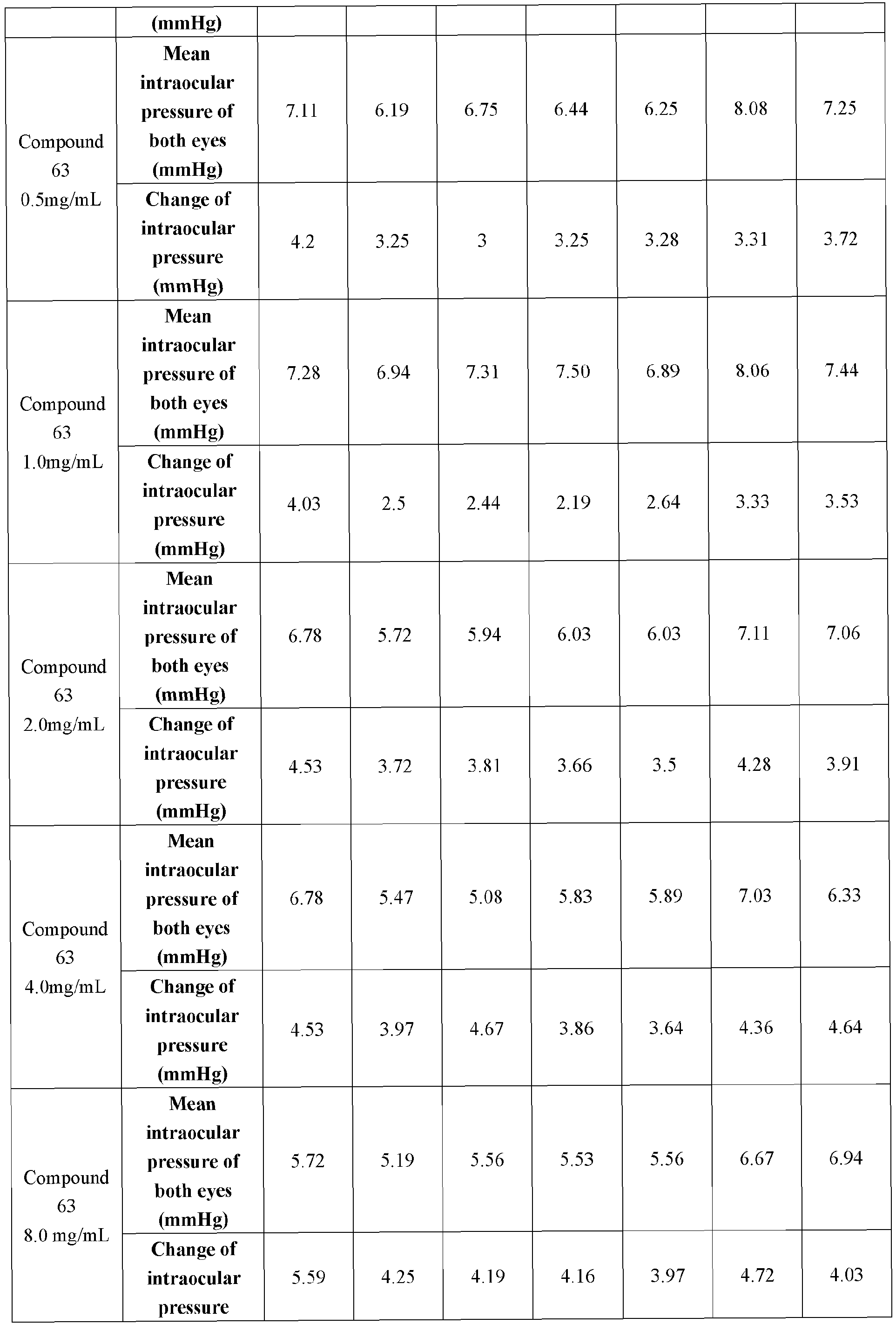

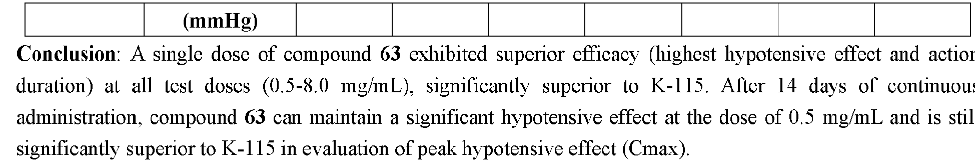

### Experiment operation 2:

42 male New Zealand rabbits were randomly divided into 7 groups, with 6 rabbits per group. Animals were randomly divided according to body weight. Animals in 1-7 groups were eye-dropped with normal saline in the left eye and with solvent/control sample/test sample in the right eye respectively by a volume of 50 µL/eye, once per day for 14 consecutive days, and the day of administration was recorded as Day 1. The intraocular pressure of the animals was measured before Day 1 administration and in 1, 2, 4, 6, 8 and 10 hours after Day 1 administration, respectively (Table 8).

Before the experiment (Day 2/Day 1), before daily administration during the administration (Days 1-14), and in 1, 2, 4, 24, 48 and 72 hours after the last administration (Day 14), the eyes of animals were examined for ocular stimulation response and fluorescein sodium by a hand-held slit lamp (scoring by referring to the scoring criteria).

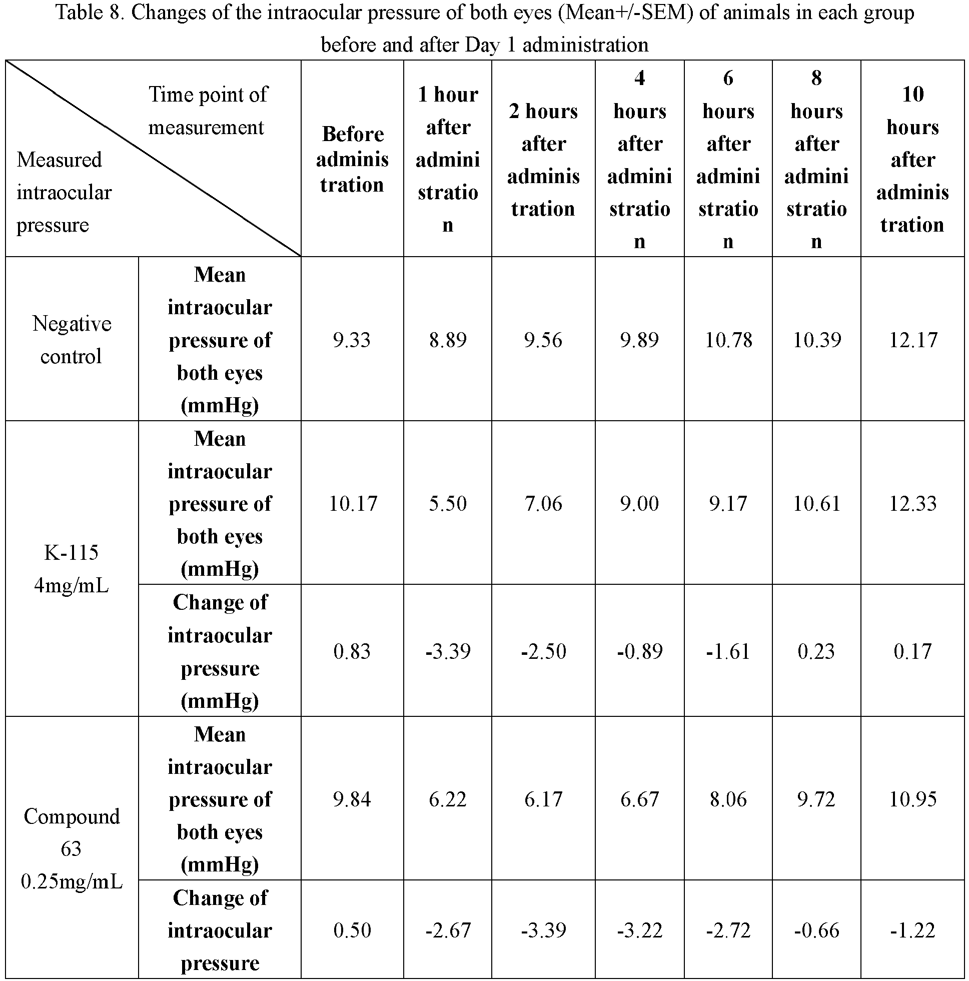

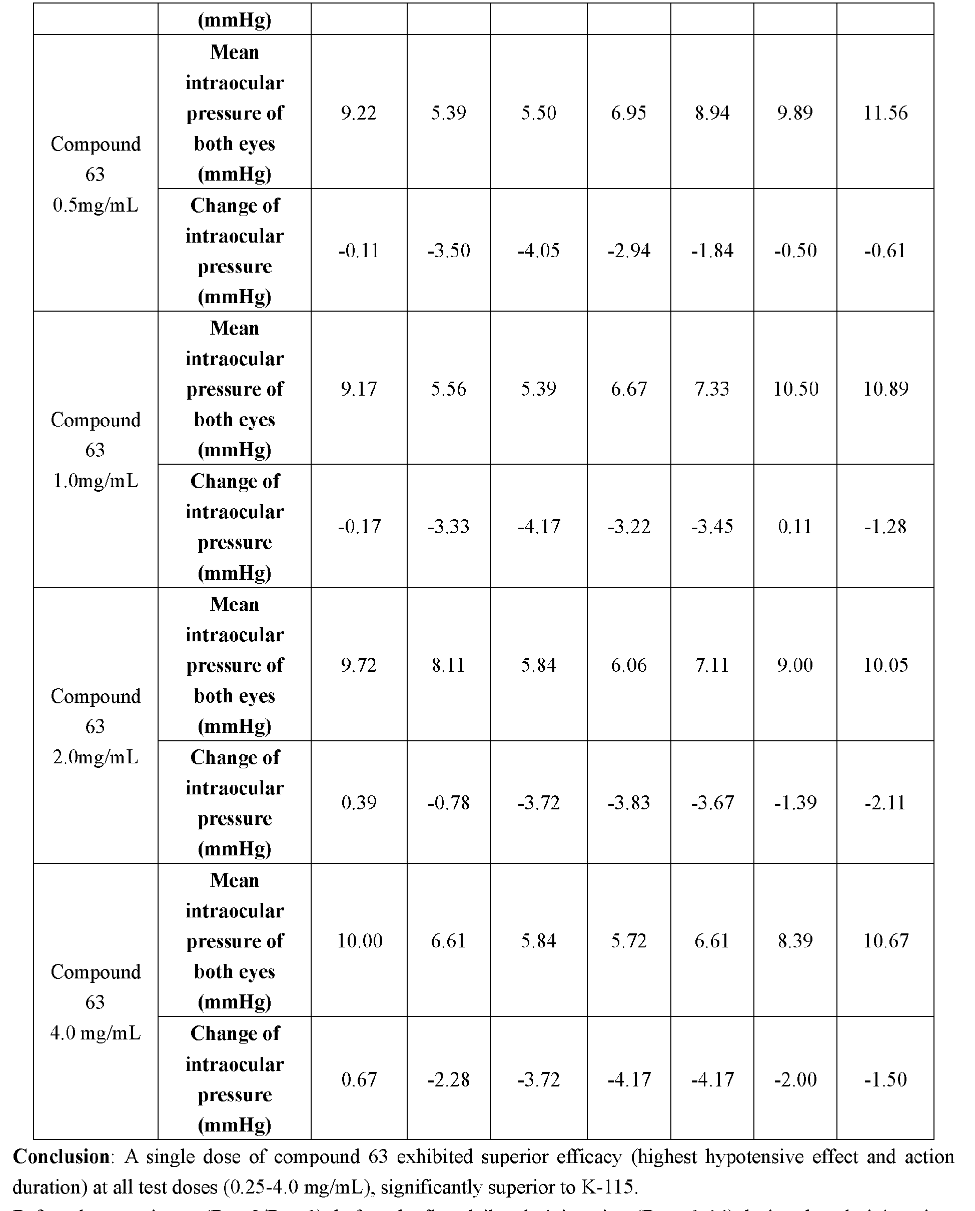

Before the experiment (Day 2/Day 1), before the first daily administration (Days 1-14) during the administration, and in 1, 2, 4, 24, 48 and 72 hours after the last administration, the eyes of animals were examined for ocular stimulation response by a hand-held slit lamp. The scoring criteria were as follows.

| **Ocular stimulation response** | Score |
|---|---|
| **Cornea** | |
| No turbidity | 0 |
| Scattered or diffuse turbidity, clear and visible iris | 1 |
| Semi-transparent region easy to be recognized, obscure iris | 2 |
| Gray semi-transparent region, details of the iris being obscure, marginally visible pupils | 3 |
| Opaque cornea, iris beyond recognition | 4 |

| **Iris** | |
|---|---|
| Normal | 0 |
| Remarkably deepened, congested and swollen crease, slight congestion around cornea, pupils still responsive to light | 1 |
| Bleeding/visible necrosis/not responsive to light (or at least one of them) | 2 |

| **Conjunctiva** | |
|---|---|
| **Congestion (palpebral conjunctiva and bulbar conjunctiva)** | |

| Normal blood vessels | 0 |
|---|---|
| Congested and bright red blood vessels | 1 |
| Congested and dark red blood vessels, beyond recognition | 2 |
| Diffuse congestion and being magenta | 3 |

| **Edema** | |
|---|---|
| No edema | 0 |
| Slight edema (eyelid included) | 1 |
| Obvious edema accompanied by partial eyelid ectropion | 2 |
| Edema to near eyelid semi-closure | 3 |
| Edema to beyond eyelid semi-closure | 4 |

| **Secretion** | |
|---|---|
| No secretion | 0 |
| A small amount of secretion | 1 |
| Secretion making eyelids and eyelashes moist or adhered | 2 |
| Secretion making the entire eye region moist or adhered | 3 |
| **Maximum total score** | **16** |

| | |
|---|---|
| **Evaluation of ocular stimulation response:** adding the maximum score of cornea, iris, conjunctiva, edema, and secretion to obtain the total score of ocular stimulation symptoms for each animal eye at each time point. For the score of ocular stimulation symptom, calculate the integral mean value of each group of animals at each observation time point, and determine the degree of ocular stimulation of each group of animals at each time point according to the following table. | |

### Evaluation standard of ocular stimulation

| **Score** | **Evaluation** |
|---|---|
| 0-3 | No stimulation |
| 4-8 | Slight stimulation |
| 9-12 | Mild stimulation |
| 13-16 | Severe stimulation |

| | |
|---|---|
| **Fluorescein sodium examination:** after each ocular stimulation response examination, a hand-held slit lamp was used for fluorescein sodium examination, and the scoring criteria were as follows: | |

| **Fluorescent staining** | |
|---|---|
| Fluorescein was used to aid the diagnosis of corneal epithelial injury. Fluorescent staining areas can be divided into grades 0-4 according to the degree of corneal opacity: | |
| No fluorescent staining. | 0 |
| Local small-area slight fluorescent staining. Observed under diffuse light, clear and visible eye structures under cornea (clear pupil edge, observation not affected by fluorescent staining). | 1 |
| Local small-area mild fluorescent staining Observed under diffuse light, although some details of the eye under the cornea missed, structure being clear and visible. | 2 |
| Large obvious fluorescent staining in the staning area Observed under diffuse light, eye structures under cornea marginally recognizable. | 3 |
| Serious fluorescent staining Observed under diffuse light, eye structures under cornea beyond recognition. | 4 |

### The results of the experiment were as follows:

According to the evaluation criteria of ocular stimulation, the total score of ocular stimulation response of each group at each time point was less than 3, and all were classified as no stimulation according to the standard.

During the experiment, the scores of fluorescein sodium examination on eyes (with left eyes treated with normal saline, and right eyes treated with the solvent, K-115 and compound 63) were all lower than 1. Corneal fluorescence staining score of 1 was observed in each group of animals at each treatment and individual time point, and biological staining was considered. There was no corneal epithelial injury in each group at each time point. Conclusion: Under the conditions of this experiment, K-115 was eye-dropped at the concentration of 4 mg/mL for 14 consecutive days by 50 µL/eye/day, showing no stimulation. Compound 63 was eye-dropped in the concentration range of 0.25-4 mg/mL for 14 consecutive days by 50 µL/eye/day, showing no stimulation.

### Experimental example 5. Toxicokinetics experiment

### Experiment objective:

The production rate of active drug ingredients and the exposure amount of active drug ingredients of the compound in plasma were detected after 14 days of continuous administration.

### Experiment materials:

Male New Zealand rabbits, aged 3-6 months, weighted 2.0-5.0 kg, purchased from PizhouDongfang Breeding Co.Ltd.

### Experiment operations:

After 14 days of continuous administration, blood samples from the group administrated with compound 63 (8.0 mg/mL) were collected at 0 hour (before administration) and in 0.5, 1, 2, 4, 8, and 24 hours after administration on Days 14-15. About 0.8 mL of whole blood was collected from the auricular artery or the saphenous vein of the hind limb (or other appropriate sites) of toxicokinetics experiment animals, and was placed in labeled collection tubes using ethylenediaminetetraacetic acid dipotassium (K₂EDTA) as an anticoagulant. Plasma was obtained by centrifugation for 10 minutes at 3000 rpm and under the condition of 2 °C to 8 °C in 60 minutes after blood collection. All samples were quantitatively detected for the content of the administered compounds in plasma of the experimental animals by liquid chromatography-coupled mass spectrometry (LC-MS) and mass spectrometry (MS) technology.

**Table 9. Test results of active compounds in plasma of New Zealand rabbits after 14 days of continuous administration**

| **Compound No.** | **Time (hour)** | **Concentration (ng/mL)** | |
|---|---|---|---|
| | | **Group 1** | **Group 2** |
| | | | |
| | 0.00 | BQL | BQL |
| | 0.50 | 3.63 | 3.44 |
| Compound 63 | 1.00 | 2.87 | 2.20 |
| | 2.00 | 1.80 | 1.66 |
| | 4.00 | 0.934 | 0.818 |
| | 8.00 | BQL | BQL |
| | 24.0 | BQL | BQL |

| | | | |
|---|---|---|---|
| Note: BQL indicates below the detection limit. **Conclusion:** The metabolite concentration of compound **63** was 0.934 ng/mL in 4 hours after high administration dose of 8 mg/mL; in 8 hours after administration, the concentration of metabolite of the compound was below the detection limit, indicating high system safety. | | | |

## Claims

1. A compound of formula (I), or a pharmaceutically acceptable salt thereof, 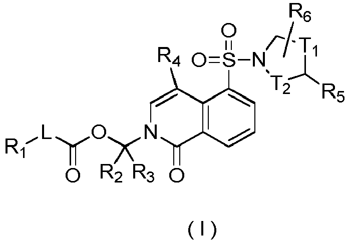 wherein,
the structure unit 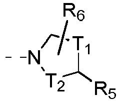 is selected from a group consisting of 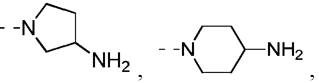 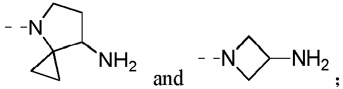
R₁ is selected from a group consisting of C₁₋₁₆ alkyl, phenyl, C₃₋₇ cycloalkyl, 3-8 membered heterocycloalkyl and 5-10 membered heteroaryl, each of C₁₋₁₆ alkyl, phenyl, C₃₋₇ cycloalkyl, 3-8 membered heterocycloalkyl and 5-10 membered heteroaryl is optionally substituted by 1, 2 or 3 Rₐ;
each of R₂ and R₃ is independently selected from a group consisting of H, F, Cl, Br, I, OH, NH₂, CN and C₁₋₃ alkyl;
R₄ is selected from a group consisting of H, F, Cl, Br, I, OH, NH₂, CN, and C₁₋₃ alkyl which is optionally substituted by 1, 2 or 3 R_{b};
L is selected from a group consisting of a single bond, -O- and -NR₁₁-;
R₁₁ is selected from a group consisting of H and C₁₋₃ alkyl;
Rₐ is selected from a group consisting of F, Cl, Br, I, OH, NH₂, CN, C₁₋₃ alkyl and C₁₋₃ alkoxy, wherein each of C₁₋₃ alkyl and C₁₋₃ alkoxy is optionally substituted by 1, 2 or 3 R;
each of R_{b}, and Rₑ is independently selected from a group consisting of H, F, Cl, Br, I, OH, NH₂, CN and C₁₋₃ alkyl;
R is selected from a group consisting of F, Cl, Br, I, OH, NH₂, CN and CH₃;
wherein, each of the 3-8 membered heterocycloalkyl and 5-10 membered heteroaryl is independently include 1, 2, 3 or 4 heteroatom(s) or heteroatom group(s) which is/are independently selected from a group consisting of -NH-, -O-, -S- and N.

2. The compound, or the pharmaceutically acceptable salt thereof according to claim 1, wherein, Rₐ is selected from a group consisting of F, Cl, Br, I, OH, NH₂, CN, CH₃, CF₃, CH₂F, CHF₂, CH₂CH₃ and OCH₃.

3. The compound, or the pharmaceutically acceptable salt thereof according to claim 1, wherein, R₁ is selected from a group consisting of C₁₋₁₂ alkyl, phenyl, cyclobutyl, cyclopentyl, cyclohexyl, tetrahydrofuranyl, tetrahydropyranyl, piperidinyl, thienyl, furyl, pyrrolyl and benzofuryl, wherein, each of C₁₋₁₂ alkyl, phenyl, cyclobutyl, cyclopentyl, cyclohexyl, tetrahydrofuranyl, tetrahydropyranyl, piperidinyl, thienyl, furyl, pyrrolyl and benzofuryl is optionaly substituted by 1, 2 or 3 Rₐ.

4. The compound, or the pharmaceutically acceptable salt thereof according to claim 3, wherein, R₁ is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, 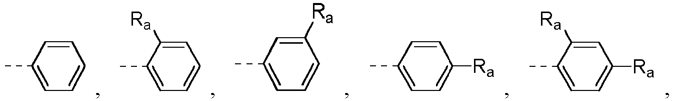 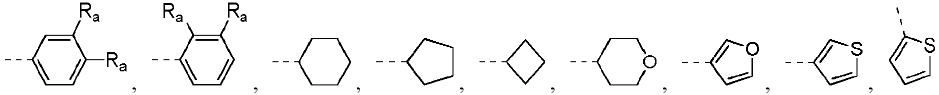 and

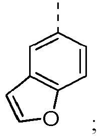

5. The compound, or the pharmaceutically acceptable salt thereof according to claim 3, wherein, R₁ is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃,

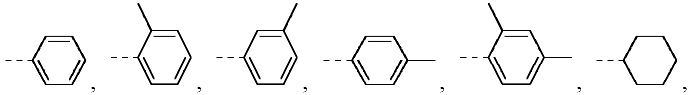

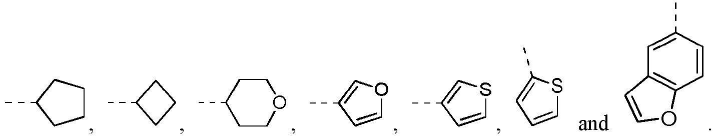

6. The compound, or the pharmaceutically acceptable salt thereof according to claim 1, wherein, each of R₂ and R₃ is independently selected from a group consisting of H, F, Cl, Br, I, OH and NH₂.

7. The compound, or the pharmaceutically acceptable salt thereof according to claim 1, wherein, Rₐ is selected from a group consisting of H, F, Cl, Br, I, OH, NH₂, CN, CH₃ and CH₂CH₃.

8. The compound, or the pharmaceutically acceptable salt thereof according to claim 1, wherein, L is selected from a group consisting of a single bond, -O-, -NH- and -N(CH₃)-.

9. The compound, or the pharmaceutically acceptable salt thereof according to claim 1, wherein, the structure unit 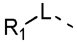 is selected from a group consisting of CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, OCH₃, OCH₂CH₃, O(CH₂)₂CH₃, O(CH₂)₃CH₃, O(CH₂)₄CH₃, O(CH₂)₅CH₃, O(CH₂)₆CH₃, OCH(CH₃)₂, OC(CH₃)₃, N(CH₃)₂,

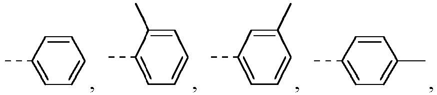

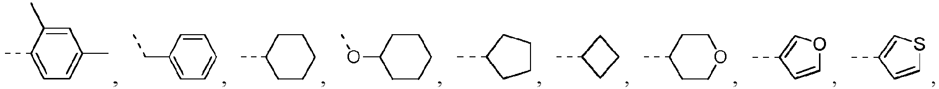

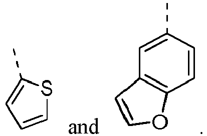

10. The compound, or the pharmaceutically acceptable salt thereof according to claim 1, which is selected from a group consisting of: 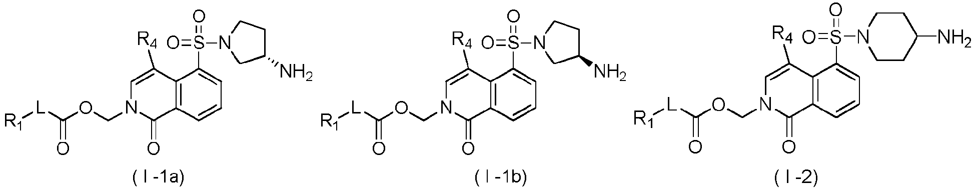 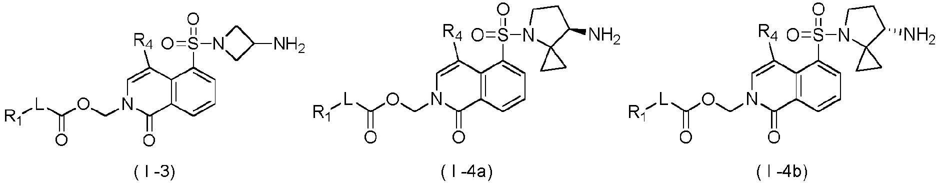 wherein,
R₁ is as defined in claim 1, 3 or 4;
R₄ is as defined in claim 1 or 7; and
L is as defined in claim 1 or 8.

11. A compound of the following formula according to claim 1, or a pharmaceutically acceptable salt thereof,

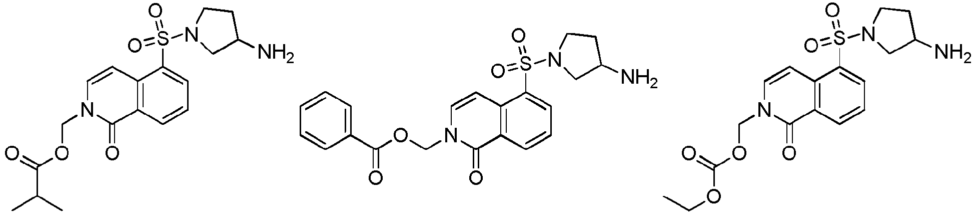

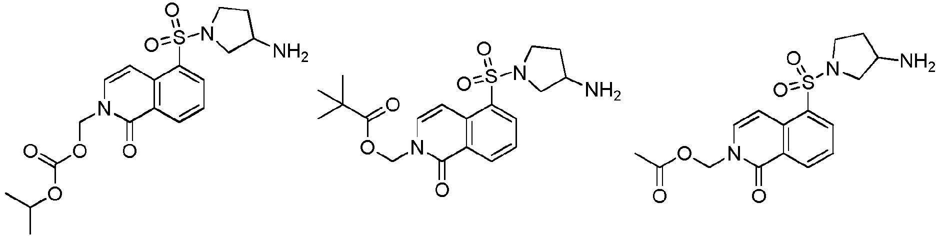

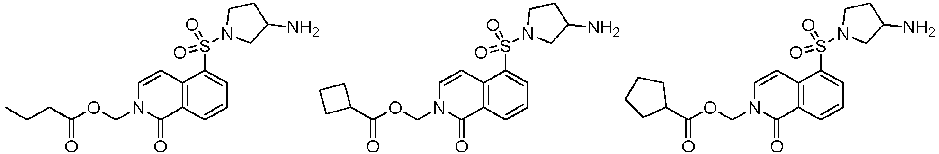

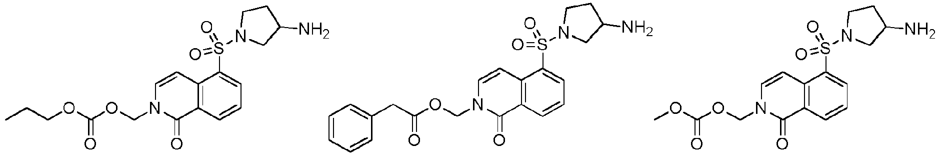

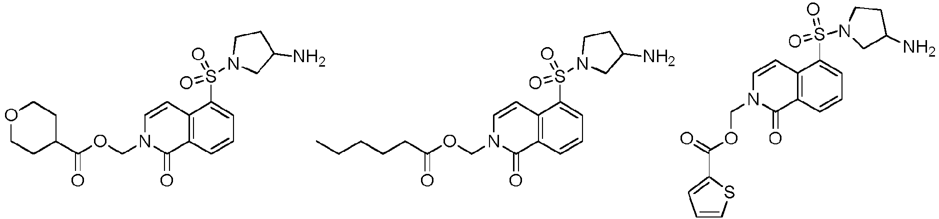

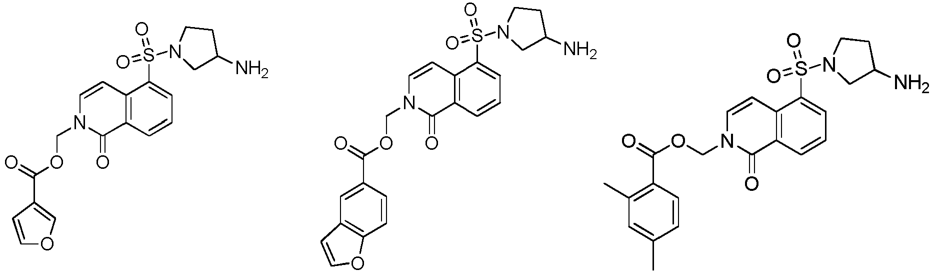

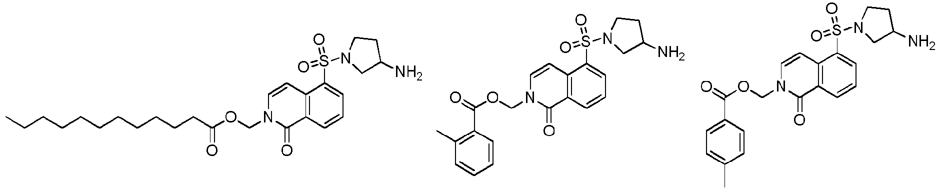

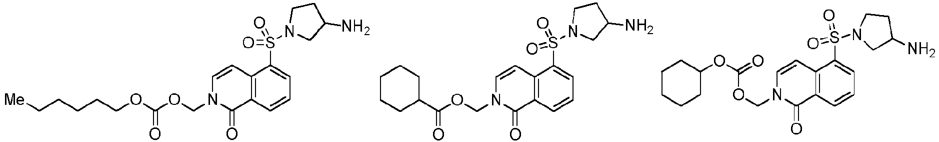

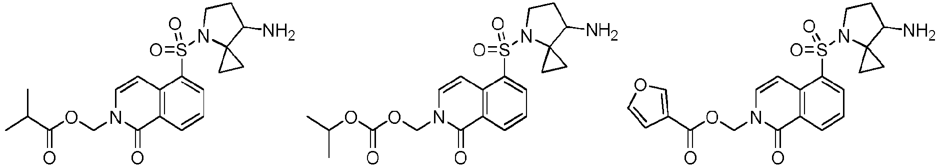

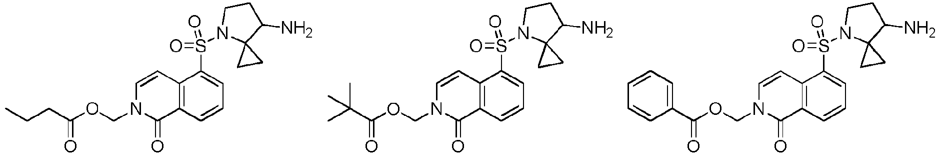

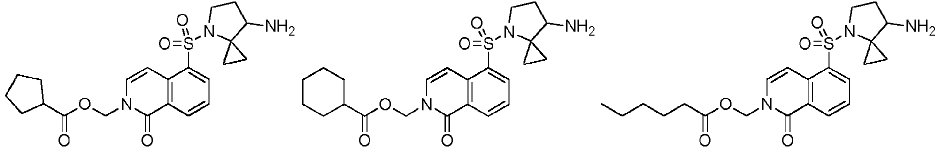

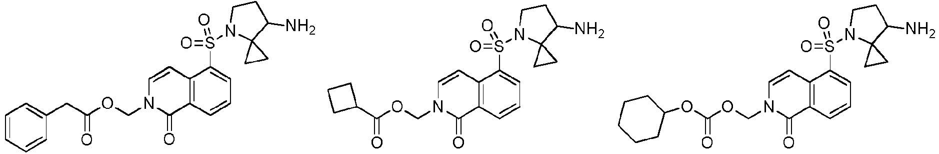

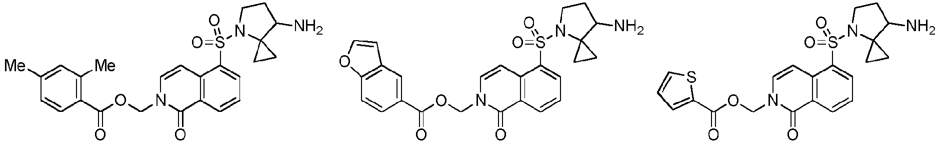

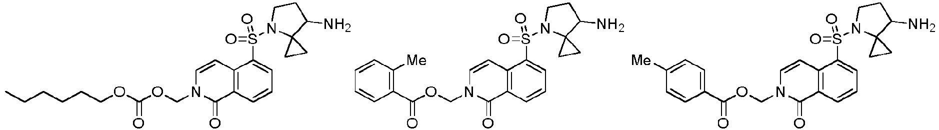

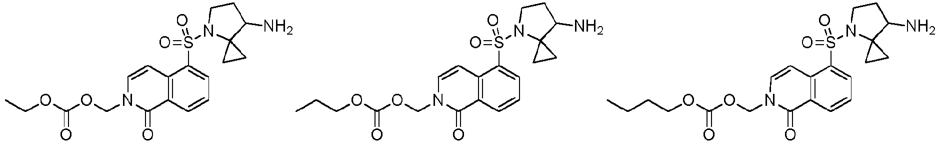

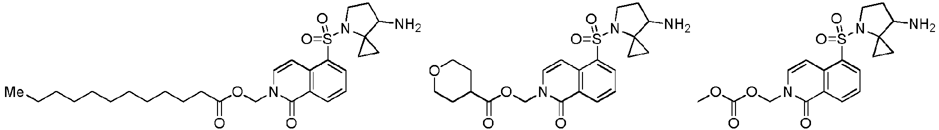

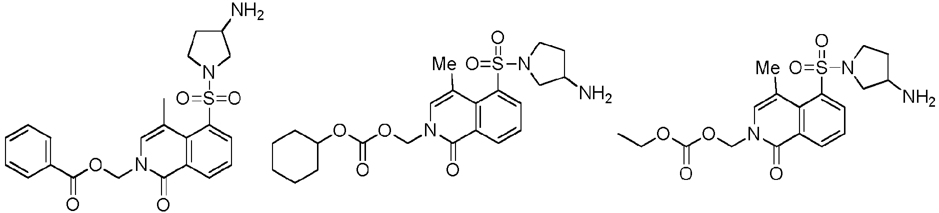

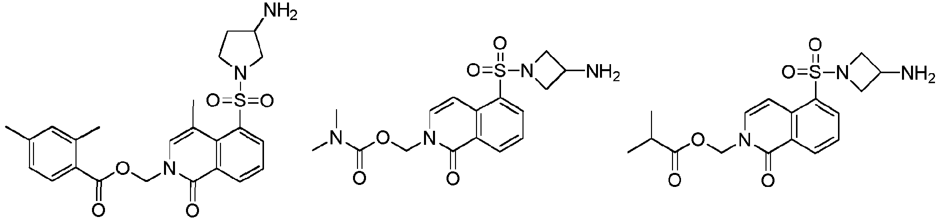

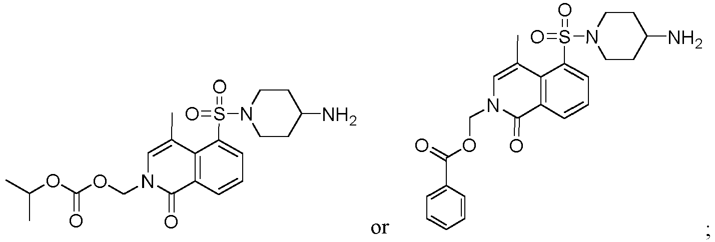

12. A compound of the following formula according to claim 11, or a pharmaceutically acceptable salt thereof:

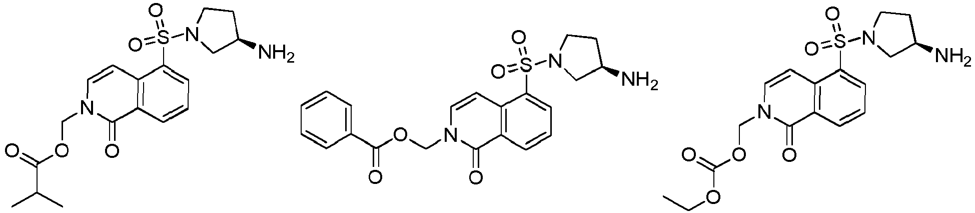

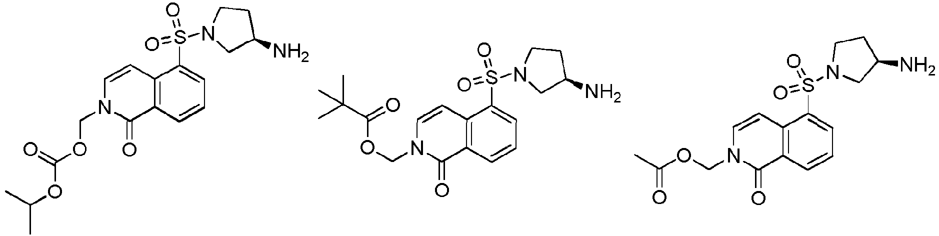

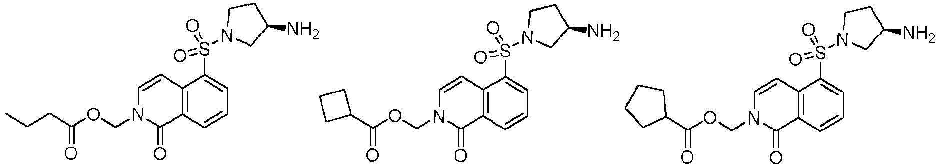

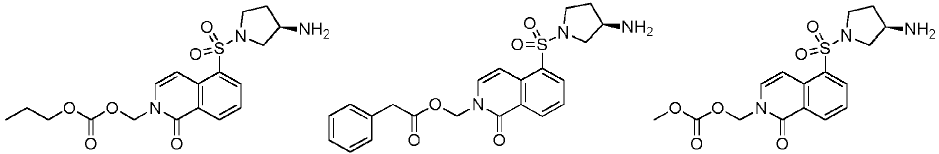

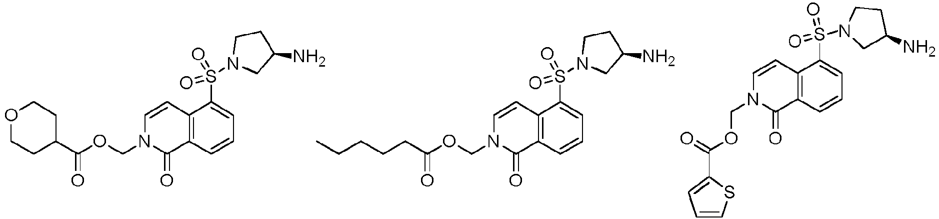

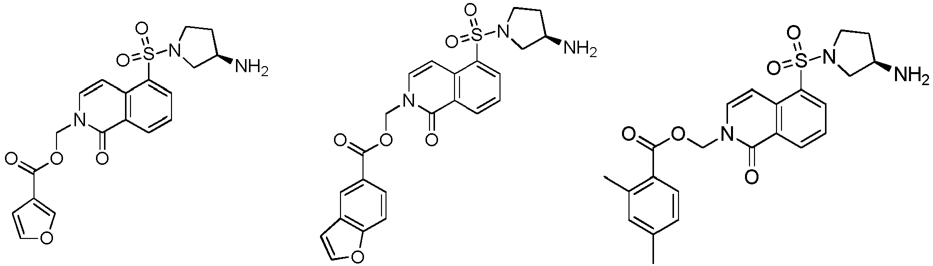

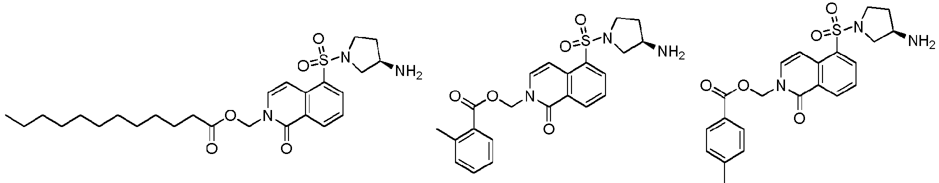

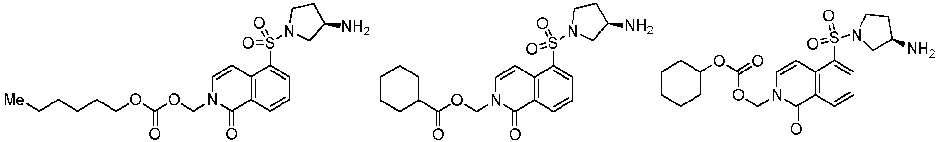

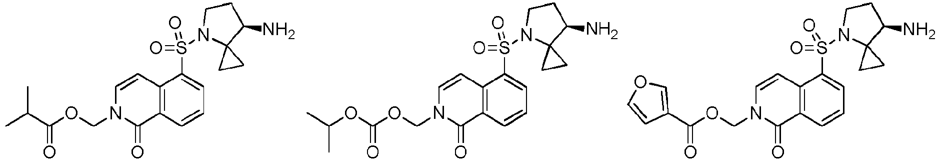

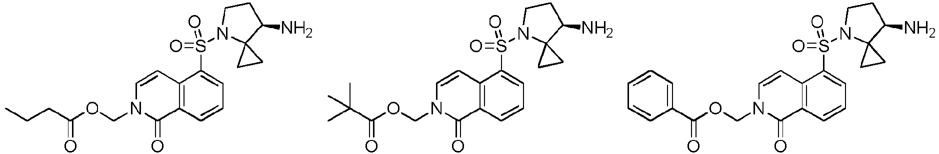

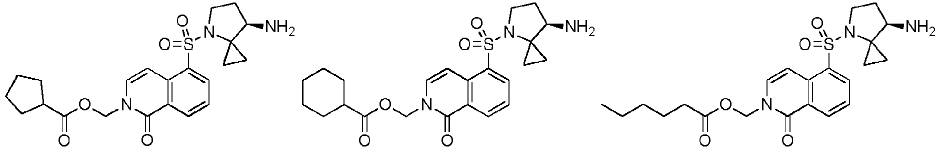

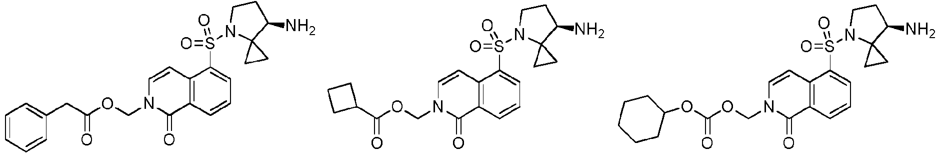

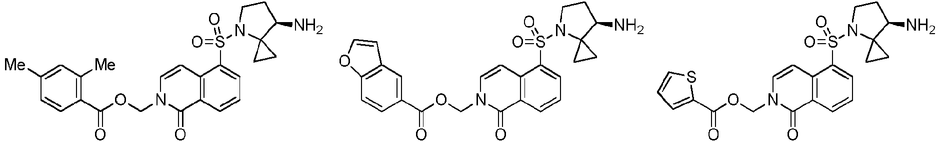

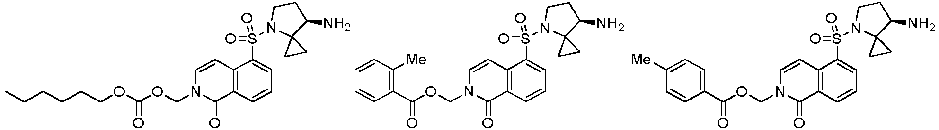

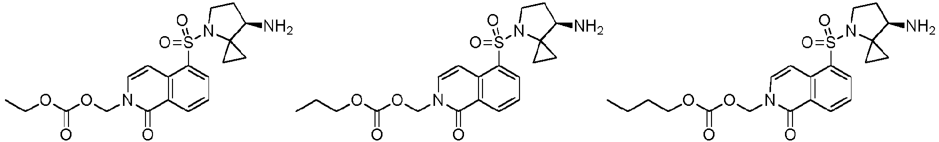

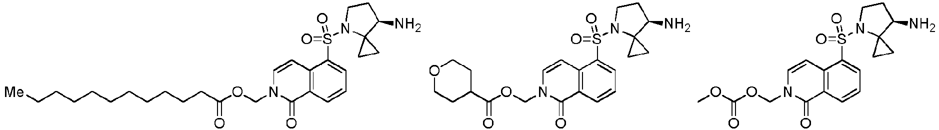

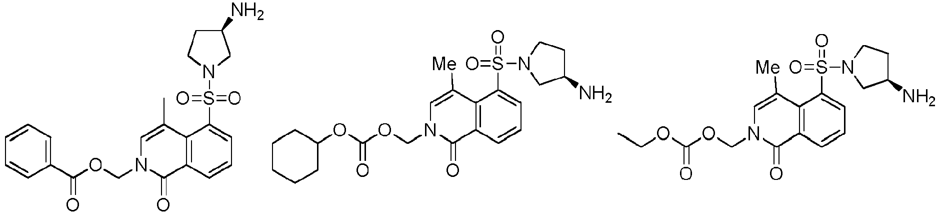

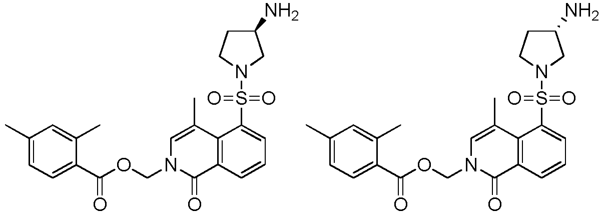

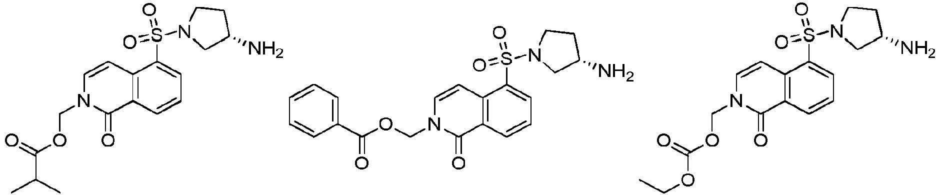

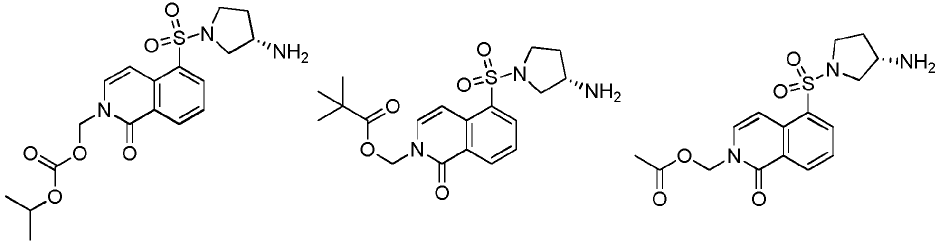

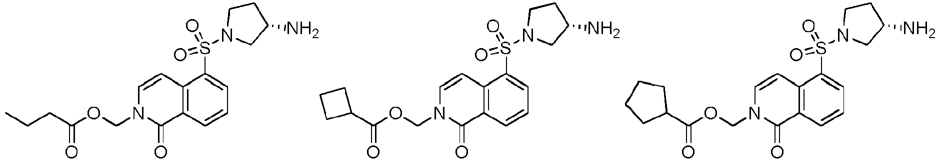

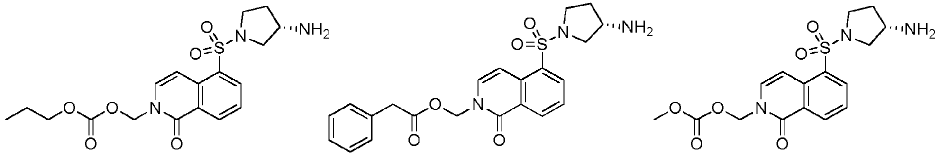

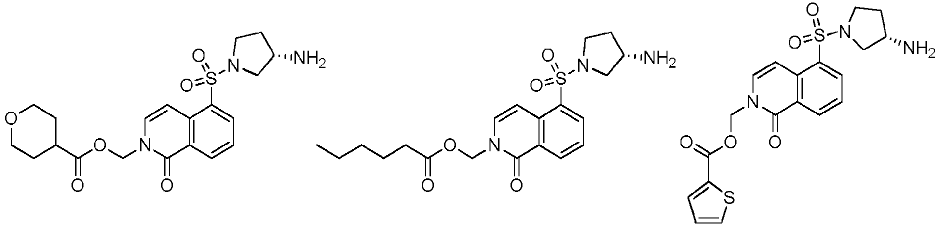

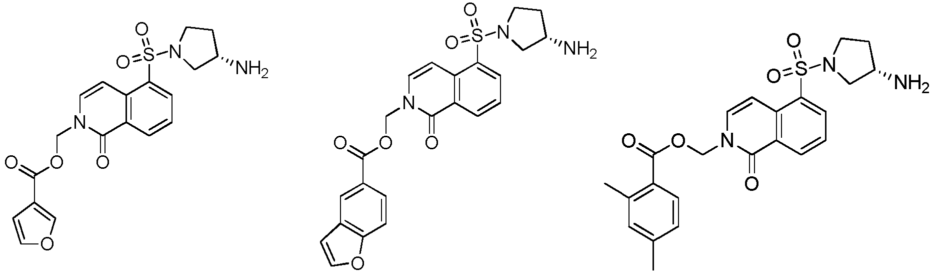

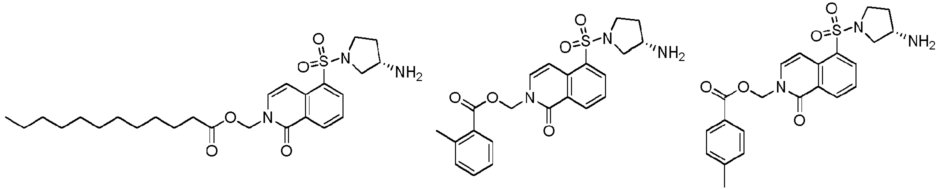

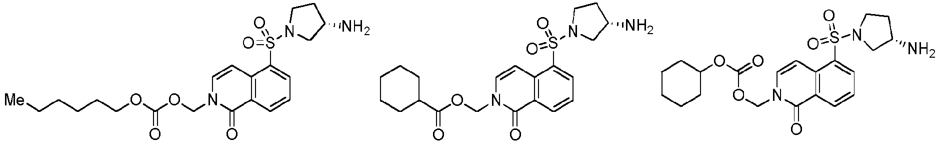

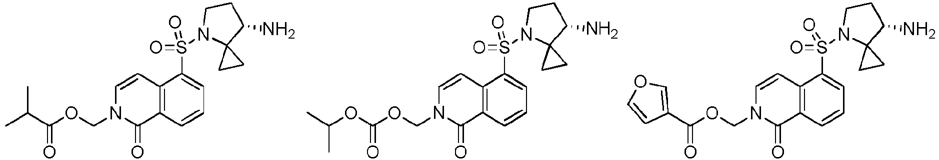

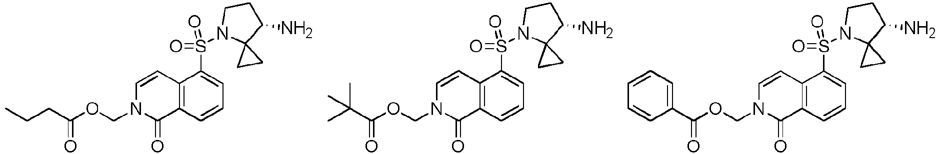

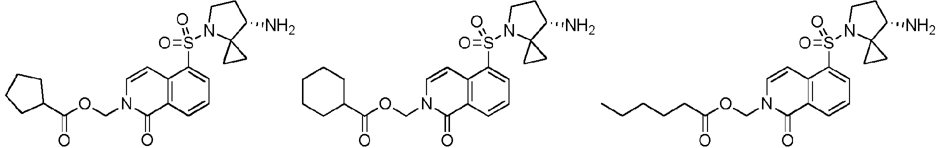

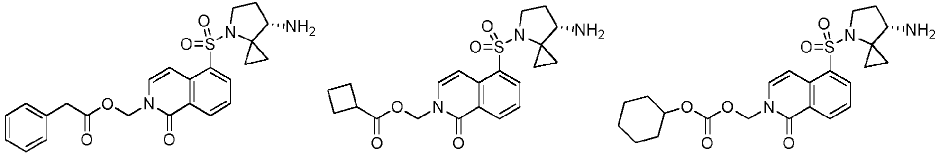

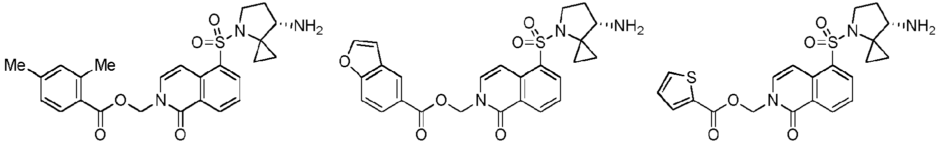

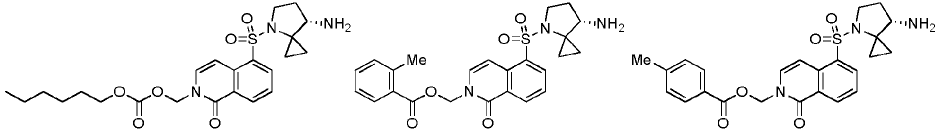

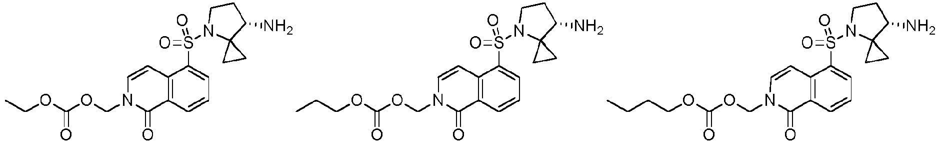

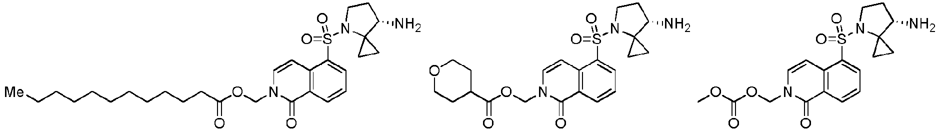

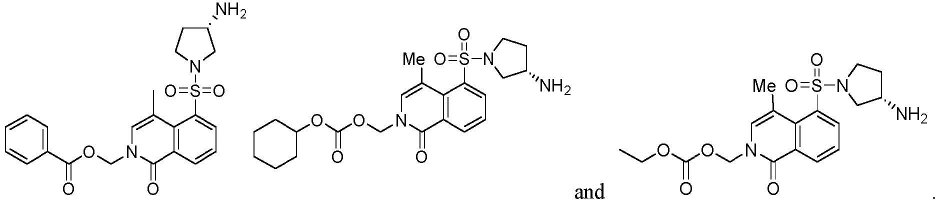

13. A pharmaceutical composition, comprising a therapeutically effective amount of the compound, or the pharmaceutically acceptable salt thereof according to any one of claims 1-12 and a pharmaceutically acceptable carrier.

14. A compound or the pharmaceutically acceptable salt thereof according to any one of claims 1-12 or a composition of claim 13 for use in the treatment of glaucoma or ocular hypertension.

## Patentansprüche

1. Verbindung der Formel (I) oder pharmazeutisch akzeptables Salz davon, 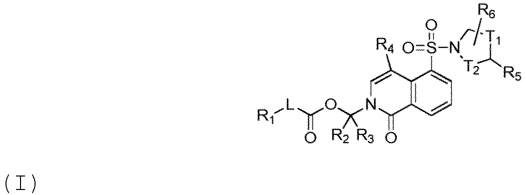 wobei
die Struktureinheit 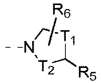 aus einer Gruppe bestehend aus 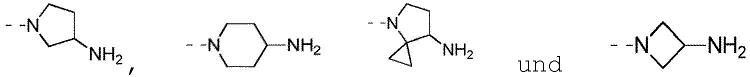 ausgewählt ist;
R₁ aus einer Gruppe bestehend aus C₁₋₁₆-Alkyl, Phenyl, C₃₋₇-Cycloalkyl, 3-8-gliedrigem Heterocycloalkyl und 5-10-gliedrigem Heteroaryl ausgewählt ist, wobei C₁₋₁₆-Alkyl, Phenyl, C₃₋₇-Cycloalkyl, 3-8-gliedrigem Heterocycloalkyl und 5-10-gliedrigem Heteroaryl jeweils gegebenenfalls durch 1, 2 oder 3 Rₐ substituiert sind;
R₂ und R₃ jeweils unabhängig aus einer Gruppe bestehend aus H, F, Cl, Br, I, OH, NH₂, CN und C₁₋₃-Alkyl ausgewählt sind;
R₄ aus einer Gruppe bestehend aus H, F, Cl, Br, I, OH, NH₂, CN und C₁₋₃-Alkyl, das gegebenenfalls durch 1, 2 oder 3 R_{b} substituiert ist, ausgewählt ist;
L aus einer Gruppe bestehend aus einer Einfachbindung, -O- und -NR₁₁- ausgewählt ist;
R₁₁ aus einer Gruppe bestehend aus H und C₁₋₃-Alkyl ausgewählt ist;
Rₐ aus einer Gruppe bestehend aus F, Cl, Br, I, OH, NH₂, CN, C₁₋₃-Alkyl und C₁₋₃-Alkoxy ausgewählt ist, wobei C₁₋₃-Alkyl und C₁₋₃-Alkoxy jeweils gegebenenfalls durch 1, 2 oder 3 R substituiert sind;
R_{b} und Rₑ jeweils unabhängig aus einer Gruppe bestehend aus H, F, Cl, Br, I, OH, NH₂, CN und C₁₋₃-Alkyl ausgewählt sind;
R aus einer Gruppe bestehend aus F, Cl, Br, I, OH, NH₂, CN und CH₃ ausgewählt ist;
wobei das 3-8-gliedrige Heterocycloalkyl und das 5-10-gliedrige Heteroaryl jeweils unabhängig 1, 2, 3 oder 4 Heteroatom(e) oder Heteroatomgruppe(n) enthalten, die unabhängig aus einer Gruppe bestehend aus -NH-, -O-, - S- und N ausgewählt ist/sind.

2. Verbindung oder pharmazeutisch akzeptables Salz davon nach Anspruch 1, wobei Rₐ aus einer Gruppe bestehend aus F, Cl, Br, I, OH, NH₂, CN, CH₃, CF₃, CH₂F, CHF₂, CH₂CH₃ und OCH₃ ausgewählt ist.

3. Verbindung oder pharmazeutisch akzeptables Salz davon nach Anspruch 1, wobei R₁ aus einer Gruppe bestehend aus C₁₋₁₂-Alkyl, Phenyl, Cyclobutyl, Cyclopentyl, Cyclohexyl, Tetrahydrofuranyl, Tetrahydropyranyl, Piperidinyl, Thienyl, Furyl, Pyrrolyl und Benzofuryl ausgewählt ist, wobei C₁₋₁₂-Alkyl, Phenyl, Cyclobutyl, Cyclopentyl, Cyclohexyl, Tetrahydrofuranyl, Tetrahydropyranyl, Piperidinyl, Thienyl, Furyl, Pyrrolyl und Benzofuryl jeweils gegebenenfalls durch 1, 2 oder 3 Rₐ substituiert sind.

4. Verbindung oder pharmazeutisch akzeptables Salz davon nach Anspruch 3, wobei R₁ aus einer Gruppe bestehend aus CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃ 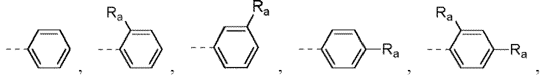 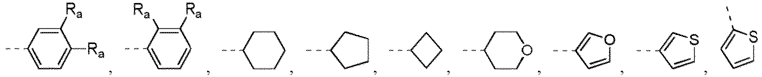 und 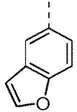 ausgewählt ist.

5. Verbindung oder pharmazeutisch akzeptables Salz davon nach Anspruch 3, wobei R₁ aus einer Gruppe bestehend aus CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)_{3,} 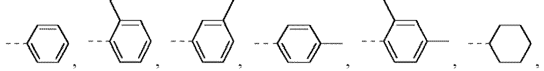 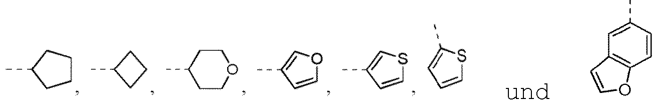 ausgewählt ist.

6. Verbindung oder pharmazeutisch akzeptables Salz davon nach Anspruch 1, wobei R₂ und R₃ jeweils unabhängig aus einer Gruppe bestehend aus H, F, Cl, Br, I, OH und NH₂ ausgewählt sind.

7. Verbindung oder pharmazeutisch akzeptables Salz davon nach Anspruch 1, wobei R₄ aus einer Gruppe bestehend aus H, F, Cl, Br, I, OH, NH₂, CN, CH₃ und CH₂CH₃ ausgewählt ist.

8. Verbindung oder pharmazeutisch akzeptables Salz davon nach Anspruch 1, wobei L aus einer Gruppe bestehend aus einer Einfachbindung, -O-, -NH- und - N(CH₃)- ausgewählt ist.

9. Verbindung oder pharmazeutisch akzeptables Salz davon nach Anspruch 1, wobei die Struktureinheit 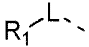 aus einer Gruppe bestehend aus CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, OCH₃, OCH₂CH₃, O(CH₂)₂CH₃, O(CH₂)₃CH₃, O(CH₂)₄CH₃, O(CH₂)₅CH₃, O(CH₂)₆CH₃, OCH(CH₃)₂, OC(CH₃)₃, N(CH₃)₂, 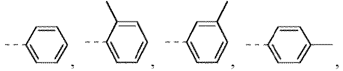 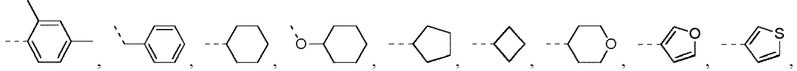 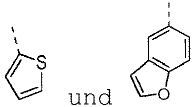 ausgewählt ist.

10. Verbindung oder pharmazeutisch akzeptables Salz davon nach Anspruch 1, die aus einer Gruppe ausgewählt ist, die aus Folgendem besteht: 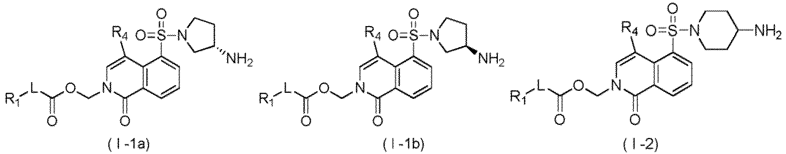 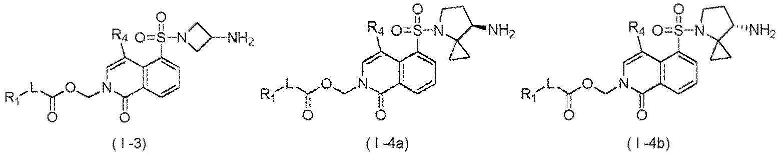 wobei
R₁ wie in Anspruch 1, 3 oder 4 definiert ist;
R₄ wie in Anspruch 1 oder 7 definiert ist; und
L wie in Anspruch 1 oder 8 definiert ist.

11. Verbindung der folgenden Formel nach Anspruch 1 oder pharmazeutisch akzeptables Salz davon,

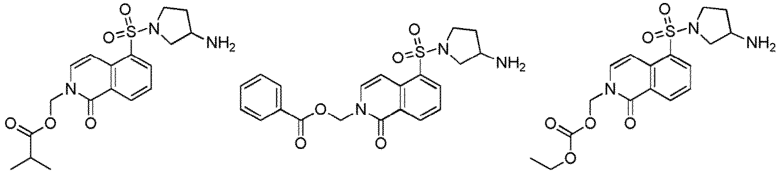

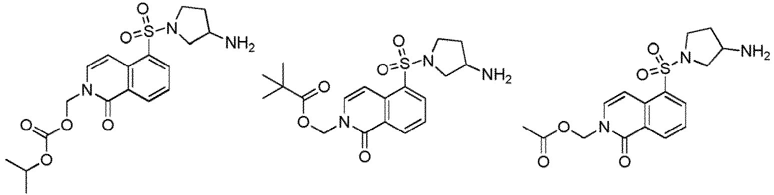

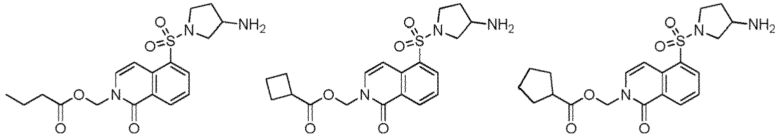

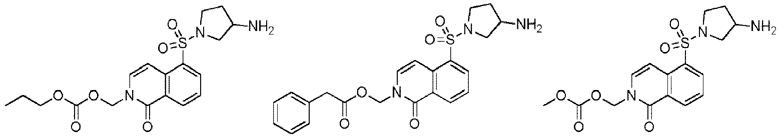

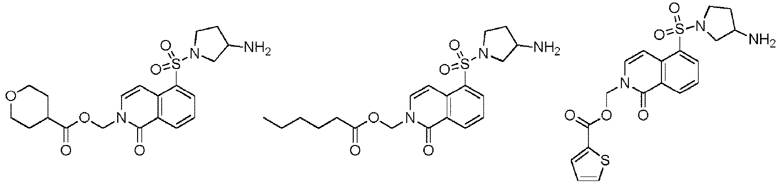

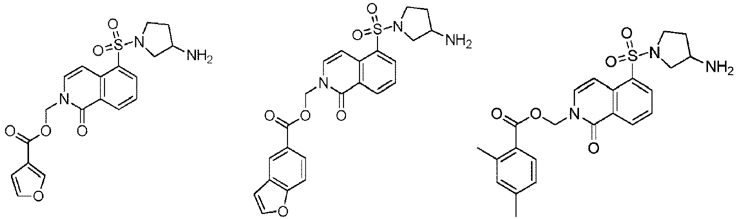

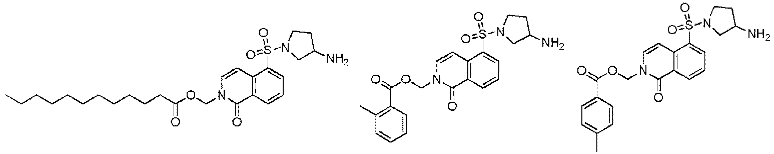

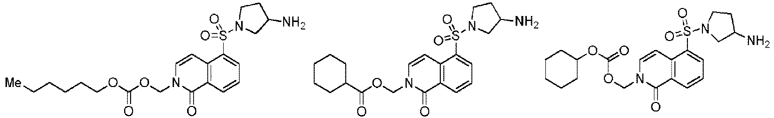

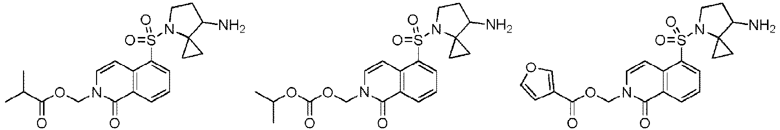

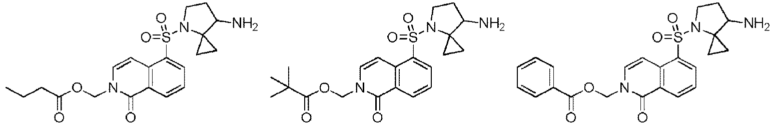

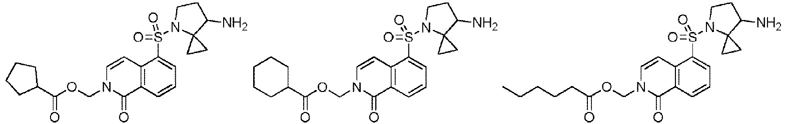

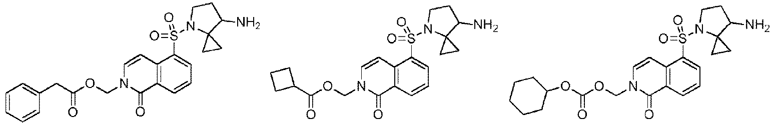

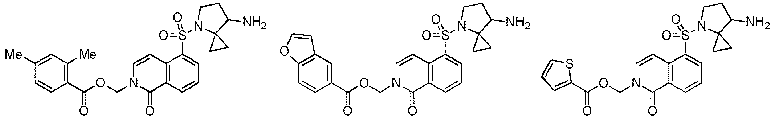

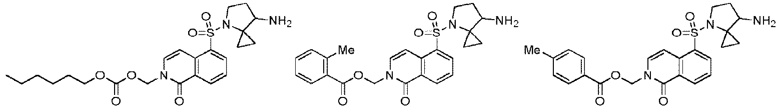

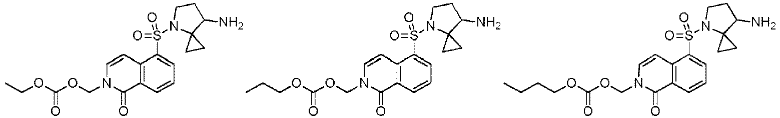

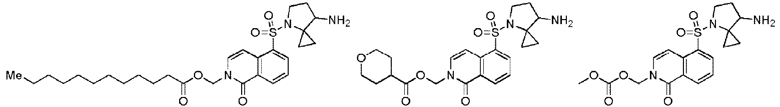

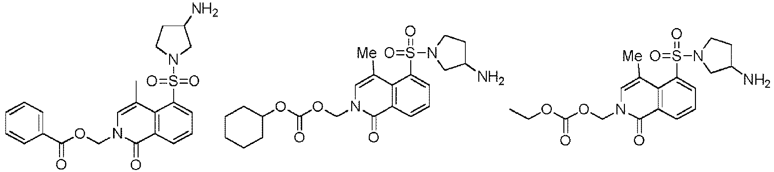

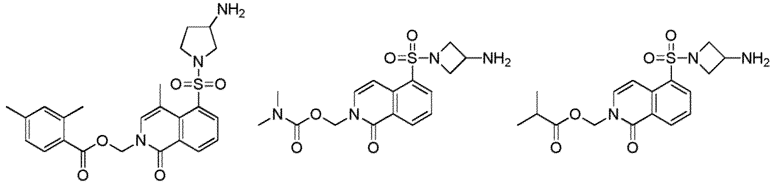

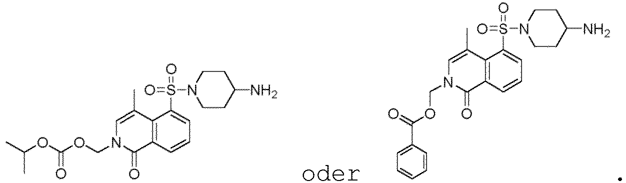

12. Verbindung der folgenden Formel nach Anspruch 11 oder pharmazeutisch akzeptables Salz davon: 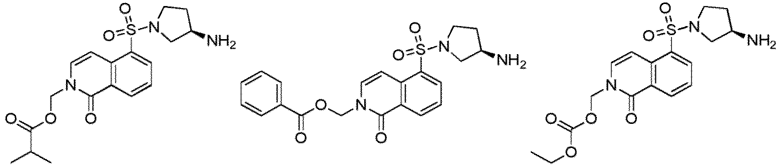 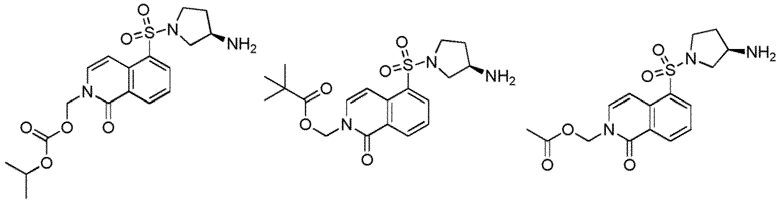 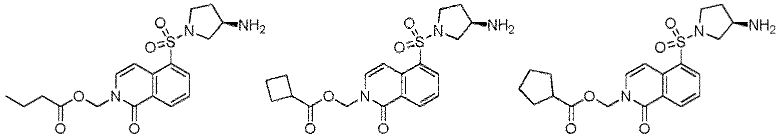 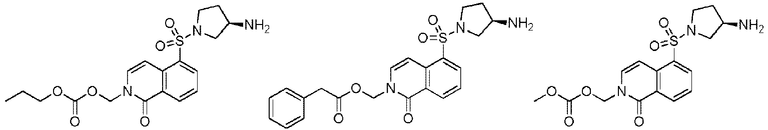 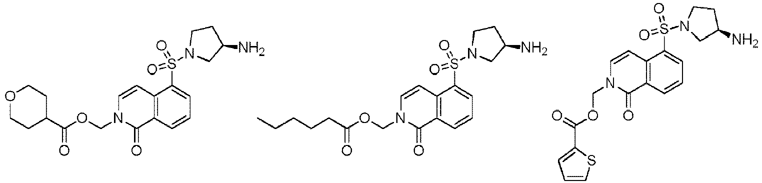 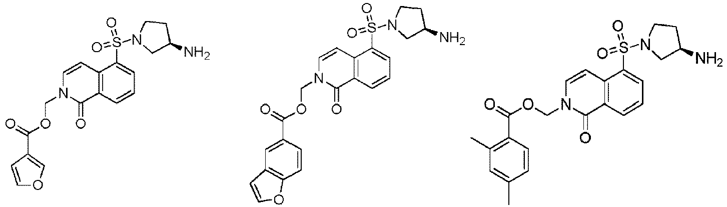 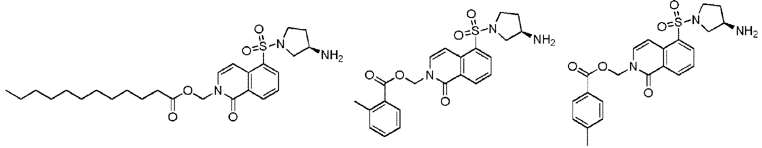 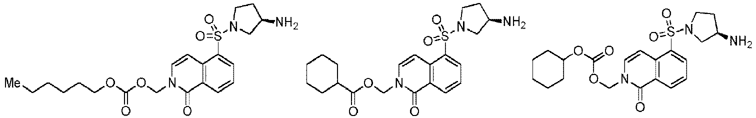 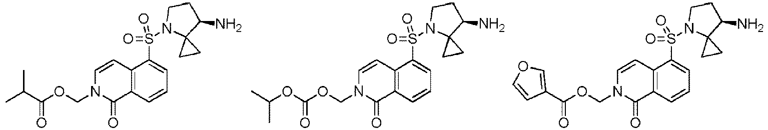 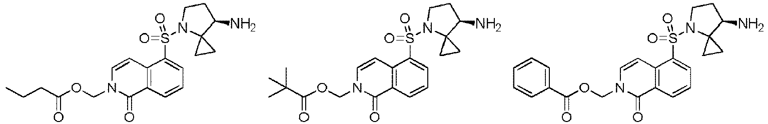 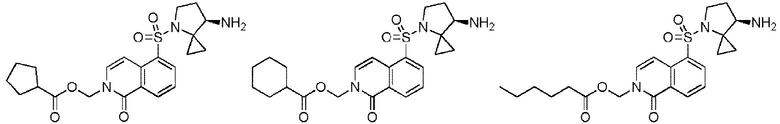 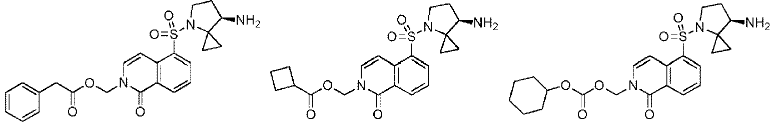 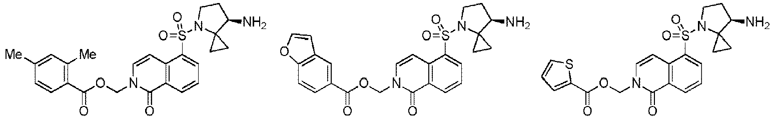 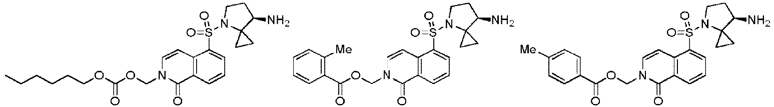 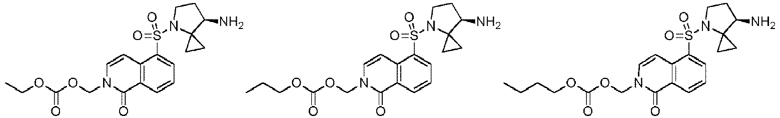 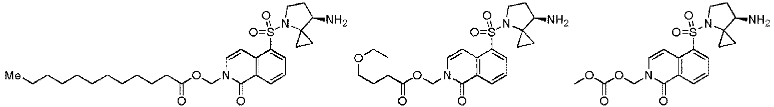 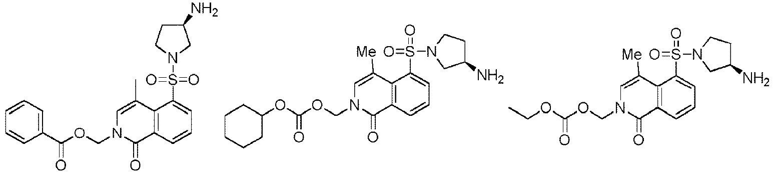 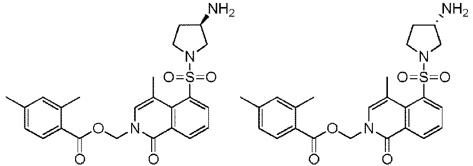 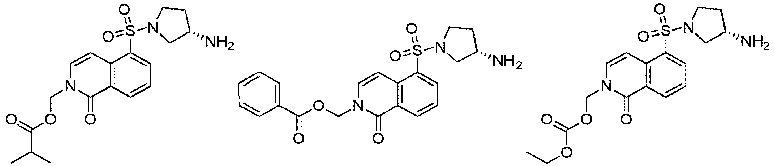 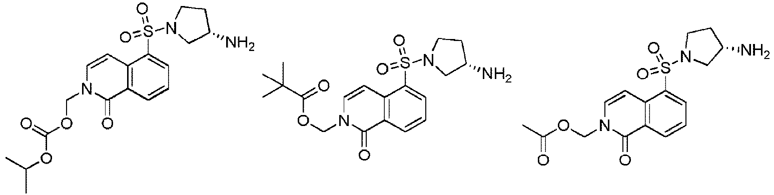 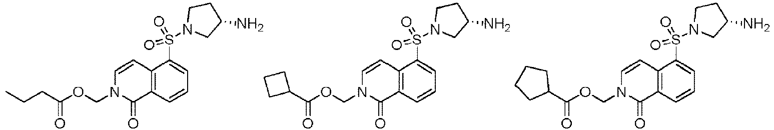 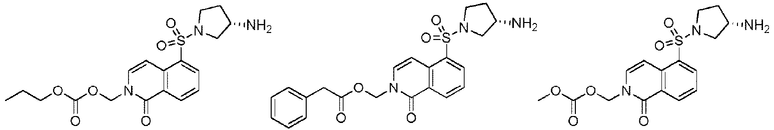 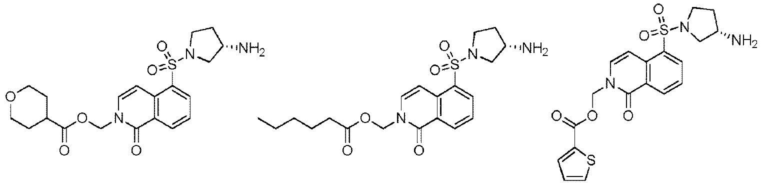 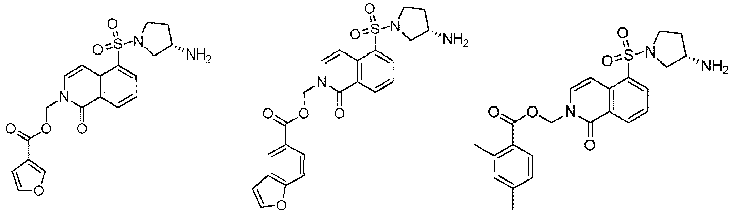 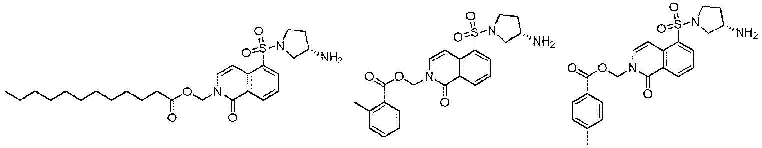 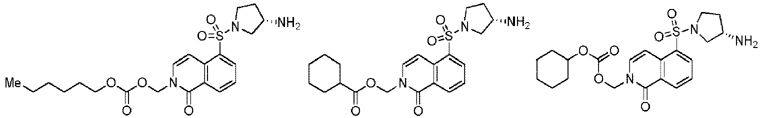 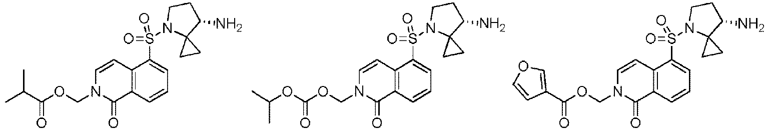 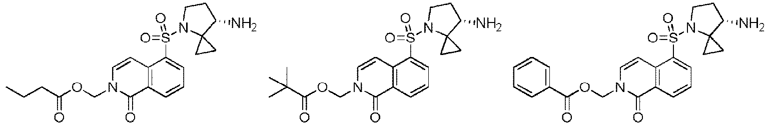 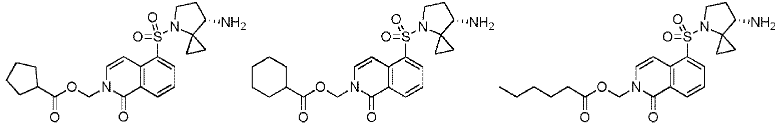 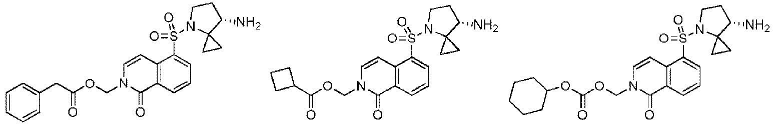 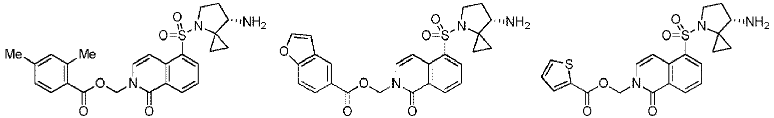 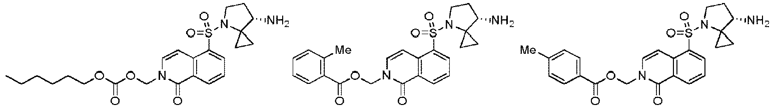 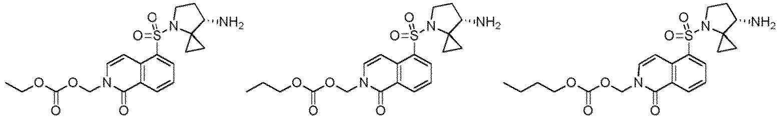 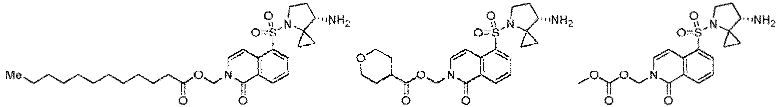 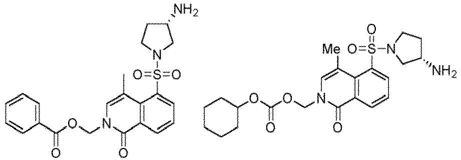 und

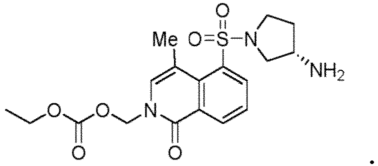

13. Pharmazeutische Zusammensetzung, umfassend eine therapeutisch wirksame Menge der Verbindung oder des pharmazeutisch unbedenklichen Salzes davon nach einem der Ansprüche 1-12 und einen pharmazeutisch unbedenklichen Träger.

14. Verbindung oder pharmazeutisch akzeptables Salz davon nach einem der Ansprüche 1-12 oder Zusammensetzung nach Anspruch 13 zur Verwendung bei der Behandlung von Glaukom oder okulärer Hypertonie.

## Revendications

1. Composé de formule (I), ou sel pharmaceutiquement acceptable correspondant, 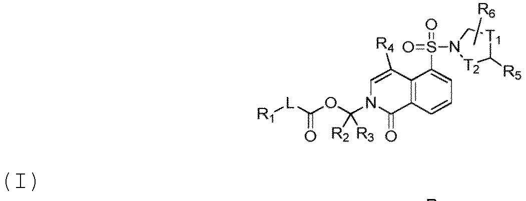
le motif de structure 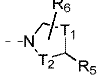 étant choisi dans un groupe constitué par 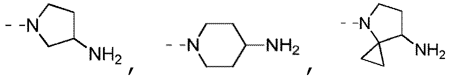 et 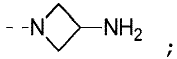
R₁ étant choisi dans un groupe constitué par C₁₋₁₆ alkyle, phényle, C₃₋₇ cycloalkyle, hétérocycloalkyle à 3 à 8 chaînons et hétéroaryle à 5 à 10 chaînons, chacun parmi C₁₋₁₆ alkyle, phényle, C₃₋₇ cycloalkyle, hétérocycloalkyle à 3 à 8 chaînons et hétéroaryle à 5 à 10 chaînons étant éventuellement substitué par 1, 2 ou 3 Rₐ ;
chacun parmi R₂ et R₃ étant indépendamment choisi dans un groupe constitué par H, F, Cl, Br, I, OH, NH₂, CN et C₁₋₃ alkyle ;
R₄ étant choisi dans un groupe constitué par H, F, Cl, Br, I, OH, NH₂, CN, et C₁₋₃ alkyle qui est éventuellement substitué par 1, 2 ou 3 R_{b} ;
L étant choisi dans un groupe constitué par une simple liaison, -O- et -NR₁₁- ;
R₁₁ étant choisi dans un groupe constitué par H et C₁₋₃ alkyle ;
Rₐ étant choisi dans un groupe constitué par F, Cl, Br, I, OH, NH₂, CN, C₁₋₃ alkyle et C₁₋₃ alcoxy, chacun parmi C₁₋₃ alkyle et C₁₋₃ alcoxy étant éventuellement substitué par 1, 2 ou 3 R ;
chacun parmi R_{b}, et Rₑ étant indépendamment choisi dans un groupe constitué par H, F, Cl, Br, I, OH, NH₂, CN et C₁₋₃ alkyle ;
R étant choisi dans un groupe constitué par F, Cl, Br, I, OH, NH₂, CN et CH₃ ;
chacun parmi hétérocycloalkyle à 3 à 8 chaînons et hétéroaryle à 5 à 10 chaînons comprenant indépendamment 1, 2, 3 ou 4 hétéroatome(s) ou groupe(s) d'hétéroatome(s) qui est/sont indépendamment choisi (s) dans un groupe constitué par -NH-, -O-, -S- et N.

2. Composé, ou sel pharmaceutiquement acceptable correspondant selon la revendication 1, Rₐ étant choisi dans un groupe constitué par F, Cl, Br, I, OH, NH₂, CN, CH₃, CF₃, CH₂F, CHF₂, CH₂CH₃ et OCH₃.

3. Composé, ou sel pharmaceutiquement acceptable correspondant selon la revendication 1, R₁ étant choisi dans un groupe constitué par C₁₋₁₂ alkyle, phényle, cyclobutyle, cyclopentyle, cyclohexyle, tétrahydrofuranyle, tétrahydropyranyle, pipéridinyle, thiényle, furyle, pyrrolyle et benzofuryle, chacun parmi C₁₋₁₂ alkyle, phényle, cyclobutyle, cyclopentyle, cyclohexyle, tétrahydrofuranyle, tétrahydropyranyle, pipéridinyle, thiényle, furyle, pyrrolyle et benzofuryle étant éventuellement substitué par 1, 2 ou 3 Rₐ.

4. Composé, ou sel pharmaceutiquement acceptable correspondant selon la revendication 3, R₁ étant choisi dans un groupe constitué par CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, 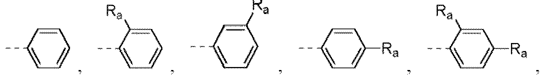 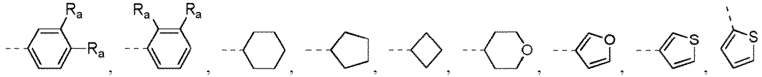 et

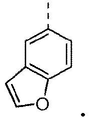

5. Composé, ou sel pharmaceutiquement acceptable correspondant selon la revendication 3, R₁ étant choisi dans un groupe constitué par CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃,

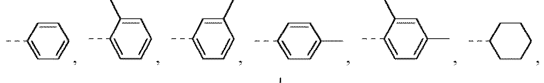

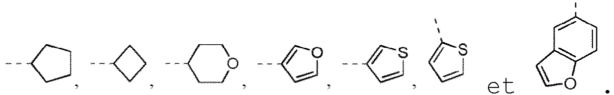

6. Composé, ou sel pharmaceutiquement acceptable correspondant selon la revendication 1, chacun parmi R₂ et R₃ étant indépendamment choisi dans un groupe constitué par H, F, Cl, Br, I, OH et NH₂.

7. Composé, ou sel pharmaceutiquement acceptable correspondant selon la revendication 1, R₄ étant choisi dans un groupe constitué par H, F, Cl, Br, I, OH, NH₂, CN, CH₃ et CH₂CH₃.

8. Composé, ou sel pharmaceutiquement acceptable correspondant selon la revendication 1, L étant choisi dans un groupe constitué par une simple liaison, -O-, - NH- et -N(CH₃)-.

9. Composé, ou sel pharmaceutiquement acceptable correspondant selon la revendication 1, le motif de structure 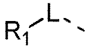 étant choisi dans un groupe constitué par CH₃, CH₂CH₃, (CH₂)₂CH₃, (CH₂)₃CH₃, (CH₂)₄CH₃, (CH₂)₅CH₃, (CH₂)₆CH₃, (CH₂)₁₀CH₃, CH(CH₃)₂, C(CH₃)₃, OCH₃, OCH₂CH₃, O(CH₂)₂CH₃, O(CH₂)₃CH₃, O(CH₂)₄CH₃, O(CH₂)₅CH₃, O(CH₂)₆CH₃, OCH(CH₃)₂, OC(CH₃)₃, N(CH₃)₂,

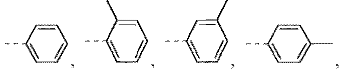

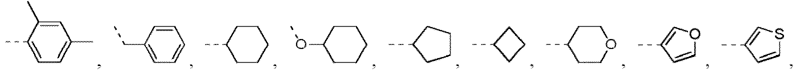

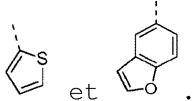

10. Composé ou sel pharmaceutiquement acceptable correspondant selon la revendication 1, qui est choisi dans un groupe constitué par : 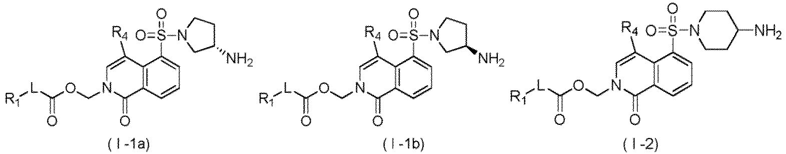 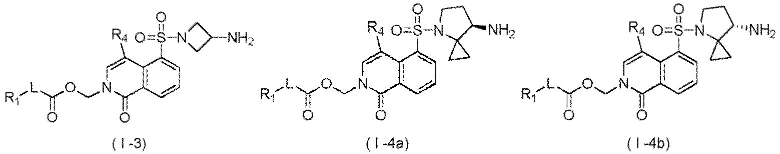
R₁ étant tel que défini dans la revendication 1, 3 ou 4 ;
R₄ étant tel que défini dans la revendication 1 ou 7 ; et
L étant tel que défini dans la revendication 1 ou 8.

11. Composé de la formule suivante selon la revendication 1, ou sel pharmaceutiquement acceptable correspondant,

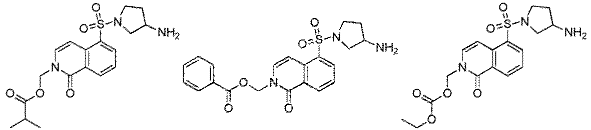

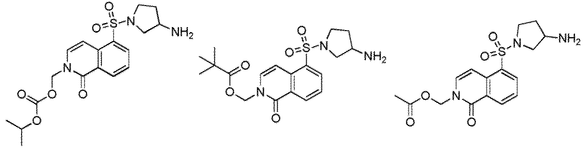

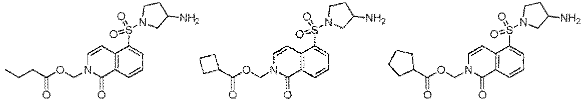

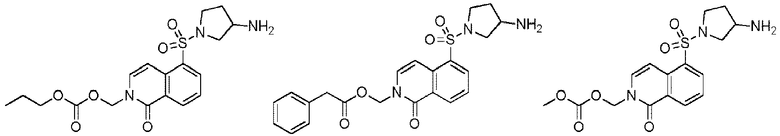

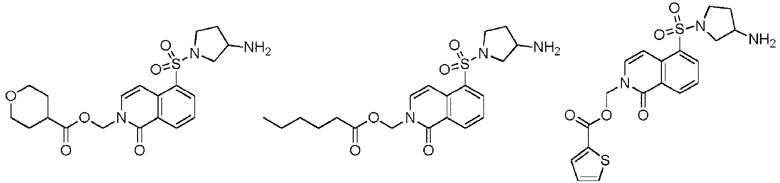

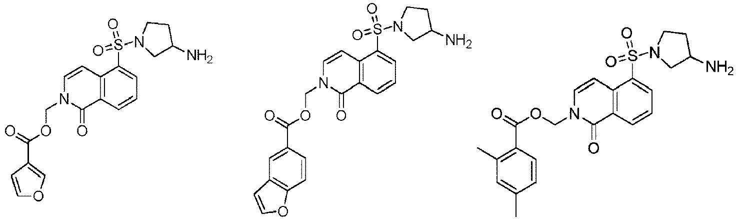

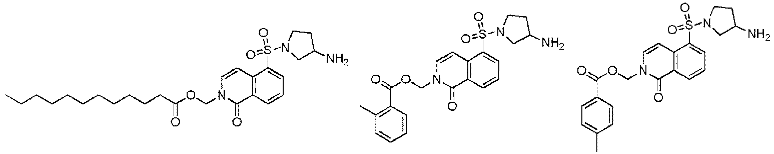

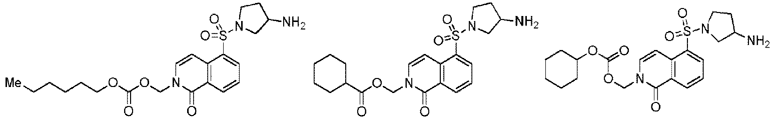

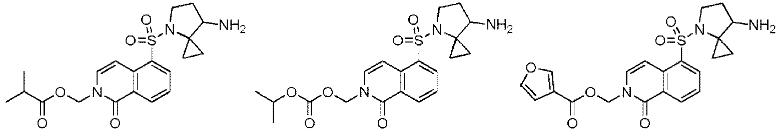

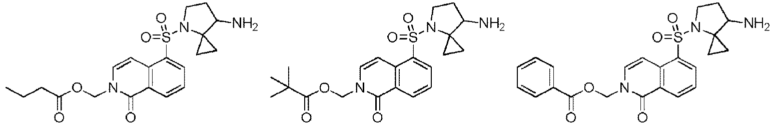

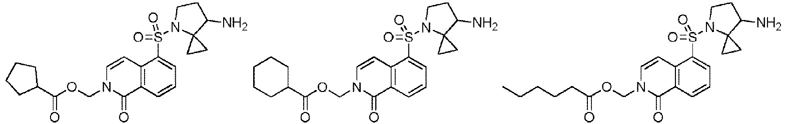

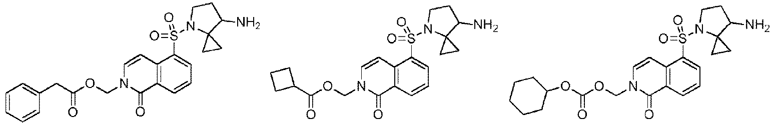

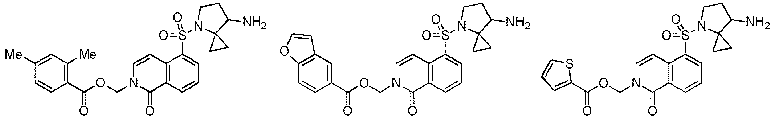

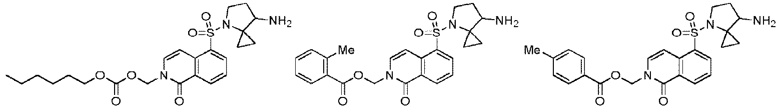

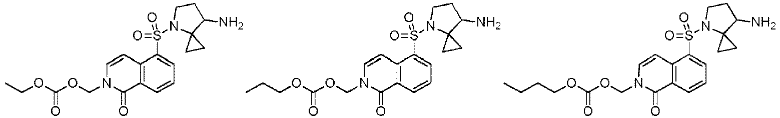

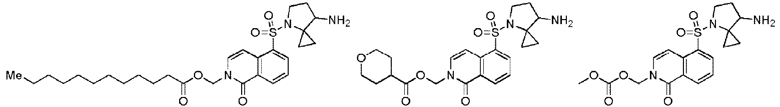

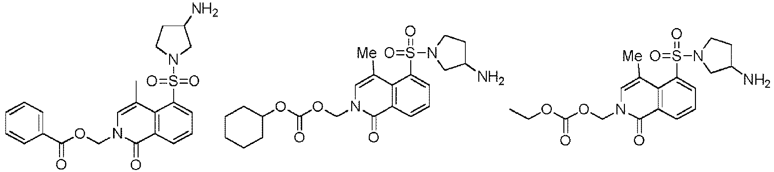

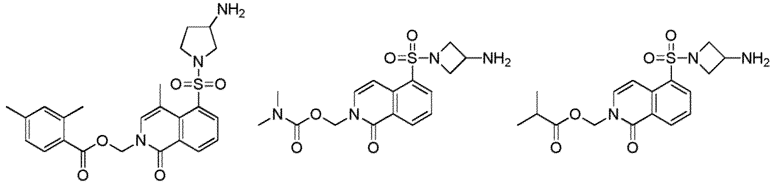

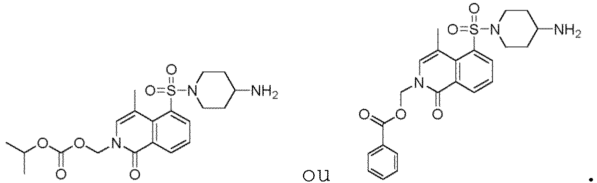

12. Composé de la formule suivante selon la revendication 11, ou sel pharmaceutiquement acceptable correspondant : 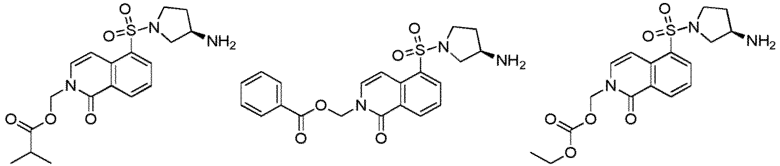 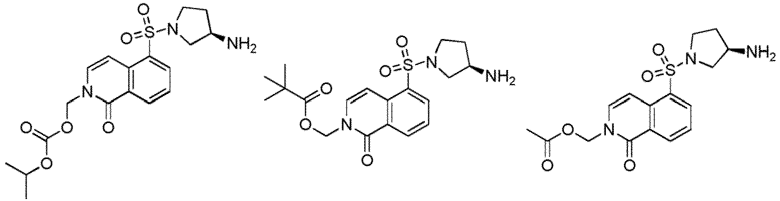 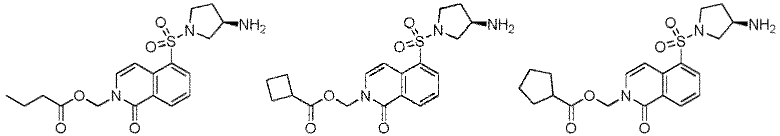 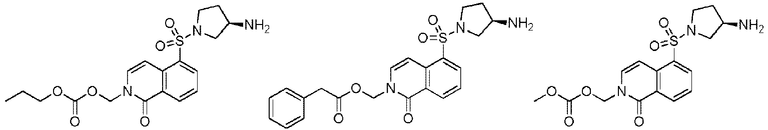 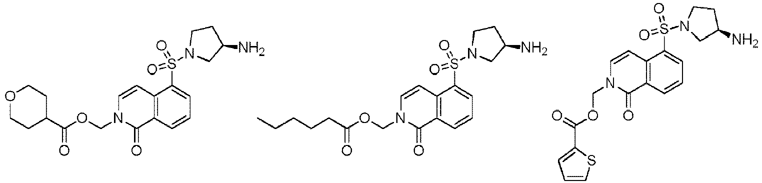 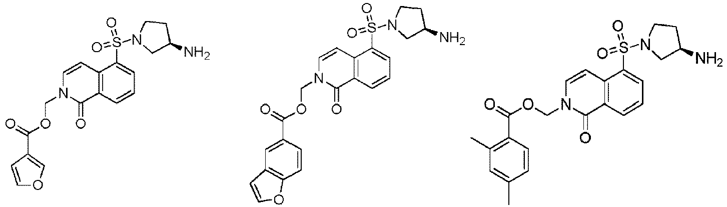 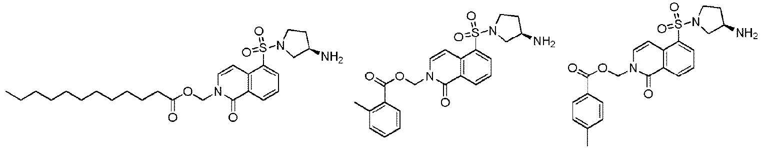 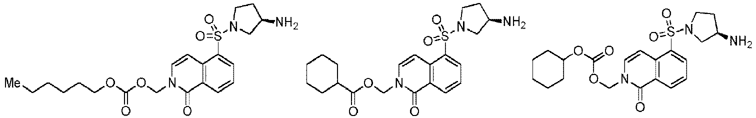 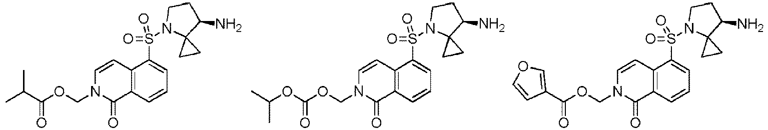 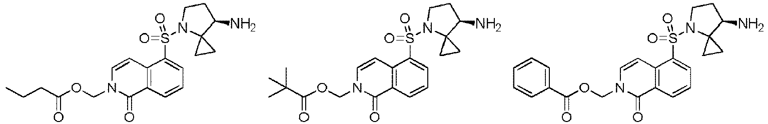 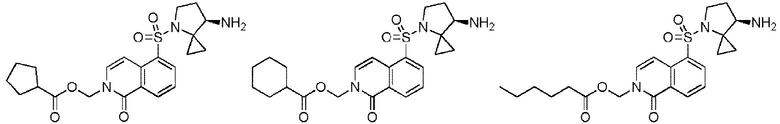 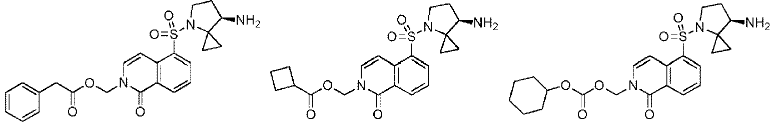 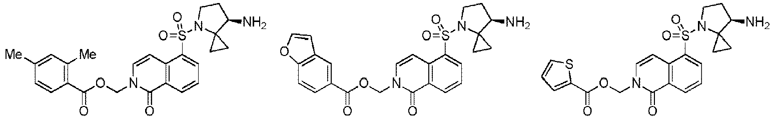 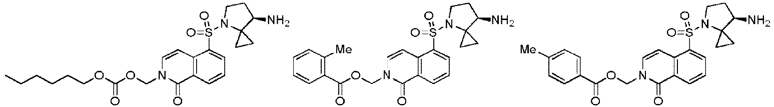 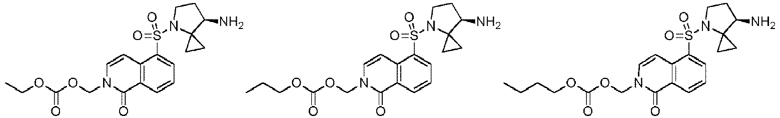 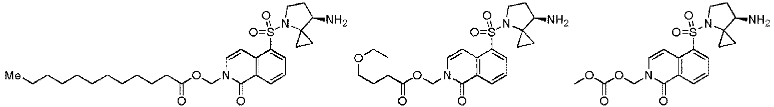 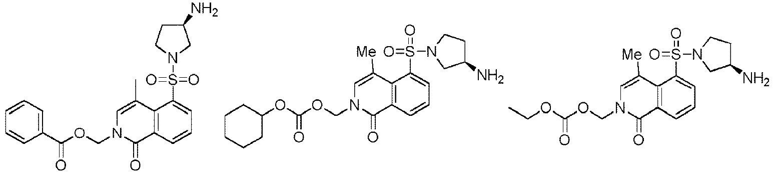 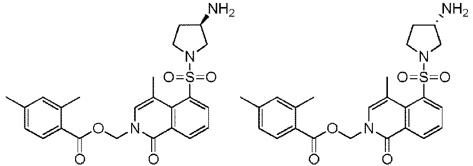 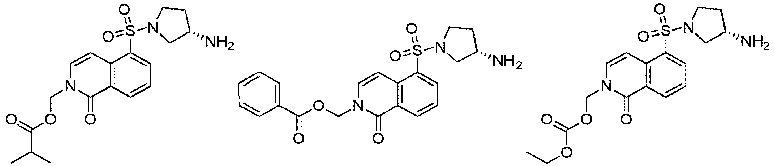 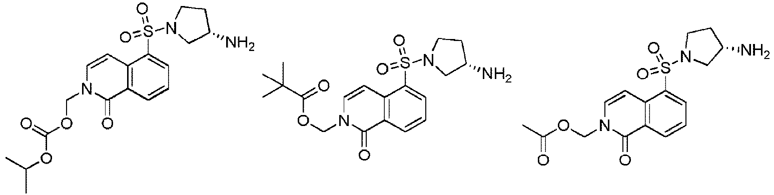 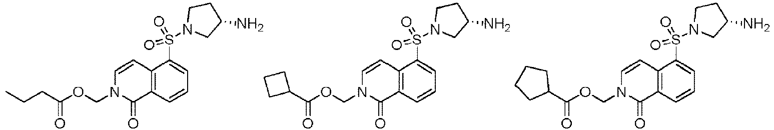 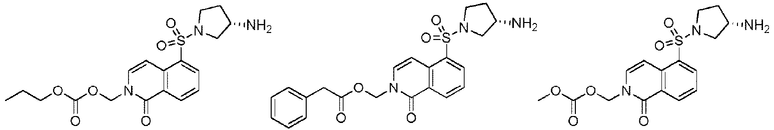 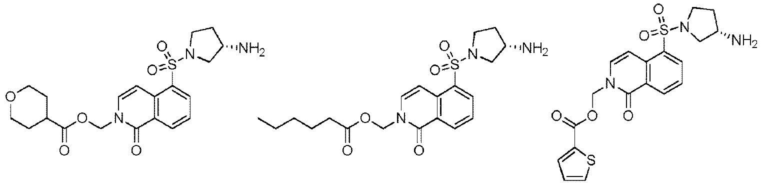 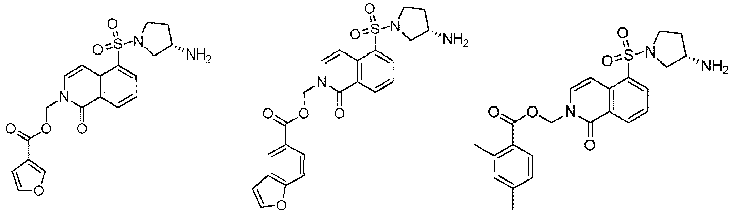 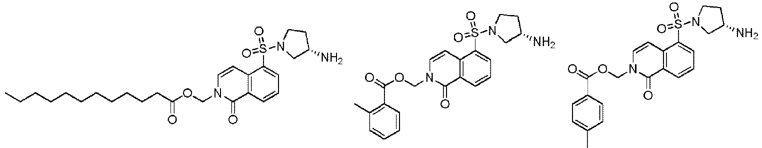 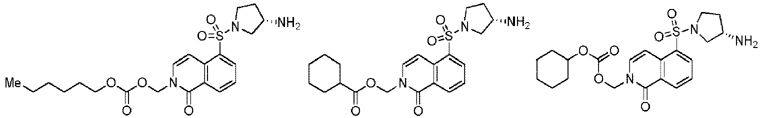 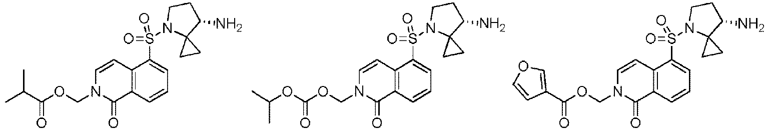 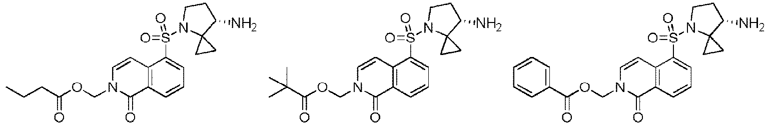 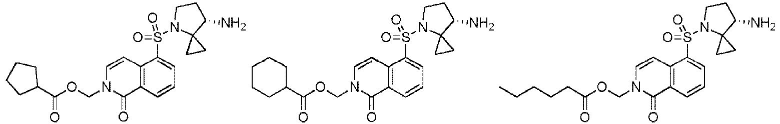 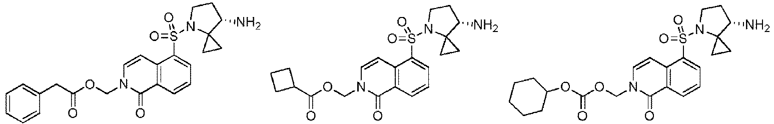 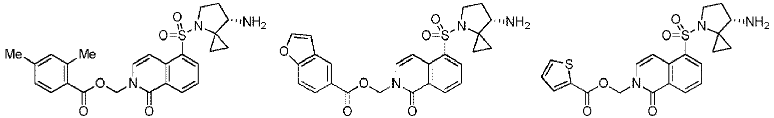 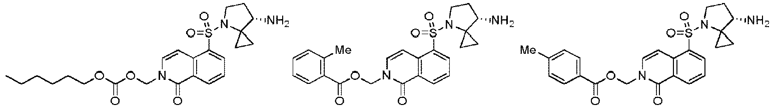 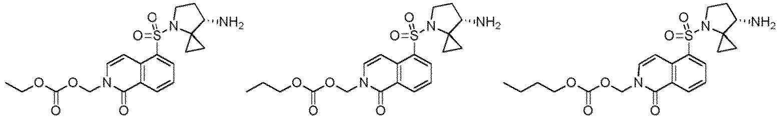 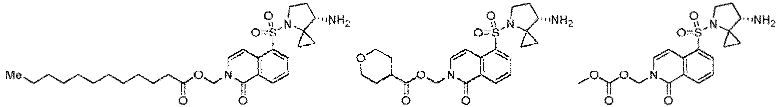 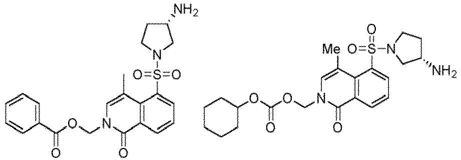 et

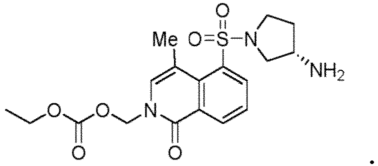

13. Composition pharmaceutique, comprenant une quantité thérapeutiquement efficace du composé ou du sel pharmaceutiquement acceptable correspondant selon l'une quelconque des revendications 1-12 et un support pharmaceutiquement acceptable.

14. Composé ou sel pharmaceutiquement acceptable correspondant selon l'une quelconque des revendications 1-12 ou composition selon la revendication 13, pour une utilisation dans le traitement du glaucome ou de l'hypertension oculaire.
